# EUROPEAN PATENT APPLICATION

(11) **EP 4 464 318 A1**
(43) Date of publication of application: **20.11.2024**
(21) Application number: 23174298.2
(22) Date of filing: 19.05.2023
(51) Int. Cl.: A61K 31/4184, A61K 31/19, A61K 31/352, A61K 31/365, A61K 31/5377, A61K 39/00, C07K 14/725, A61P 25/00, A61P 35/02

(54) **TARGETING TGF-B-ACTIVATED KINASE-1 ACTIVATION IN THE TREATMENT OF NEUROINFLAMMATORY CONDITIONS**

(71) Applicant: Albert-Ludwigs-Universität Freiburg, 79098 Freiburg (DE)
(72) Inventor: Zeiser, Robert, 79100 Freiburg (DE)
(74) Representative: Hertin und Partner Rechts- und Patentanwälte PartG mbB

(57) **Abstract**

The invention relates in a first aspect to a transforming growth factor-β-activated kinase-1 (TAK1) inhibitor for use in the treatment and/or prevention of a neuroinflammatory disorder, wherein the is preferably a T-cell induced microglia-mediated neuroinflammation. The invention relates in another aspect to a method for inhibiting microglia activation in a mammalian subject comprising inhibition of TAK1 which inhibits (reduces severity of) neuroinflammatory disorders, toxicities of cytokine release syndrome (CRS), neurotoxicity and/or neurocognitive defects. In another aspect the present invention relates to an inhibitor of microglial activation for use in the treatment of CAR T ICANS. In a further aspect the present invention relates to a method of treatment of a mammalian subject suffering a neuroinflammatory disorder, the method comprising administering a TAK1 inhibitor to said subject.

## Description

The invention lies in the field of molecular biology and medicine, particularly in the field of immunobiology and kinase inhibition.

The invention relates in a first aspect to a transforming growth factor-β-activated kinase-1 (TAK1) inhibitor for use in the treatment and/or prevention of a neuroinflammatory disorder, wherein the neuroinflammatory disorder is preferably a T-cell induced microglia-mediated neuroinflammation. The invention relates in another aspect to a method for inhibiting microglia activation in a mammalian subject comprising inhibition of TAK1 which inhibits neuroinflammatory disorders, toxicities of cytokine release syndrome (CRS), neurotoxicity and/or neurocognitive defects in said subject. In another aspect the present invention relates to an inhibitor of microglial activation for use in the treatment of CAR T ICANS. In a further aspect the present invention relates to a method of treatment of a mammalian subject suffering a neuroinflammatory disorder, the method comprising administering a TAK1 inhibitor to said subject.

### BACKGROUND OF THE INVENTION

Chimeric antigen receptor-modified T (CAR T) cells targeting CD19 (CAR19 T cells) induce high remission rates in B-cell malignancies, but their transfer is frequently associated with cytokine release syndrome and neurotoxicity (1), also known as immune effector cell-associated neurotoxicity syndrome (ICANS). ICANS is considered a major complication after CAR19 therapy for B cell malignancies and has been associated with an increased morbidity. In recent reports, neurotoxicity after CAR19 T- cell infusion was observed in 20-70% of the patients, with 10-34% experiencing grade 3-4 toxicity (2, 3). The most common neurological symptoms were encephalopathy, headache, delirium, transient impairment of cognition, attention deficits, tremor, aphasia, and focal weakness (2, 4, 5). Cerebrospinal fluid (CSF) analyses in patients developing severe neurotoxicity revealed high levels of IL-6 (interleukin 6), IL-8 (interleukin 8), C-C motif chemokine ligand 2 (CCL-2), and C-X-C motif chemokine ligand 10 (CXCL-10) (1), suggesting that these may play a role in the pathophysiology. Furthermore, patients with severe ICANS exhibited signs of endothelial activation and increased blood-brain barrier (BBB) permeability (6). The BBB disruption may be caused by high concentrations of systemic cytokines in the CSF, thereby inducing brain vascular pericyte stress and secretion of endothelium-activating cytokines (6). In support of these findings, single-cell RNA-sequencing (scRNA-seq) of the central nervous system (CNS) of CAR T cell-treated patients showed that mural cells, which surround the endothelium and are critical for BBB integrity, express CD19, which makes them CAR19 target cells (7). Another study using scRNA-seq in patients treated with CAR19 reported that a rare cell population with monocyte-like transcriptional features was associated with high-grade neurotoxicity (8).

Studies in mice showed that monocyte-derived IL-1 (interleukin 1) and IL-6 are functionally involved in the development of CAR T cell-induced neurotoxicity (9), and that IL-1 blockade has therapeutic activity (10). Additionally, studies demonstrated that GM-CSF inhibition might serve as a potential strategy to reduce CRS and neuroinflammation in xenograft models (11). Corticosteroids are recommended as a first-line therapy for the treatment of ICANS (12). However, recent studies indicate that their use is connected to decreased progression free survival after CAR T cell infusion (13) and shorter overall survival. As corticosteroid therapies can diminish or entirely abolish the anti-cancer effects of CAR T cell therapy, the intervention with corticosteroids or glucocorticoids in patients experiencing ICANS after CAR T cell therapy, significantly impairs or prevents the anti-cancer effect of the CAR T cell therapy. ICANS is therefore presently one of the major impairing factors of the success of CAR T cell-based cancer therapy.

Therefore, novel therapeutic strategies have been suggested that target (myeloid) immune activation, without hindering CAR T cell function, which could improve clinical outcome of the anti-cancer therapy. Moreover, targeting kinases has become an attractive therapeutic strategy for inflammatory disease, leading to FDA approval of several kinase inhibitors for steroid-refractory chronic graft-versus-host disease (GVHD) (14, 15), and of the Janus kinase 1 and 2 inhibitor ruxolitinib for steroid-refractory acute GVHD (16).

While previous studies convincingly show a role for certain cytokines, BBB disruption and endothelial damage in the pathogenesis of ICANS, the role of microglia as the endogenous parenchymal CNS immune cells (17, 18) or CAR19-induced neurotoxicity has remained unclear.

In summary, patients developing severe irAEs (grade 3 and 4) after ICB are currently treated with a complete and durable interruption of the immunotherapy and the administration of glucocorticosteroids (Haanen et al., 2017). Both interventions may have negative effects on the anti-tumor immune response. Currently no prospective randomized trial supports the use of glucocorticosteroids or second line therapies such as the frequently used approaches with mycophenolate mofetile (MMF), tumor necrosis factor (TNF) antagonists or cyclosporine A. Novel approaches that are active against irAE are lacking.

Therefore, therapeutic approaches for the treatment and prevention of neuroinflammatory side effects of CAR T cell (immuno-)therapy that preferably do not interfere with said therapy or diminish its anti-tumor effects are an unmet medical need.

### SUMMARY OF THE INVENTION

In light of the prior art the technical problem underlying the present invention is to provide improved therapeutic strategies for the treatment and/or prevention of neuroinflammatory disorders, such as side effects of immunotherapy. One object of the invention is that said novel therapeutic strategies do not interfere with an anti-tumor therapy in a subject, such as anti-tumor effects of CAR T cell (immuno-)therapy.

This problem is solved by the features of the independent claims. Preferred embodiments of the present invention are provided by the dependent claims.

The invention therefore relates in a first aspect to a transforming growth factor-β-activated kinase-1 (TAK1) inhibitor for use in the treatment of a neuroinflammatory disorder.

In embodiments the invention relates to a transforming growth factor-β-activated kinase-1 (TAK1) inhibitor for use in the treatment and/or prevention of a neuroinflammatory disorder.

The present interventions for patients that receive immunotherapy, such as CAR T cells, and experience immune effector cell-associated neurotoxicity syndrome (ICANS) are based on corticosteroids, which have been shown to decrease progression-free survival of patients (13). Additionally, a higher cumulative dose of corticosteroids, and prolonged and early use after CAR-T cell infusion were associated with significantly shorter overall survival (13).

The inventors surprisingly found that both, the administration of a TAK1 inhibitor (TAK1i) could successfully be used in the treatment of ICANS in a subject. Moreover, the inventors unexpectedly revealed that TAK1 inhibition effectively prevents cytokine release syndrome (CRS) and neurotoxicity and has the capacity to treat and/or prevent ICANS while anti-tumor effects of immunotherapy, particularly chimeric antigen receptor-modified T (CAR T) cells targeting CD19 (CAR19 T cell) therapy, are preserved and are surprisingly even enhanced. This beneficial effect, as well as the observed synergy and enhancement of anti-tumor efficacy, was entirely unexpected as it has not been described or suggested before.

The inventors surprisingly identified a new therapy for targeting neuroinflammatory and neurotoxic side effects of immunotherapy, such as ICANS, which is the inhibition of TGF-β-activated kinase-1 (TAK1). As shown in the Examples herein, pharmacological TAK1-inhibition or selective genetic Tak1 deletion in microglia using Cx3cr1^{creER} Tak1^{fl/fl} mice resulted in reduced ICANS severity. Importantly, TAK1-inhibition allowed for effective CAR19-induced anti-lymphoma effects *in vivo.*

As shown in the examples herein the inventors examined the role of microglia as the primary parenchymal immune cells of the central nervous system (CNS) during ICANS using an immunocompetent mouse model and human samples. Upon mouse CD19-directed CAR T cell (CAR 19) transfer in B-cell lymphoma-bearing mice, they observed morphological signs of microglia activation, increased production of GM-CSF (Colony Stimulating Factor 2), CCL-2 (C-C motif chemokine ligand 2), and tumor necrosis factor (TNF) by microglia, as well as neurocognitive deficits and impairments in said mice. Of note, microglial depletion with PLX5622 reduced neurocognitive deficits. The TGF-β-activated kinase-1 (TAK1)/NF-κB p38-MAPK-pathway was activated in microglia upon CAR19 transfer. Pharmacological TAK1-inhibition or genetic Tak1-deletion in microglia using Cx3cr1^{creER}:Tak1^{fl/fl} mice unexpectedly resulted in reduced microglia activation, lower TNF and GM-CSF production, and improved neurocognitive activity. FACS and single cell sequencing analysis revealed that gene expression profiles of microglia significantly changed upon CAR19 T cell therapy. GO-enrichment analysis of said single cell sequencing data revealed the upregulation of gene expression of IL-6, and genes associated with cell migration and/or cell proliferation, such as e.g., TNF. Moreover, also the gene expression of postsynaptic neurotransmitters, which are involved in memory (trans-)formation, was changed significantly. Further, the inventors could surprisingly unravel in their experiments that TAK1-inhibition allowed for potent CAR19-induced anti-lymphoma effects *in vivo.* Patients with ICANS exhibited microglia activation *in vivo* when studied by translocator protein positron emission tomography (TSPO-PET) and in post-mortem analysis. In summary, the inventors have identified a novel functional role for microglia in promoting CAR19- mediated neurotoxicity and have shown the TAK1/NF-κB p38-MAPK-axis to be a pathogenic signaling pathway activated in microglia during ICANS, using both genetic and pharmacological approaches. The inventors have also identified TAK1-inhibition as a new interference therapy against cytokine release syndrome, neurotoxicity and ICANS.

The present invention therefore provides novel targeted strategies inhibiting selective immune activation without blocking immunotherapy, such as CAR-T cell therapy, function that could improve clinical treatment outcome.

Hence, in embodiments the neuroinflammatory disorder comprises or consist of immune effector cell-associated neurotoxicity syndrome (ICANS) in a subject.

In embodiments the neuroinflammatory disorder comprises or consist of a cytokine-release syndrome (CRS).

In embodiments the neuroinflammatory disorder is a T-cell induced microglia-mediated neuroinflammation.

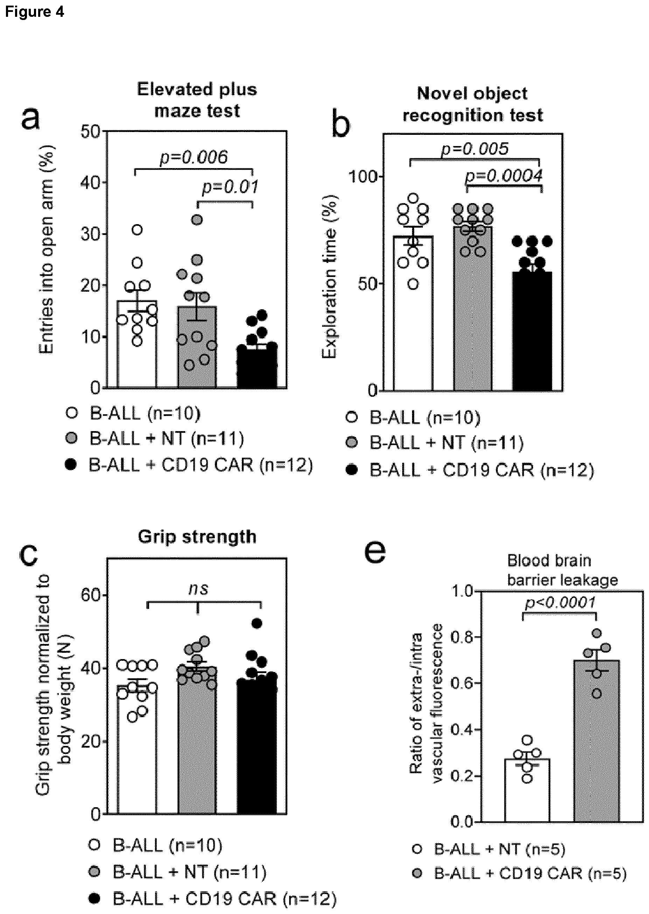

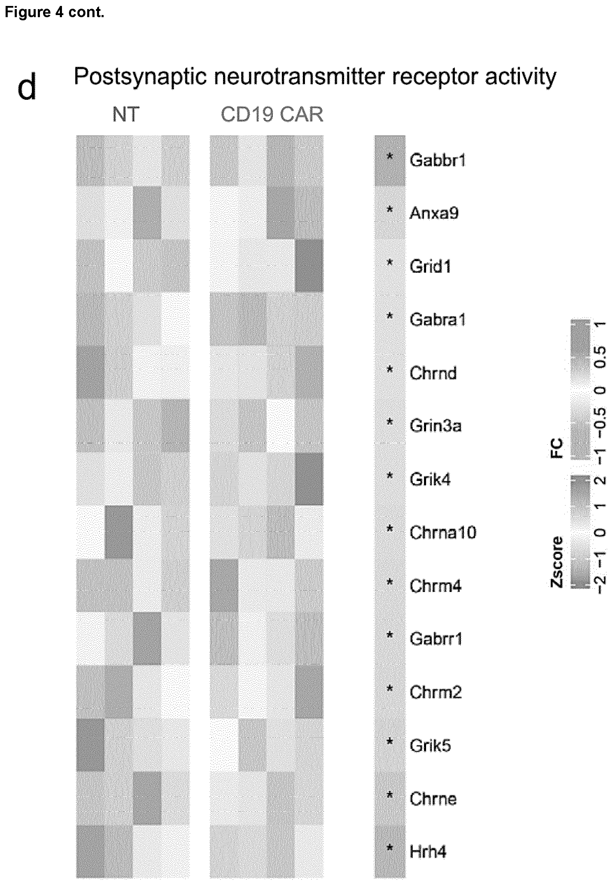

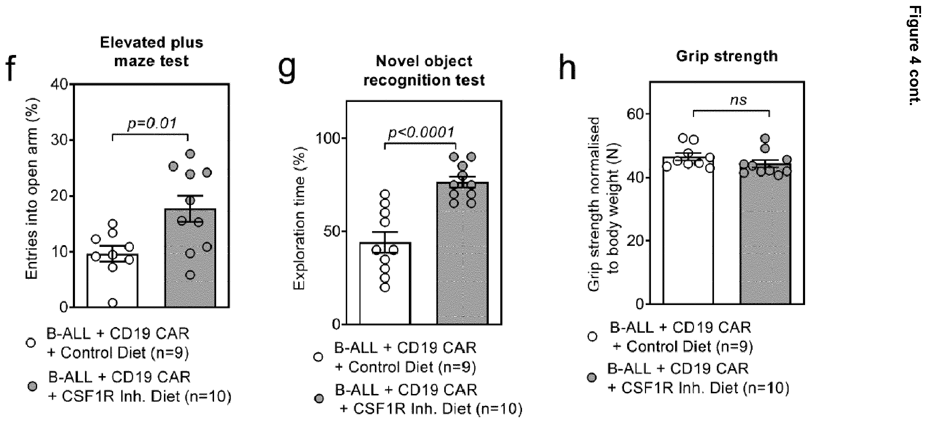

The inventors surprisingly revealed a novel role for microglia in ICANS, by identifying the TAK1/NF-κB p38-MAPK-axis as pathogenic signaling pathway in mice and were able to provide a rationale to test takinib, as a non-limiting example for a TAK1-inhibitor, in a clinical trial for prevention of ICANS after CAR19 T cell-based immunotherapy. By applying a phosphokinase search approach in microglia, the inventors could show that kinases of the MAPK-pathway are hyperphosphorylated in activated microglia during ICANS. Using said phosphokinase search approach, genetic loss-of-function studies and TAK1-inhibitor based studies, they identified the TAK1/NF-κB p38-MAPK-pathway as a pharmacological target to reduce ICANS severity, without impairing the anti-lymphoma effects of the CAR19 T cell based immunotherapy. Upon administration of a TAK1i the inventors could show that phosphorylation and therefore activation of TNF, GM-CSF, p38 and p70 S6K could be reduced in microglia cells, thus likely causing or ad least adding to the observed reduction of neurological impairments in mice. Patients with ICANS exhibited microglia activation in imaging-mass-cytometry-based analysis (IMC). The inventors could further show, that a CSF1R inhibiting diet also inhibits the microglia activation *in vivo* (see, e.g., Fig. 4).

Therefore, in embodiments the neuroinflammatory disorder is a microglia-mediated neuroinflammation.

In embodiments the neuroinflammatory disorder is a T-cell induced microglia-mediated neuroinflammation. In embodiments the T-cell induced microglia-mediated neuroinflammation is suspected to or associated with a T cell-based immunotherapy. In embodiments the T cell-based immunotherapy is a CAR T cell therapy.

In embodiments the TAK1 inhibitor reduces microglial activation in said subject.

In embodiments said inhibitor inhibits TAK1 pharmacologically and/or genetically by inhibiting its expression. The pharmacological inhibition is preferably achieved by a substance, compound, a small molecule or composition that inhibits TAK1, such as, e.g., its activation, its activity, its function and/or its kinase activity. The genetical inhibition is preferably the inhibition of the expression, gene transcription, mRNA splicing, and/or translation of TAK1, or the induction or enhancement of the degradation of TAK1 mRNA and/or TAK1 protein (e.g., proteasomal degradation). In embodiments the genetical inhibition can be achieved through RNAs, mRNAs, siRNAs, other interfering RNAs, antisense nucleic acids (DNA or RNA), DNA constructs, proteins, enzymes, antibodies, a substance, compound, a small molecule or a composition comprising any one of the afore or a combination thereof.

In embodiments the patient is suffering from one or more of the symptoms selected from cognitive deficits, neurotoxicity, seizures, pathological headaches, increased blood brain barrier (BBB) permeability, partial or complete loss of eye sight, partial or complete loss of hearing, partial or complete loss of sensation, partial or complete paralysis, muscle weakness, memory loss, numbness in one or more limbs, tremor, paresis, confusion or disorientation.

Neuroinflammatory disorders which are not related to immunotherapy are also considered applicable for the treatment approaches disclosed herein. Neuroinflammatory disorders, such as multiple sclerosis (MS), amyotrophic lateral sclerosis (ALS), Alzheimer's disease (AD) or Parkinson's disease (PD) are considered herein to be susceptible to TAK1-inhibition therapy. Neuroinflammatory disorders, such as MS, AD or PD have been shown before to be associated with increased microglia activation and the neuroinflammation observed in said conditions is considered in the context of the invention to be (at least partially) microglia-mediated.

Hence, in embodiments the neuroinflammatory disorder is associated with a condition selected from the group comprising immune effector cell-associated neurotoxicity syndrome (ICANS), multiple sclerosis (MS), amyotrophic lateral sclerosis (ALS), Alzheimer's disease (AD) or Parkinson's disease (PD). Accordingly, in embodiments the invention relates to a TAK1 inhibitor for use in the treatment of a neuroinflammatory disorder, which is selected from the group comprising immune effector cell-associated neurotoxicity syndrome (ICANS), multiple sclerosis (MS), amyotrophic lateral sclerosis (ALS), Alzheimer's disease (AD) or Parkinson's disease (PD).

In embodiments of the TAK1 inhibitor for use in the treatment of a neuroinflammatory disorder, the neuroinflammatory disorder comprises or consist of a condition selected from the group comprising microglia-mediated neuroinflammation, T-cell induced microglia-mediated neuroinflammation, cytokine-release syndrome (CRS), T-cell induced microglia-mediated neuroinflammation, a condition comprising elevated microglial activation, a neuroinflammatory disorder of the central nervous system (CNS), multiple sclerosis (MS), amyotrophic lateral sclerosis (ALS)/motor neuron disease (MND), immune effector cell-associated neurotoxicity-syndrome (ICANS), Acute disseminated encephalomyelitis (ADEM), Accute Optic Neuritis (AON), Transverse Myelitis Neuromyelitis Optica (NMO), Alzheimer's disease (AD) and Parkinson's disease (PD).

In embodiments the neuroinflammatory disorder comprises or consist of a neuroinflammatory disorder of the central nervous system (CNS).

In embodiments the neuroinflammatory disorder is an ICANS in a mammalian subject, wherein said subject is receiving a chimeric antigen receptor (CAR) T-cell based immunotherapy for the treatment of cancer. In embodiments of the invention the neuroinflammatory disorder is an ICANS in a mammalian subject, wherein said subject is receiving a chimeric antigen receptor (CAR) T-cell based immunotherapy for the treatment of haematological (B-cell) malignancies.

Accordingly, in embodiments of the invention the neuroinflammatory disorder is an ICANS in a mammalian subject, which is suspected to be or is caused/associated with an immunotherapy the subject will, is or has been receiving. In embodiments the immunotherapy is a chimeric antigen receptor (CAR) T-cell based immunotherapy. In embodiments the subject is receiving an immunotherapy for the treatment of haematological (B-cell) malignancies. In embodiments the ICANS is considered or suspected to constitute a side effect caused by an immunotherapy the subject will, is or has been receiving. In embodiments wherein the subject is planned to receive immunotherapy in the near future, such as e.g., within 1, 2, 6, 12, 24, 36, 48, or 72 hours the TAK1 inhibition therapy or TAK1 inhibitor is considered to be used in or as a preventative treatment of a neuroinflammatory disorder.

In embodiments of the invention haematological (B cell) malignancies are selected from the group comprising B-cell lymphoma, B-cell acute lymphoblastic leukemia (B-ALL), precursor B-cell ALL, B-cell non-Hodgkin lymphoma (B-NHL), mixed lineage leukemia (MLL), large cell transformation of follicular lymphoma (trFL), Diffuse large B-cell lymphoma (DLBCL), Follicular lymphoma (FL), Chronic lymphocytic leukemia (CLL) /small lymphocytic lymphoma (SLL), mantle cell lymphoma (MCL), marginal zone lymphoma (MZL), Burkitt lymphoma (BL), lymphoplasmacytic lymphoma, hairy cell leukemia (HCL), primary central nervous system (CNS) lymphoma, primary intraocular lymphoma and monoclonal B-cell lymphocytosis (MBL).

In preferred embodiments the anti-cancer effects of the CAR T-cell based immunotherapy are not impaired. In preferred embodiments the anti-lymphoma and/or anti-leukemia effects of the CAR T-cell based immunotherapy are not impaired.

One of the most striking and entirely unexpected results the inventors observed upon TAK1 inhibition was that said TAK1 inhibition achieved a synergistic effect regarding the anti-cancer efficacy of a parallel, prompt or concurrent chimeric antigen receptor (CAR) T-cell based immunotherapy in a subject. In other words, the inventors revealed that inhibition of TAK1, for example through a TAK1-inhibitor, surprisingly achieved a synergistic anti-cancer efficacy with a prompt or concurrent chimeric antigen receptor (CAR) T-cell based immunotherapy in a subject.

The invention therefore also relates in one aspect to the combined use of a transforming growth factor-β-activated kinase-1 (TAK1) inhibitor and chimeric antigen receptor (CAR) T-cell based immunotherapy in the treatment of haematological (B-cell) malignancies. Non-limiting examples of haematological (B-cell) malignancies are described herein. Hence, the invention relates in embodiments to a combination medication or combination therapy comprising a transforming growth factor-β-activated kinase-1 (TAK1) inhibitor and chimeric antigen receptor (CAR) T-cell based immunotherapy for use in the treatment of haematological (B-cell) malignancies.

IL-1 and IL-6 have been shown to be functionally involved in the development of CAR T cell-induced neurotoxicity (9). Current prophylaxis and therapy for ICANS includes tocilizumab, a monoclonal antibody targeting interleukin-6 (56). Previous studies have shown that IL-6 production is induced via TAK1-NFκB pathway activation (57). However, in contrast to a selective blockade of the IL-6R (interleukin 6 receptor), the use of TAK1 inhibitors (TAK1i), e.g., takinib, is especially advantageous, as targeted inhibition of TAK1, which is a central regulatory kinase in the TAK1-TAB signalosome (58, 59). TAK1 is known to mediate production of multiple pro-inflammatory cytokines involved in ICANS including TNF (58, 59). TREM2 expression was shown to be associated with the production of inflammatory cytokines in a PI3K/AKT and NF-kB-dependent manner in neuroinflammatory diseases (32, 60), which is consistent with the inventors observation (see Examples) that increased levels of TREM2 and TAK1 are found in activated microglia after CAR19 T cell therapy.

Hence, in embodiments the neuroinflammatory disorder is associated with an increased production, induction and/or activation of IL-6, p38, p70 S6K, GM-CSF, MCP-1 and/or TNF by activation of microglia, which is preferably identified by analyzing cerebrospinal fluid (CSF) of a subject. Herein p70 S6K refers to ribosomal protein S6 kinase B1, p38 refers to Mitogen-Activated Protein Kinase 14 (MAPK14), IL-6 refers to Interleukin 6, GM-CSF refers to Colony Stimulating Factor 2, MCP-1 refers to C-C motif chemokine ligand 2 (CCL2), and TNF refers to tumor necrosis factor. In embodiments the neuroinflammatory disorder is associated with the activation of one or more of said molecules/factors, e.g., of IL-6, p38, p70 S6K, GM-CSF, MCP-1 and/or TNF. In embodiments said activation is achieved by the phosphorylation of respective molecules/factors. In embodiments the neuroinflammatory disorder is associated with the activation of p38 and/or p70 S6K. In embodiments said activation is achieved by the phosphorylation of respective molecules/factors, such as, e.g., phospho-p38 and/or phospho-p70 S6K.

The activation of e.g., p38 and p70 S6K, comprises or is in embodiments achieved by phosphorylation of said proteins in certain positions, e.g., by a kinase. In some embodiments the activation of a protein can comprise the phosphorylation and/or chemical modification of said protein and/or a conformational change of said protein. In embodiments the production and/or induction of a protein can comprise the increase and/or induction of expression of said protein and/or the increase and/or induction of excretion of said protein by a cell.

In embodiments of the invention described herein the administration of the TAK1 inhibitor leads to reduced microglia activation, lower production of GM-CSF, MCP-1 and TNF.

In embodiments the administration of the TAK1 inhibitor reduces neuroinflammation.

In embodiments, administration of the TAK1 inhibitor reduces symptoms selected from cognitive deficits, neurotoxicity, seizures, pathological headaches, increased blood brain barrier (BBB) permeability, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects in a subject.

In embodiments of the TAK1 inhibitor for use according to the invention, the TAK1 inhibitor is selected from the group comprising Takinib (EDHS-206), HS-276, Dehydroabietic acid, RGB-286638 free base, Sarsasapogenin, 5Z-7-Oxozeaenol, NG25 and pharmaceutically acceptable salts thereof.

In embodiments the TAK1 inhibitor is Takinib (EDHS-206) or pharmaceutically acceptable salts thereof. In embodiments the TAK1 inhibitor is HS-276 or pharmaceutically acceptable salts thereof. In embodiments the TAK1 inhibitor is Dehydroabietic acid or pharmaceutically acceptable salts thereof. In embodiments the TAK1 inhibitor is RGB-286638 free base or pharmaceutically acceptable salts thereof. In embodiments the TAK1 inhibitor is Sarsasapogenin or pharmaceutically acceptable salts thereof. In embodiments the TAK1 inhibitor is 5Z-7-Oxozeaenol or pharmaceutically acceptable salts thereof. In embodiments the TAK1 inhibitor is NG25 or pharmaceutically acceptable salts thereof.

In embodiments the inhibitor is administered transiently or constantly.

In embodiments of the TAK1 inhibitor for use according to the invention, the treatment (of a subject) further comprises (simultaneous or sequential) administration of one or more other therapies selected from the group comprising corticosteroids, Anti-IL-6 receptor antibodies, anti-IL-1beta antibodies, anti-IL-1beta receptor antagonists (Anakinra), calcineurin inhibitors (Cyclosporine A, tacrolimus), Interferon-beta, glatiramer acetate, dimethyl fumarate, diroximel fumarate, monomethyl fumarate, teriflunomid, fingolimod, fingolimod, fingolimod, mitoxantrone, ponesimod, ozanimod fingolimod, ofatumumab, alemtuzumab, ocrelizumab, natalizumab and ublituximab-xiiy. In embodiments treatments comprising TAK1-inhibitor administration and one or more of the afore enlisted substances may be considered a combination treatment or combination medication according to the invention.

The invention therefore relates in another aspect to a method for inhibiting microglia activation in a mammalian subject comprising inhibition of TAK1, which inhibits (reduces severity of) neuroinflammatory disorders, toxicities of cytokine release syndrome (CRS), neurotoxicity and/or neurocognitive defects in said subject. In other words, in embodiments the method for inhibiting microglia activation in a mammalian subject comprises the inhibition of TAK1, such that in said subject a neuroinflammatory disorder is inhibited or its severity reduced, toxicities of cytokine release syndrome (CRS) are inhibited, neurotoxicity is inhibited and/or neurocognitive defects are inhibited.

In embodiments of the method according to the invention said subject is receiving a chimeric antigen receptor (CAR) T-cell based immunotherapy for the treatment of haematological (B-cell) malignancies. In embodiments of the method according to the invention said subject is receiving a CD19-directed CAR T-cell based immunotherapy for the treatment of haematological (B cell) malignancies.

In embodiments of the method according to the invention said therapeutic inhibition of TAK1 inhibits an immune effector cell-associated neurotoxicity syndrome (ICAN) in said subject. In embodiments of the method according to the invention said therapeutic inhibition of TAK1 inhibits ICAN which is induced by receiving a CAR(19) T cell (CAR(19) T) based immunotherapy for the treatment of haematological (B-cell) malignancies.

In embodiments of the method according to the invention said therapeutic inhibition of TAK1 comprises inhibiting a kinase activity of a kinase from an immune signaling pathways promoting (auto)immune response and inflammatory processes.

In embodiments of the method according to the invention said therapeutic inhibition of TAK1 comprises inhibiting the TGF-β-activated kinase-1 (TAK1)/ /NF-κB and/or p38-MAPK, wherein said TAK1/ /NF-xB p38-MAPK-pathway is activated in microglia upon CAR(19) T cell based immunotherapy.

In embodiments of the method according to the invention the inhibition of microglia activation in a mammalian subject does not reduce or impair the activity of CAR(19) T cells against the haematological B cell malignancies in said subject.

In embodiments of the method according to the invention the haematological (B cell) malignancies are selected from the group comprising leukemia, B-cell lymphoma, B-cell acute lymphoblastic leukaemia (B-ALL), precursor B-cell ALL, B-cell non-Hodgkin lymphoma (B-NHL), mixed lineage leukemia (MLL), large cell transformation of follicular lymphoma (trFL), Diffuse large B-cell lymphoma (DLBCL), Follicular lymphoma (FL), Chronic lymphocytic leukemia (CLL) /small lymphocytic lymphoma (SLL), mantle cell lymphoma (MCL), marginal zone lymphoma (MZL), Burkitt lymphoma (BL), lymphoplasmacytic lymphoma, hairy cell leukemia (HCL), primary central nervous system (CNS) lymphoma, primary intraocular lymphoma and monoclonal B-cell lymphocytosis (MBL).

In embodiments of the method according to the invention an effective amount of an inhibitor of TAK1/NF-κB p38-MAPK is used to inhibit or reduce microglial activation compared to that which would occur in the absence of the inhibitor.

In embodiments of the method according to the invention inhibiting the activation of microglia by inhibiting the TAK1/NF-xB p38-MAPK reduces the severity of ICAN and /or neurocognitive defects.

In embodiments of the method according to the invention an TAK1 inhibitor is used for inhibiting microglia activation.

In embodiments of the method according to the invention the TAK1 inhibitor is selected from the group comprising Takinib (EDHS-206), HS-276, Dehydroabietic acid, RGB-286638 free base, Sarsasapogenin, 5Z-7-Oxozeaenol, NG25 and pharmaceutically acceptable salts thereof.

In another aspect the present invention relates to a method for inhibiting TAK1 activity present in microglia in a mammalian subject, comprising the step of administering to said subject an effective inhibitory, preferably a therapeutically effective (inhibitory), amount of an inhibitor which reduces severity of ICAN induced by receiving a CAR T-cell based immunotherapy (e.g., CAR(19) T cell (CAR(19) T) based immunotherapy) for the treatment of haematological (B-cell) malignancies.

In further aspect the present invention relates to a method of inhibiting TAK1 and/or p38/MAPK activity present in microglia in a mammalian subject to reduce ICANS severity without impairing the anti-lymphoma effects of the CAR(19) T cell (CAR(19) T) based immunotherapy, using an effective, preferably a therapeutically effective, amount of an inhibitor of TAK1/NF-κB p38-MAPK to said subject.

In another aspect the present invention relates to the use of a TAK1 inhibitor for inhibiting microglia activation against CAR T ICANS while preserving anti-tumor effects. In embodiments of the present invention TAK1 inhibitor is used for inhibiting microglia activation which is associated with CAR T ICANS and/or ICANS caused by or associated with a CAR T cell therapy.

In embodiments of the use according to the invention the TAK1 inhibitor is selected from the group comprising Takinib (EDHS-206), HS-276, Dehydroabietic acid, RGB-286638 free base, Sarsasapogenin, 5Z-7-Oxozeaenol, NG25 and pharmaceutically acceptable salts thereof.

In another aspect the present invention relates to an inhibitor of microglial activation for use in the treatment of CAR T ICANS.

In another aspect the present invention relates to a method of treatment of a mammalian subject suffering from CAR T-induced ICANS comprising administering a TAK1 inhibitor. In embodiments of the method of treatment of a mammalian subject suffering from CAR T ICANS the method comprises administering to said subject a therapeutically effective amount of a TAK1 inhibitor. In embodiments the present invention relates to a method of treatment of a mammalian subject suffering a neuroinflammatory disorder, wherein the method comprises administering to said subject a TAK1 inhibitor, preferably administering a therapeutically effective amount of a TAK1 inhibitor, to said subject.

In embodiments of a TAK1 inhibitor for use according to the invention, the TAK1 inhibitor is administered at least 1 minute, 5, 10, 15, 20, 30, 45 or 60 minutes, or at least 1 hour, 2, 3, 4, 5, 6, 7, 8, 9, 10, 11, 12, 24, 36, or 48 hours, or at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks after administration of CAR(19) T cell (CAR(19) T) based (immuno)therapy.

In embodiments of a TAK1 inhibitor for use according to the invention, the TAK1 inhibitor is administered at least 1 minute, 5, 10, 15, 20, 30, 45 or 60 minutes, or at least 1 hour, 2, 3, 4, 5, 6, 7, 8, 9, 10, 11, 12, 24, 36, or 48 hours, or at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks before administration of CAR(19) T cell (CAR(19) T) based (immuno)therapy.

In embodiments of a TAK1 inhibitor for use according to the invention, the TAK1 inhibitor is administered at least once daily for at least 1, 2, 3, 4, 5, 6, 7, 14, 21, 28, 30 days or at least 1, 2, 3, 4, 5, 6, 7, 8, 9, 10, 11, 12, 13, 14, 15, 16, 20, 24, or 52 weeks during administration of CAR(19) T cell (CAR(19) T)- based (immuno)therapy.

Preferably an inhibitor of TGF-β-activated kinase-1 (TAK1) is administered at least once daily for at least 2 to 5 weeks during administration of CAR19 T cell therapy, more preferably for at least 3 weeks during administration of CAR(19) T cell (CAR(19) T) based (immuno)therapy.

In embodiments of an inhibitor of TGF-β-activated kinase-1 (TAK1) for use according to the invention, the subject suffers from cancer. In embodiments of an inhibitor of TGF-β-activated kinase-1 (TAK1) for use according to the invention, the subject has been receiving, will be receiving (within minutes, hours or days) or is currently receiving a treatment against cancer. In embodiments said treatment is an immunotherapy, for example, an antibody-based or CAR T cell therapy.

In embodiments, the subject suffers from cancer, such as haematological (B-cell) malignancies treatable by CAR(19) T cell (CAR(19) T) based immunotherapy.

In embodiments of an inhibitor of TGF-β-activated kinase-1 (TAK1) for use according to the invention, the cancer is selected from the group comprising (haematological) B cell malignancies, B-cell leukemia, B-cell lymphoma, B-cell acute lymphoblastic leukemia (B-ALL), precursor B-cell ALL, B-cell non-Hodgkin lymphoma (B-NHL), mixed lineage leukemia (MLL), large cell transformation of follicular lymphoma (trFL), Diffuse large B-cell lymphoma (DLBCL), Follicular lymphoma (FL), Chronic lymphocytic leukemia (CLL) /small lymphocytic lymphoma (SLL), mantle cell lymphoma (MCL), marginal zone lymphoma (MZL), Burkitt lymphoma (BL), lymphoplasmacytic lymphoma, hairy cell leukemia (HCL), primary central nervous system (CNS) lymphoma, primary intraocular lymphoma and monoclonal B-cell lymphocytosis (MBL) ., B-cell acute lymphoblastic leukemia (B-ALL), Non-Hodgkin Lymphoma (NHL) or another cancer treatable by immunotherapy, such as CAR(19) T cell (CAR(19) T) based immunotherapy, e.g., such as any cancer disclosed herein.

In embodiments of the TAK1 inhibitor for use according to the invention, the CAR19 T cell therapy comprises administration of Chimeric antigen receptor-modified T (CAR T) cells targeting CD19 (CAR19 T cells).

In embodiments of the TAK1 inhibitor for use according to the invention, the immunotherapy is an antibody-based cancer therapy. Non-limiting examples of antibody-based cancer therapy are monoclonal antibodies, (monoclonal) antibodies against programmed cell death-1 (PD-1), CD19 , CD20, CD3 or any other monoclonal antibody. In the context of the present invention any antibody-based cancer therapy is imagined.

In embodiments the immunotherapy is a therapy directed against Multiple Sclerosis (MS). In embodiments, the immunotherapy is an antibody-based therapy directed against Multiple Sclerosis, such as e.g., a therapy comprising an anti-CD20 antibody or anti CD19 antibody.

In embodiments of a TAK1 inhibitor for use according to the invention, the administration of said TAK1-inhibitor does not interfere with the anti-tumor response induced by an anti-cancer therapy, which is preferably a CAR(19) T cell (CAR(19) T) based immunotherapy. In embodiments of a TAK1 inhibitor for use according to the invention, a CAR(19) T cell (CAR(19) T) induced anti-tumor response is not impaired by the administration of the inhibitor of TAK1.

In embodiments, the subject is receiving immunosuppressive drugs, such as steroids, corticosteroid, cyclosporine and/or anti-TNF antibodies (for example infliximab) and/or is refractory to immunosuppressive drugs.

In embodiments, the inhibitor of TGF-β-activated kinase-1 (TAK1) for use according to the present invention is administered upon occurrence of neurological, CRS and/or ICANS symptoms. In embodiments, the inhibitor of TAK1 for use according to the present invention is administered to prevent the occurrence of neurological, CRS and/or ICANS symptoms.

In embodiments, the inhibitor of TGF-β-activated kinase-1 (TAK1) for use according to the present invention is administered to a subject upon occurrence of cognitive deficits, neurotoxicity, seizures, pathological headaches, increased blood brain barrier (BBB) permeability, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects in said subject.

In embodiments, the inhibitor of TGF-β-activated kinase-1 (TAK1) for use according to the present invention is administered to a subject upon occurrence of neuroinflammation in said subject. In embodiments, the inhibitor of TGF-β-activated kinase-1 (TAK1) for use according to the present invention is administered to a subject upon occurrence of CNS neuroinflammation in said subject.

In embodiments, the inhibitor of TGF-β-activated kinase-1 (TAK1) for use according to the present invention is administered to a subject upon the occurrence, detection and/or suspected occurrence of (CNS) microglia activation, and/or increased (CNS) production and/or (CNS) levels of GM-CSF, MCP-1 and/or TNF in said subject.

In embodiments an inhibitor of TAK1 is administered for 1, 2, 3, 4, 5, 6 or 7 days or for 1, 2, 3, 4, 5, 6, 7, 8, 9, 10, 12, 14, 16, 18, 20, 22, 24, 26, 28, 30, 35, 40, 45 or 50 weeks, preferably for 1-5 weeks, preferably for at least 1-3 weeks, more preferably for at least 2 weeks before, upon or after occurrence of neurological, CRS and/or ICANS symptoms more preferably for at least 3 weeks upon or after occurrence of neurological, CRS and/or ICANS symptoms. In embodiments, the inhibitor of TAK1 is administered during CAR(19) T cell (CAR(19) T) based immunotherapy or after the CAR(19) T cell (CAR(19) T) based immunotherapy was discontinued or completed. In embodiments, the inhibitor of TAK1 is administered before a subject is receiving CAR(19) T cell (CAR(19) T) based immunotherapy.

In embodiments of the combination medications described herein, the inhibitor of TAK1, is administered at least once to the subject, preferably at least twice, preferably on consecutive days. Accordingly, the subject may receive more than one dose of the inhibitor of TGF-β-activated kinase-1 (TAK1), wherein preferably not more than two doses per day are administered. In further embodiments, the subject receives at least 2, 3, 4 or 5 dosages of the inhibitor of TGF-β-activated kinase-1 (TAK1). In embodiments, the inhibitor of TGF-β-activated kinase-1 (TAK1) is administered to the subject at least every 8 weeks, preferably every 2-4 weeks. In embodiments the inhibitor of TGF-β-activated kinase-1 (TAK1) is administered before, during and/or after the administration of the CAR(19) T cell (CAR(19) T) based immunotherapy. In embodiments the inhibitor of TGF-β-activated kinase-1 (TAK1) bis administered at least once, more preferably on two days, and at least once per day. In embodiments wherein the TAK1 inhibitor and the CAR(19) T cell (CAR(19) T) based immunotherapy are administered to a subject at the same time or during the same time period or in temporal proximity to each other, said "combined administration" may be regarded as combination therapy or combination medication.

Therefore, the present invention further relates to a combination medication of a TAK1 inhibitor and CAR(19) T cell (CAR(19) T) based immunotherapy for use as a medicament. In embodiments the present invention further relates to a combination medication of a TAK1 inhibitor and CAR(19) T cell (CAR(19) T) based immunotherapy for use in the treatment of cancer. In embodiments the present invention further relates to a combination medication of a TAK1 inhibitor and CAR(19) T cell (CAR(19) T) based immunotherapy for use in the treatment of leukemia, B-cell malignancies or lymphoma.

In embodiments the inhibitor of TGF-β-activated kinase-1 (TAK1) for use according to the invention is administered at least 30 minutes after administration of CAR(19) T cell (CAR(19) T) based immunotherapy.

In embodiments CAR(19) T cell (CAR(19) T) based immunotherapy for use according to the invention, the subject has been diagnosed with and/or is suffering from cancer. In embodiments said cancer is a B-cell malignancy. Non-limiting examples of B-cell malignancies are described herein.

In each embodiment described herein the TAK1 inhibitor may also be used in the preventative treatment of a neuroinflammatory disorder and/or for preventing a neuroinflammatory disorder. Therefore, each use of a TAK1 inhibitor in the (therapeutic) treatment of a neuroinflammatory disorder described herein may additionally or alternatively comprise the use in the prevention (preventative treatment) of a neuroinflammatory disorder.

Each optional or preferred feature of the invention that is disclosed or described in the context of one aspect of the invention is herewith also disclosed in the context of the other aspects of the invention described herein.

All features disclosed in the context of the inhibitor of TGF-β-activated kinase-1 (TAK1) for use in the treatment and/or prevention of a neuroinflammatory condition in a subject according to the invention also relate to and are herewith disclosed also in the context of the methods of the invention, the method of treatment of the invention and the combination therapy for use in the treatment and/or prevention of cancer, preferably a B-cell malignancy, and vice versa.

### DETAILED DESCRIPTION OF THE INVENTION

All cited documents of the patent and non-patent literature are hereby incorporated by reference in their entirety.

The present invention is directed to a transforming growth factor-β-activated kinase-1 (TAK1) inhibitor for use in the treatment of a neuroinflammatory disorder.

As used herein, the term "subject' means a human or non-human animal selected for treatment or therapy. The subject or patient, such as the subject in need of treatment or prevention, may be an animal, a vertebrate animal, a mammal, a rodent, a murine, a canine, a feline, an equine, a primate, a simian, a monkey, an ape, or a human. The meaning of the terms "animal", "mammal", etc. is well known in the art and can, for example, be deduced from Wehner und Gehring (1995; Thieme Verlag). The term subject may also refer to a "patient" and may be used herein interchangeably therewith. In the context of this invention, it is particularly envisaged that animals are to be treated which are economically, agronomically or scientifically important. Preferably, the subject/patient is a mammal. More preferably, the subject/patient is a human.

In embodiments of the present invention, the subject that has received or is receiving a chimeric antigen receptor-modified T (CAR T) cells targeting CD19 (CAR19 T cell) therapy suffers from cancer, such as a cancer treatable by chimeric antigen receptor-modified T (CAR T) cells targeting CD19 (CAR19 T cell) therapy.

### Neuroinflammatory disorders

In embodiments the "neuroinflammatory disorder" comprises or consist of a condition selected from the group comprising microglia-mediated neuroinflammation, T-cell induced microglia-mediated neuroinflammation, cytokine-release syndrome (CRS), T-cell induced microglia-mediated neuroinflammation, a condition comprising elevated microglial activation, a neuroinflammatory disorder of the central nervous system (CNS), multiple sclerosis (MS), amyotrophic lateral sclerosis (ALS)/motor neuron disease (MND), immune effector cell-associated neurotoxicity-syndrome (ICANS), Acute disseminated encephalomyelitis (ADEM), Accute Optic Neuritis (AON), Transverse Myelitis Neuromyelitis Optica (NMO), Alzheimer's disease (AD) and Parkinson's disease (PD).

"Neuroinflammatory disorders" comprise diseases in which immune reactions damage components of the nervous system. Inflammatory mechanisms in neuroinflammatory disorders are also thought to be involved in the pathogenesis of many other CNS diseases, including neurodegenerative disorders. Common CNS diseases such as multiple sclerosis, amyotrophic lateral sclerosis, Alzheimer's and Parkinson's disease trigger a neuroinflammatory response that affects the brain and spinal cord. The immune and nervous systems use similar or the same mediators, receptors, and cells to regulate the immune and nervous systems and neuro-immune interactions. Neuroinflammatory disorders can also comprise autoimmune diseases, which may be, without limitation thereto, conditions in which the immune system mistakenly attacks a subject's own body, such as a subjects own nervous system and/or CNS. in autoimmune diseases dysregulated adaptive immunity (mediated by B and T lymphocytes) towards anatomical self-antigens commonly play a role.

In embodiments a patient is suffering from one or more of the symptoms selected from cognitive deficits, neurotoxicity, seizures, pathological headaches, increased blood brain barrier (BBB) permeability, partial or complete loss of eye sight, partial or complete loss of hearing, partial or complete loss of sensation, partial or complete paralysis, muscle weakness, memory loss, numbness in one or more limbs, tremor, paresis, confusion or disorientation.

"Cytokines" are cell signaling molecules which are secreted by certain immune and non-immune cells and can have an, either stimulating or inhibiting, effect on the immune system.

Cytokine release syndrome and neurotoxicity, which is also known as immune effector cell-associated neurotoxicity syndrome (ICANS) causes as most common neurological symptoms encephalopathy, headache, delirium, transient impairment of cognition, attention deficits, tremor, aphasia, and/or focal weakness (2, 4, 5). Cerebrospinal fluid (CSF) analyses in patients developing severe neurotoxicity revealed high levels of IL-6 (interleukin 6), IL-8 (interleukin 8), C-C Motif Chemokine Ligand 2 (CCL-2), and C-X-C Motif Chemokine Ligand 10 (CXCL-10) (1), suggesting that these may play a role in the pathophysiology. Furthermore, patients with severe ICANS can exhibit signs of endothelial activation and increased blood-brain barrier (BBB) permeability (6). The BBB disruption is considered to be caused by high concentrations of systemic cytokines in the CSF, thereby inducing brain vascular pericyte stress and secretion of endothelium-activating cytokines. ICANS can be caused by CAR T cell therapy. Monocyte-derived IL-1 (interleukin 1) and IL-6 are functionally involved in the development of CAR T cell-induced neurotoxicity (9). Presently corticosteroids are recommended as a first-line therapy for the treatment of ICANS (12), however such therapies diminish or abolish the anti-cancer effects of CAR T cell therapy, such that intervention with corticosteroids or glucocorticoids in patients experiencing ICANS after CAR T cell therapy, impairs or prevents the anti-cancer effect of the CAR T cell therapy. ICANS is therefore presently one of the major impairing factors of the success of CAR T cell-based anti-cancer therapy.

"Neurotoxicity" is a form of toxicity in which a biological, chemical or physical agent has an adverse effect on the structure or function of the central and/or peripheral nervous system. It occurs when exposure to a certain molecule alters the normal activity of the nervous system in a manner that causes permanent or reversible damage to nervous tissue. Neurotoxicity can be described by different grades and can be accompanied by fever and neurological symptoms. Mild encephalopathy can show symptoms such as the appearance of disorientation to time or place, or impaired attention or short-term memory with preserved alertness. Severe neurotoxicity can show symptoms such as mild somnolence, disorientation, impaired attention and difficulty naming before progressing to global aphasia, myoclonus, depressed level of consciousness, encephalopathy and seizures (1).

"Cytokine Release Syndrome" (CRS) is a systemic inflammatory response that can be induced by a variety of factors, including infections and certain drugs. CRS has been reported following infusion of several antibody-based therapies, donor stem cell transplantation, during graft-versus-host disease, as well as during T-cell-engaging therapies, such as CAR T-cell-based therapy.

### Immune cells

Immune cells as described herein relate to biological cells involved in the immune response in a subject. Immune cells are preferably selected from microglia cells, T Cells, B Cells, Dendritic Cells, Granulocytes, Innate Lymphoid Cells (ILCs), Megakaryocytes, Monocytes/Macrophages, Natural Killer (NK) Cells, Platelets, Red Blood Cells (RBCs) and/or Thymocytes.

The term "immune cells" comprises the MNCs comprised in blood, which may also be called peripheral blood mononuclear cell (PBMC). PBMCs comprise any peripheral blood cell having a round nucleus and consist mainly of lymphocytes (T cells, B cells, NK cells) and monocytes, whereas erythrocytes and platelets have no nuclei, and granulocytes (neutrophils, basophils, and eosinophils) have multi-lobed nuclei. In humans, lymphocytes (lymphoid cells) make up the majority of the PBMC population, followed by monocytes, and only a small percentage of dendritic cells. These cells can be extracted from whole blood using ficoll, a hydrophilic polysaccharide that separates layers of blood, and "density gradient centrifugation", which will separate the blood into a top layer of plasma, followed by a layer of PBMCs and a bottom fraction of polymorphonuclear cells (such as neutrophils and eosinophils) and erythrocytes. The polymorphonuclear cells can be further isolated by lysing the red blood cells. Basophils are sometimes found in both the denser and the PBMC fractions.

"Microglia" or microglia cells are the most abundant guard cells of the innate immune system of the central nervous system. Microglia are endowed with a high degree of plasticity that allows them to adopt diverse phenotypes and thereby perform multiple functions. In the healthy, mature central nervous system, microglia retain a highly branched morphology with a small soma and fine cell processes. Microglia cells monitor the surrounding microenvironment without disrupting neurovascular coupling. When brain homeostasis is disrupted, such as during injury or disease, microglia can ba activated leading to rapid and profound changes in their cell shapes, gene expression, and functional behavior. Activated microglia migrate to a CNS lesion and phagocytose cell debris or damaged cells. Activated microglia also drive neuroinflammation by secreting key inflammatory mediators, and by releasing a variety of substances that positively or negatively effect surrounding cells.

In preferred embodiments of the present invention leukocytes may particularly refer to lymphocytes, wherein leukocytes or lymphocytes.

"T cells" or T lymphocytes are a type of lymphocyte (a subtype of white blood cell) that plays a central role in cell-mediated immunity. They can be distinguished from other lymphocytes, such as B cells and natural killer cells, by the presence of a T-cell receptor on the cell surface. The several subsets of T cells each have a distinct function. T cell subtypes include, without limitation, T helper type 1 (Th1) cells, T helper type 2 (Th2) cells, T helper type 9 (Th9) cells, T helper type 17 (Th17) cells, T helper type 22 (Th22) cells, Follicular helper T (Tfh) cells, Regulatory T (Treg) cells, Natural killer T (NKT) cells, Gamma delta T cells, CD8+ cytotoxic T lymphocytes (CTLs). Further non-limiting embodiments of T cells include thymocytes, immature T lymphocytes, mature T lymphocytes, resting T lymphocytes, cytokine-induced killer cells (CIK cells) or activated T lymphocytes. Cytokine-induced killer (CIK) cells are typically CD3- and CD56-positive, non-major histocompatibility complex (MHC)-restricted, natural killer (NK)-like T lymphocytes. The T cell can be a CD4+ T cell, a cytotoxic T cell (CTL; CD8+ T cell), CD4+CD8+ T cell, CD4 CD8 T cell, or any other subset of T cells.

"Chimeric antigen receptors" (CARs) are also known as chimeric immune receptors, chimeric T-cell receptors, or artificial T-cell receptors. CARs are receptor proteins engineered to confer on T cells the novel ability to target a specific antigen. The receptors are chimeric in that they combine both antigen-binding and T cell-activating functions in a single receptor. CAR-T cell therapy uses T cells engineered with CARs to treat cancer. For "CAR T cell" or "CAR-T" immunotherapy T cells are engineered to recognize cancer cells in order to target and destroy them more effectively. CAR T cells can either be derived either from a patient's own blood (autologous) or from another donor (allogeneic). Isolated T cells are genetically engineered to express a specific CAR, which enables them to recognize an antigen specifically expressed by tumor cells and present on their surface. Said target antigen has to be absent in healthy cells. Upon infusion of CAR-T cells into a patient, they behave like a drug, fighting cancer cells. Once a CAR T cell comes into contact with its target antigen on a cells surface, the CAR-T cell binds to it and becomes activated, proliferates and induces cytotoxicity in the cancer cell. This cytotoxicity comprises several mechanisms, including increasing the release of factors that can affect other cells, such as cytokines, interleukins, and growth factors. In one exemplary and non-limiting example CAR T-cell therapy is considered a type of treatment in which a patient's T cells are changed in the laboratory so they will bind to cancer cells and kill them. White blood cells, including the T cells, are extracted from the blood of a patient by an apheresis machine. Subsequently the gene for a special receptor called a chimeric antigen receptor (CAR) is inserted *in vitro* into the isolated T cells. Millions of the CAR T cells are grown in the laboratory and then given back to the patient (autologous) or to another recipient (allogeneic) by infusion. The CAR T cells are able to bind to an antigen on the cancer cells of the recipient (e.g., a cancer patient) such that said cancer cells are killed (see e.g., US-NIH; www.cancer.gov).

CARs targeting CD19, a protein expressed by normal B cells and B-cells present in B-cell malignancies, can be used as therapy, for example, for B-cell leukemias and B-cell lymphomas (herein also termed "chimeric antigen receptor-modified T (CAR T) cells targeting CD19 (CAR19 T cell) therapy" or short "CAR19 T cell therapy"). Therefore, in preferred embodiments the terms "chimeric antigen receptor-modified T (CAR T) cell therapy" and "chimeric antigen receptor-modified T (CAR T) cells targeting CD19 (CAR19 T cell) therapy" may be used herein interchangeably or refer to each other or comprise each other. Therefore in embodiments aspects of the invention disclosed in the context of CAR T cell therapy are also disclosed in the context of CAR19 T cell therapy and vice versa.

The term "immunomodulatory functions" relates to functions or properties of molecules or cells that induce a changes or modulation in the function, action or status of any component of the immune system.

### Cancer

In the context of the present invention, the term "cancer" relates to the treatment of all kinds of cancer, independent of whether the cancer is associated with the formation of a solid tumor or whether the cancer cells do not form a solid tumor, as it is the case for most leukemias.

Cancer comprises a group of diseases that can affect any part of the body and is caused by abnormal cell growth and proliferation. These proliferating cells have the potential to invade the surrounding tissue and/or to spread to other parts of the body where they form metastasis. Worldwide, there were 14 million new cases of cancer and 8.2 million cancer related deaths in 2012 (World Cancer Report 2014). The majority of cancers is caused by environmental signals involving tobacco use, obesity and infections among others, while around 5-10% are genetic cases. Cancers can be classified into subcategories based on the cell of origin. The most common subcategories are carcinomas from epithelial cells, sarcomas from connective tissue and lymphomas and leukemias from hematopoietic cells. Cancer is associated with a high variety of local and systemic symptoms and cannot be cured in many cases. In light of the high number of new cancer patients and cancer related deaths novel treatment strategies are required.

Cancer according to the present invention refers to all types of cancer or neoplasm or malignant tumors found in mammals, including leukemias, sarcomas, melanomas and carcinomas. Either solid tumors and/or liquid tumors (such as leukemia or lymphoma) may be treated.

Melanomas include, but are not limited to include, for example, acral-lentiginous melanoma, amelanotic melanoma, benign juvenile melanoma, Cloudman's melanoma, S91 melanoma, Harding-Passey melanoma, juvenile melanoma, lentigo maligna melanoma, malignant melanoma, nodular melanoma, subungal melanoma, and superficial spreading melanoma.

Leukemias include, but are not limited to, haematological (B cell) malignancies, B-cell lymphoma, B-cell acute lymphoblastic leukaemia (B-ALL), precursor B-cell ALL, B-cell non-Hodgkin lymphoma (B-NHL), mixed lineage leukemia (MLL), large cell transformation of follicular lymphoma (trFL), Diffuse large B-cell lymphoma (DLBCL), Follicular lymphoma (FL), Chronic lymphocytic leukemia (CLL) /small lymphocytic lymphoma (SLL), mantle cell lymphoma (MCL), marginal zone lymphoma (MZL), Burkitt lymphoma (BL), lymphoplasmacytic lymphoma, hairy cell leukemia (HCL), primary central nervous system (CNS) lymphoma, primary intraocular lymphoma and monoclonal B-cell lymphocytosis (MBL), acute nonlymphocytic leukemia, chronic lymphocytic leukemia, acute granulocytic leukemia, chronic granulocytic leukemia, acute promyelocytic leukemia, adult T-cell leukemia, aleukemic leukemia, a leukocythemic leukemia, basophylic leukemia, blast cell leukemia, bovine leukemia, chronic myelocytic leukemia, leukemia cutis, embryonal leukemia, eosinophilic leukemia, Gross' leukemia, hairy-cell leukemia, hemoblastic leukemia, hemocytoblastic leukemia, histiocytic leukemia, stem cell leukemia, acute monocytic leukemia, leukopenic leukemia, lymphatic leukemia, lymphoblastic leukemia, lymphocytic leukemia, lymphogenous leukemia, lymphoid leukemia, lymphosarcoma cell leukemia, mast cell leukemia, megakaryocytic leukemia, micromyeloblastic leukemia, monocytic leukemia, myeloblastic leukemia, myelocytic leukemia, myeloid granulocytic leukemia, myelomonocytic leukemia, Naegeli leukemia, plasma cell leukemia, plasmacytic leukemia, promyelocytic leukemia, Rieder cell leukemia, Schilling's leukemia, stem cell leukemia, subleukemic leukemia, and undifferentiated cell leukemia.

Sarcomas include, but are not limited to a chondrosarcoma, fibrosarcoma, lymphosarcoma, melanosarcoma, myxosarcoma, osteosarcoma, Abernethy's sarcoma, adipose sarcoma, liposarcoma, alveolar soft part sarcoma, ameloblastic sarcoma, botryoid sarcoma, chloroma sarcoma, chorio carcinoma, embryonal sarcoma, Wilms' tumor sarcoma, endometrial sarcoma, stromal sarcoma, Ewing's sarcoma, fascial sarcoma, fibroblastic sarcoma, giant cell sarcoma, granulocytic sarcoma, Hodgkin's sarcoma, idiopathic multiple pigmented hemorrhagic sarcoma, immunoblastic sarcoma of B cells, lymphoma, immunoblastic sarcoma of T-cells, Jensen's sarcoma, Kaposi's sarcoma, Kupffer cell sarcoma, angiosarcoma, leukosarcoma, malignant mesenchymoma sarcoma, parosteal sarcoma, reticulocytic sarcoma, Rous sarcoma, serocystic sarcoma, synovial sarcoma, and telangiectaltic sarcoma.

Carcinomas include, but are not limited to acinar carcinoma, acinous carcinoma, adenocystic carcinoma, adenoid cystic carcinoma, carcinoma adenomatosum, carcinoma of adrenal cortex, alveolar carcinoma, alveolar cell carcinoma, basal cell carcinoma, carcinoma basocellulare, basaloid carcinoma, basosquamous cell carcinoma, bronchioalveolar carcinoma, bronchiolar carcinoma, bronchogenic carcinoma, cerebriform carcinoma, cholangiocellular carcinoma, chorionic carcinoma, colloid carcinoma, comedo carcinoma, corpus carcinoma, cribriform carcinoma, carcinoma en cuirasse, carcinoma cutaneum, cylindrical carcinoma, cylindrical cell carcinoma, duct carcinoma, carcinoma durum, embryonal carcinoma, encephaloid carcinoma, epiermoid carcinoma, carcinoma epitheliale adenoides, exophytic carcinoma, carcinoma exulcere, carcinoma fibrosum, gelatiniform carcinoma, gelatinous carcinoma, giant cell carcinoma, carcinoma gigantocellulare, glandular carcinoma, granulosa cell carcinoma, hair-matrix carcinoma, hematoid carcinoma, hepatocellular carcinoma, Hurthle cell carcinoma, hyaline carcinoma, hypernephroid carcinoma, infantile embryonal carcinoma, carcinoma in situ, intraepidermal carcinoma, intraepithelial carcinoma, Krompecher's carcinoma, Kulchitzky-cell carcinoma, large-cell carcinoma, lenticular carcinoma, carcinoma lenticulare, lipomatous carcinoma, lymphoepithelial carcinoma, carcinoma medullare, medullary carcinoma, melanotic carcinoma, carcinoma molle, mucinous carcinoma, carcinoma muciparum, carcinoma mucocellulare, mucoepidermoid carcinoma, carcinoma mucosum, mucous carcinoma, carcinoma myxomatodes, nasopharyngeal carcinoma, oat cell carcinoma, carcinoma ossificans, osteoid carcinoma, papillary carcinoma, periportal carcinoma, preinvasive carcinoma, prickle cell carcinoma, pultaceous carcinoma, renal cell carcinoma of kidney, reserve cell carcinoma, carcinoma sarcomatodes, schneiderian carcinoma, scirrhous carcinoma, carcinoma scroti, signet-ring cell carcinoma, carcinoma simplex, small-cell carcinoma, solanoid carcinoma, spheroidal cell carcinoma, spindle cell carcinoma, carcinoma spongiosum, squamous carcinoma, squamous cell carcinoma, string carcinoma, carcinoma telangiectaticurn, carcinoma telangiectodes, transitional cell carcinoma, carcinoma tuberosum, tuberous carcinoma, verrucous carcinoma, and carcinoma villosum.

Additional cancers include, but are not limited to Hodgkin's Disease, Non-Hodgkin's Lymphoma, multiple myeloma, neuroblastoma, breast cancer, ovarian cancer, lung cancer, rhabdomyosarcoma, primary thrombocytosis, primary macroglobulinemia, small-cell lung tumors, primary brain tumors, stomach cancer, colon cancer, malignant pancreatic insulanoma, malignant carcinoid, urinary bladder cancer, premalignant skin lesions, testicular cancer, lymphomas, thyroid cancer, esophageal cancer, genitourinary tract cancer, malignant hypercalcemia, cervical cancer, endometrial cancer, adrenal cortical cancer, and prostate cancer.

In some embodiments, "tumor" shall include, without limitation, a prostate tumor, a pancreatic tumor, a squamous cell carcinoma, a breast tumor, a melanoma, a basal cell carcinoma, a hepatocellular carcinoma, a choloangiocellular carcinoma, testicular cancer, a neuroblastoma, a glioma or a malignant astrocytic tumor such as glioblastma multiforme, a colorectal tumor, an endometrial carcinoma, a lung carcinoma, an ovarian tumor, a cervical tumor, an osteosarcoma, a rhabdo/leiomyosarcoma, a synovial sarcoma, an angiosarcoma, an Ewing sarcoma/PNET and a malignant lymphoma. These include primary tumors as well as metastatic tumors (both vascularized and non-vascularized).

### Therapeutic application of the invention

"Glucocorticosteroids", which are further called "glucocorticoids", "corticosteroids" or simply "steroids", are the presently most effective anti-inflammatory substances used in the treatment of chronic inflammatory, auto-immune and immune diseases. Glucocorticoids induce immunosuppression, which majorly comprises the decreases in the function and numbers of lymphocytes, of both B cells and T cells. Glucocorticoids are lipophilic hormones, which can be subclassified into glucocorticoids produced in the zona fasciculata of the adrenal cortex, mineralocorticoids produced in the zona glomerulosa, and sex hormones produced in the zona reticularis and to a great extent in the gonads. A variety of synthetic glucocorticoids are available for therapeutic use. Examples of glucocorticoids are cortisol (hydrocortisone), cortisone, prednisone, prednisolone, methylprednisolone and dexamethasone.

"TGF-β-activated kinase 1" or short "TAK1" is a serine/threonine kinase. TAK1 (MAP3K7) is a MAP3K family kinase involved in TGF-β1 signaling that can be activated by TGF-β1. Transforming growth factor-β (TGF-β) is a multifunctional cytokine that regulates a variety of cellular functions and has been shown to play an important role in inflammation. TAK1 plays also an important role as kinase upstream of NF-κB and is known to play a central role in promoting neuroinflammation in neurodegenerative diseases, and to be, for example, activated in active microglia. TAK1 can also be activated by various other stimuli, including proinflammatory cytokines such as tumor necrosis factor-a (TNF-α) and interleukin-1 (IL-1) and environmental stress. Phosphorylation of Thr-187 and Ser-192 in the activation loop of TAK1 leads to activation of TAK1. TAK1 activation induces activation of downstream signaling cascades, such as MKK3/6-p38 MAPK, MKK4/7-JNK, and nuclear factor-kappa B (NF-κB)-inducing kinase-IκB kinase.

A protein kinase inhibitor is a type of enzyme inhibitor that blocks the action of one or more protein kinases. Protein kinases are enzymes that phosphorylate a protein (attach a phosphate or PO₄ group) and thus can affect its function and/or conformation. The phosphate groups are usually attached to serine, threonine, or tyrosine amino acids of the protein: Most kinases act on both serine and threonine, tyrosine kinases act on tyrosine, and some kinases act on all three. There are also protein kinases that phosphorylate other amino acids, including histidine kinases that phosphorylate histidine residues. Protein phosphorylation is considered to regulate numerous biological processes. Protein kinase inhibitors can, for example, also be used to treat diseases, which are associated with or caused by hyperactive protein kinases (including mutated or overexpressed kinases in cancer) or to modulate cell function to overcome other disease factors.

A TAK1 inhibitor (TAKi) in the context of the present invention is preferably selected from the group comprising Takinib (EDHS-206), HS-276, Dehydroabietic acid, RGB-286638 free base, Sarsasapogenin, 5Z-7-Oxozeaenol, NG25 and pharmaceutically acceptable salts thereof.

In embodiments the TAK1 inhibitor binds to autophosphorylated and/or non-phosphorylated TAK1. In embodiments the TAK1 inhibitor binds to the ATP binding pocket of TAK1. In embodiments the TAK1 inhibitor binds to TAK1 in an ATP-competitive or in a non-competitive manner.

A "pharmaceutically acceptable salt" of a TAK1 inhibitor refers in the context of the present invention to a salt that is pharmaceutically acceptable and that possesses or retains the desired pharmacological activity, biological effectiveness and/or properties of the free bases of the associated TAK1 inhibitor (namely the parent compound).

Takinib (EDHS-206) is an orally active and selective TAK1 inhibitor (IC50=9.5 nM). Takinib is an inhibitor of autophosphorylated and non-phosphorylated TAK1 that binds within the ATP binding pocket of TAK1. Takinib wash shown to inhibit TAK1 by slowing down the rate-limiting step of TAK1 activation.

HS-276, a potent and highly selective (orally bioavailable) TAK1 inhibitor (Ki =2.5 nM; IC50 = 2.3 nM).

Dehydroabietic acid (DAA, DHAA), is a naturally occurring diterpene resin acid derived from coniferous plants such as *Pinus* and *Picea.* DAA has been shown to show anti-inflammatory effects via inhibition of TAK1 mediated pathways.

RGB-286638 free base is a Cyclin-dependent kinase (CDK) inhibitor that inhibits TAK1 with an IC50 of 5 nM.

Sarsasapogenin (SAR, Parigenin) is a steroidal sapogenin that inhibits TAK1 phosphorylation.

5Z-7-Oxozeaenol (CAS 66018-38-0) is a cell-permeable, highly potent, selective, irreversible, and ATP-competitive inhibitor of TAK1 activity (IC50 = 8 nM).

NG25 is an inhibitor of TAK1 with an enzymatic IC50 of 149 nM for TAK1.

"Immunotherapy" is a type of cancer treatment. It uses substances produced by the body or in the laboratory to strengthen the immune system and help the body find and destroy cancer cells. Immunotherapy can be used to treat many different types of cancer. It can be used alone or in combination with chemotherapy, other cancer treatments and/or other treatments. Immunotherapies for the treatment of cancer commonly comprise, without limitation thereto, (monoclonal) antibodies, CAR T-cell therapy, immune checkpoint inhibitors, cancer vaccines, immunomodulatory substances and/or cytokines.

According to the present invention, a "pharmaceutical combination" or "combination medicine" or "combination treatment or treatment" is the combined presence of a TAK1-inhibitor and a CAR T cell therapy according to the invention, in (timely) proximity to one another. In one embodiment, the combination is suitable for combined administration.

In one embodiment, the pharmaceutical combination, combination therapy or combination medication as described herein is characterized in that a TAK1-inhibitor is in a pharmaceutical composition in admixture with a pharmaceutically acceptable carrier, and the CAR T cell therapy is in a separate pharmaceutical composition in admixture with a pharmaceutically acceptable carrier. The combination medication/treatment/therapy or pharmaceutical combination of the present invention can therefore in some embodiments relate to the presence of two or three or even more separate compositions or dosage forms in proximity to each other. The therapeutic agents (and/or cells) in combination are not required to be present in a single composition.

### Combined administration

According to the present invention, the term "combined administration", otherwise known as coadministration or joint treatment, encompasses in some embodiments the administration of separate formulations of the compounds described herein, whereby treatment may occur within minutes of each other, in the same hour, on the same day, in the same week or in the same month as one another. Alternating administration of two agents is considered as one embodiment of combined administration. Staggered administration is encompassed by the term combined administration, whereby one agent may be administered, followed by the later administration of a second agent, optionally followed by administration of the first agent, again, and so forth. Simultaneous administration of multiple agents is considered as one embodiment of combined administration. Simultaneous administration encompasses in some embodiments, for example the taking of multiple compositions comprising the multiple agents at the same time, e.g., orally by ingesting separate tablets simultaneously. A combination medicament, such as a single formulation comprising multiple agents disclosed herein, and optionally additional anti-cancer and/or immuno-suppressing (e.g., steroids) or immuno-modulating medicaments, may also be used in order to co-administer the various components in a single administration or dosage.

In the context of the present invention combined administration or combined use may comprise in preferred embodiments the administration of a TAK1 inhibitor and a CAR T cell therapy to a sample/cells of the subject.

In embodiments the invention relates to a combined medication for use in the treatment of neuroinflammatory disorders, comprising (simultaneous or sequential) administration of a TAK1 inhibitor and one or more other therapies selected from the group comprising corticosteroids, Anti-IL-6 receptor antibodies, anti-IL-1beta antibodies, anti-IL-1beta receptor antagonists (Anakinra), calcineurin inhibitors (Cyclosporine A, tacrolimus), Interferon-beta, glatiramer acetate, dimethyl fumarate, diroximel fumarate, monomethyl fumarate, teriflunomid, fingolimod, fingolimod, fingolimod, mitoxantrone, ponesimod, ozanimod fingolimod, ofatumumab, alemtuzumab, ocrelizumab, natalizumab and ublituximabxiiy.

A combined therapy or combined administration of one agent may precede or follow treatment with the other agent to be combined, by intervals ranging from minutes to weeks. In embodiments where the second agent and the first agent are administered separately, one would generally ensure that a significant period of time did not expire between the time of each delivery, such that the first and second agents would still be able to exert an advantageously combined synergistic effect on a treatment site. In such instances, it is contemplated that one would contact the subject with both modalities within about 12-96 hours of each other and, in embodiments preferably, within about 6-48 hours of each other. In some situations, it may be desirable to extend the time period for treatment significantly, however, where several days (2, 3, 4, 5, 6 or 7) to several weeks (1, 2, 3, 4, 5, 6, 7, 8, 9, 10, 11 or 12) lapse between the respective administrations.

In embodiments, the therapeutic agent or combination medication is administered from day 1, 2, 3, 4, 5, 6 or day 7 of the occurrence of ICANS symptoms, or for 1, 2, 3, 4, 5, 6, 7, 8, 9, 10, 12, 14, 16, 18, 20, 22, 24, 26, 28, 30, 35, 40, 45 or 50 weeks, or from day 1 to at least 3 weeks, after occurrence of ICANS symptoms. In embodiments, the therapeutic agent or combination medication is administered between 1 - 5 days before the administration of the ICB therapy, or for 1, 2, 3, 4, 5, 6 or 7 days or 1, 2, 3, 4, 5, 6, 7, 8, 9, 10, 12, 14, 16, 18, 20, 22, 24, 26, 28, 30, 35, 40, 45 or 50 weeks before the administration of the present therapy. In preferred embodiments, the therapeutic agent or combination medication is administered between 1-14 days, more preferable for 1-5 days before CAR19 T cell therapy.

In the meaning of the invention, any form of administration of the multiple agents described herein is encompassed by combined administration, such that a beneficial additional therapeutic effect, preferably a synergistic effect, is achieved through the combined administration of a TAK1 inhibitor and CAR(19) T cell (CAR(19) T) based immunotherapy. In preferred embodiments said synergistic effect is a synergistic anti-cancer effect. In embodiments said synergistic effect is an (synergistically) increased anti-cancer effect of the CAR(19) T cell (CAR(19) T) based immunotherapy. In embodiments the (synergistically) increased anti-cancer effect is directed against a cancer that can be treated with or is susceptible to CAR(19) T cell (CAR(19) T) based immunotherapy.

It is understood that substituents and substitution patterns of the compounds described herein can be selected by one of ordinary skill in the art to provide compounds that are chemically stable and that can be readily synthesized by techniques known in the art and further by the methods set forth in this disclosure.

Another aspect of the disclosure includes "pharmaceutical compositions" prepared for administration to a subject and which include a "therapeutically effective amount" of one or more of the compounds disclosed herein. In certain embodiments, the pharmaceutical compositions are useful for treating immune effector cell-associated neurotoxicity-syndrome (ICANS) in a subject. The therapeutically effective amount of a disclosed compound (e.g., agent, substance, blood sample or cells) will depend on the route of administration, the species of subject and the physical characteristics of the subject being treated. Specific factors that can be taken into account include disease severity and stage, weight, diet and concurrent medications. The relationship of these factors to determining a therapeutically effective amount of the disclosed compounds is understood by those of skill in the art.

Pharmaceutical compositions for administration to a subject can include at least one further pharmaceutically acceptable additive such as carriers, thickeners, diluents, buffers, preservatives, surface active agents and the like in addition to the molecule of choice. Pharmaceutical compositions can also include one or more additional active ingredients such as antimicrobial agents, anti-inflammatory agents, anesthetics, and the like. The pharmaceutically acceptable carriers useful for these formulations are conventional. Remington's Pharmaceutical Sciences, by E. W. Martin, Mack Publishing Co., Easton, PA, 19th Edition (1995), describes compositions and formulations suitable for pharmaceutical delivery of the compounds herein disclosed.

In general, the nature of the carrier will depend on the particular mode of administration being employed. For instance, parenteral formulations usually contain injectable fluids that include pharmaceutically and physiologically acceptable fluids such as water, physiological saline, balanced salt solutions, aqueous dextrose, glycerol or the like as a vehicle. For solid compositions (for example, powder, pill, tablet, or capsule forms), conventional non-toxic solid carriers can include, for example, pharmaceutical grades of mannitol, lactose, starch, or magnesium stearate. In addition to biologically-neutral carriers, pharmaceutical compositions to be administered can contain minor amounts of non-toxic auxiliary substances, such as wetting or emulsifying agents, preservatives, and pH buffering agents and the like, for example sodium acetate or sorbitan monolaurate.

The pharmaceutical compositions can be administered by intramuscular, intraocular, subcutaneous, intravenous, intra-arterial, intra-articular, intraperitoneal, intrathecal, intracerebroventricular, or parenteral routes. Optionally, compositions can be administered to subjects by a variety of mucosal administration modes, including by oral, rectal, intraocular, intranasal, intrapulmonary, or transdermal delivery, or by topical delivery to other surfaces.

The compositions according to the invention can alternatively contain as pharmaceutically acceptable carrier substances as required to approximate physiological conditions, such as pH adjusting and buffering agents, tonicity adjusting agents, wetting agents and the like, for example, sodium acetate, sodium lactate, sodium chloride, potassium chloride, calcium chloride, sorbitan monolaurate, and triethanolamine oleate. For solid compositions, conventional nontoxic pharmaceutically acceptable vehicles can be used which include, for example, pharmaceutical grades of mannitol, lactose, starch, magnesium stearate, sodium saccharin, talcum, cellulose, glucose, sucrose, magnesium carbonate, and the like.

In accordance with the treatment methods according to the invention, the compound can be delivered to a subject in a manner consistent with conventional methodologies associated with management of the disorder for which treatment or prevention is sought. In accordance with the disclosure herein, a prophylactically or therapeutically effective amount of the compound and/or other biologically active agent is administered to a subject in need of such treatment for a time and under conditions sufficient to prevent, inhibit, and/or ameliorate a selected disease or condition or one or more symptom(s) thereof.

"Administration of and "administering a" compound should be understood to mean providing a compound, a prodrug of a compound, a combination of at least two compounds or a pharmaceutical composition as described herein. The compound(s) or composition(s) can be administered by another person to the subject (e.g., intravenously) or it can be self-administered by the subject (e.g., tablets).

Dosage can be varied by the attending clinician to maintain a desired concentration at a target site (for example, the lungs or systemic circulation). Higher or lower concentrations can be selected based on the mode of delivery, for example, intravenous or subcutaneous delivery, trans-epidermal, rectal, oral, pulmonary, or intranasal delivery. Dosage can also be adjusted based on the release rate of the administered formulation, for example, of an intrapulmonary spray versus powder, sustained release oral versus injected particulate or transdermal delivery formulations, and so forth.

The present invention also relates to a method of treatment of subjects suffering from the medical conditions disclosed herein. The method of treatment comprises preferably the administration of a therapeutically effective amount of a compound disclosed herein to a subject in need thereof.

A "therapeutically effective amount" refers to a quantity of a specified compound sufficient to achieve a desired effect in a subject being treated with said compound. For example, this may be the amount of a compound disclosed herein useful in treating a condition of immune effector cell-associated neurotoxicity-syndrome (ICANS) in a subject. The therapeutically effective amount or diagnostically effective amount of a compound will be dependent on the subject being treated, the severity of the affliction, and the manner of administration of the therapeutic composition. Dosage regimens can be adjusted to provide an optimum prophylactic or therapeutic response. A therapeutically effective amount is also one in which any toxic or detrimental side effects of the compound and/or other biologically active agent is outweighed in clinical terms by therapeutically beneficial effects.

A non-limiting range for a therapeutically effective amount of a TAK1-inhibitior and/or other biologically active agent within the methods and formulations of the disclosure is about 0.001 mg/kg body weight to 50 mg/kg body weight, 0.01 mg/kg body weight to about 20 mg/kg body weight, such as about 0.05 mg kg to about 5 mg/kg body weight, or about 0.2 mg/kg to about 2 mg/kg body weight, or about 1*10^4 cells/kg body weight.

Examples of Takinib dosages regimens were analyzed in Scarneo et al., 2019 (Scarneo SA, Eibschutz LS, Bendele PJ, Yang KW, Totzke J, Hughes P, Fox DA, Haystead TAJ. Pharmacological inhibition of TAK1, with the selective inhibitor takinib, alleviates clinical manifestation of arthritis in CIA mice. Arthritis Res Ther. 2019 Dec 17;21(1):292. doi: 10.1186/s13075-019-2073-x. PMID: 31847895; PMCID: PMC6918687).

A non-limiting range for a therapeutically effective amount of CAR19 T cells, such as e.g., CAR(19) T cells (CAR(19) Ts), is about 1*10^3 cells/kg body weight, about 5*10^3 cells/kg body weight, about 1*10^4 cells/kg body weight, about 5*10^4 cells/kg body weight, about 1*10^5 cells/kg body weight, about 5*10^5 cells/kg body weight, about 1*10^6 cells/kg body weight, about 5*10^6 cells/kg body weight, about 1*10^7 cells/kg body weight, about 5*10^7 cells/kg body weight, or about 1*10^8 cells/kg body weight or more.

As used herein, the term "approximately" or "about" is used to describe and account for small variations. For example, the term may refer to less than or equal to 10, such as less than or equal down to 1, when appropriate, also the term may refer to more than or equal to 10, such as more than or equal up to 100 or more, when appropriate. It is to be understood that range format is used for the sake of simplicity and brevity and is to be flexibly understood to include numeric values expressly stated as boundaries of a range, encompassing each numeric value and sub-ranges.

The instant disclosure also includes kits, packages and multi-container units containing the herein described pharmaceutical compositions, active ingredients, and/or means for administering the same for use in the prevention and treatment of diseases and other conditions described herein in mammalian subjects.

### REFERENCES

1. B. D. Santomasso, Park, J.H., Salloum, D., Riviere I, Flynn J, Mead E, Halton E, Wang X, Senechal B, Purdon T, Cross JR, Liu H, Vachha B, Chen X, DeAngelis LM, Li D, Bernal Y, Gonen M, Wendel HG, Sadelain M, Brentjens RJ., Clinical and Biological Correlates of Neurotoxicity Associated with CAR T-cell Therapy in Patients with B-cell Acute Lymphoblastic Leukemia. Cancer Discov. 8, 958-971 (2018).
2. C. Belin, Devic, P., Ayrignac X, Dos Santos A, Paix A, Sirven-Villaros L, Simard C, Lamure S, Gastinne T, Ursu R, Berger C, Platon L, Tessoulin B, Azoulay E, Wallet F, Thieblemont C, Bachy E, Cartron G, Laplaud DA, CarpentierAF., Description of neurotoxicity in a series of patients treated with CAR T-cell therapy. Scientific reports 10, 18997 (2020).
3. R. C. Sterner, Sterner, R.M., Immune effector cell associated neurotoxicity syndrome in chimeric antigen receptor-T cell therapy. Front Immunol. 13, 879608 (2022).
4. D. B. Rubin, Danish HH, Ali AB, Li K, LaRose S, Monk AD, Cote DJ, Spendley L, Kim AH, Robertson MS, Torre M, Smith TR, Izzy S, Jacobson CA, Lee JW, Vaitkevicius H., Neurological toxicities associated with chimeric antigen receptor T-cell therapy. Brain. 142, 1334-1348 (2019).
5. J. Rice, Nagle, S., Randall J, Hinson HE., Chimeric Antigen Receptor T Cell-Related Neurotoxicity: Mechanisms, Clinical Presentation, and Approach to Treatment. Curr Treat Options Neurol. 21, 40 (2019).
6. J. Gust, Hay, K.A., Hanafi LA, Li D, Myerson D, Gonzalez-Cuyar LF, Yeung C, Liles WC, Wurfel M, Lopez JA, Chen J, Chung D, Harju-Baker S, Ozpolat T, Fink KR, Riddell SR, Maloney DG, Turtle CJ., Endothelial Activation and Blood-Brain Barrier Disruption in Neurotoxicity after Adoptive Immunotherapy with CD19 CAR-T Cells. Cancer Discov. 12, 1404-1419 (2017).
7. K. R. Parker, Migliorini D, Perkey E, Yost KE, Bhaduri A, Bagga P, Haris M, Wilson NE, Liu F, Gabunia K, SchollerJ, Montine TJ, Bhoj VG, Reddy R, Mohan S, Maillard I, Kriegstein AR, June CH, Chang HY, Posey AD Jr, Satpathy AT., Single-Cell Analyses Identify Brain Mural Cells Expressing CD19 as Potential Off-Tumor Targets for CAR-T Immunotherapies. Cell 183, 126-142 (2020).
8. Q. Deng, Han G, Puebla-Osorio N, Ma MCJ, Strati P, Chasen B, Dai E, Dang M, Jain N, Yang H, Wang Y, Zhang S, Wang R, Chen R, Showell J, Ghosh S, Patchva S, Zhang Q, Sun R, Hagemeister F, Fayad L, Samaniego F, Lee HC, Nastoupil LJ, Fowler N, Eric Davis R, Westin J, Neelapu SS, Wang L, Green MR. , Characteristics of anti-CD19 CAR T cell infusion products associated with efficacy and toxicity in patients with large B cell lymphomas. Nature medicine 12, 1878-1887 (2020).
9. M. Norelli, Camisa, B., Barbiera G, Falcone L, Purevdorj A, Genua M, Sanvito F, Ponzoni M, Doglioni C, Cristofori P, Traversari C, Bordignon C, Ciceri F, Ostuni R, Bonini C, Casucci M, Bondanza A., Monocyte-derived IL-1 and IL-6 are differentially required for cytokine-release syndrome and neurotoxicity due to CAR T cells. Nature medicine 24, 739-748 (2018).
10. T. Giavridis, van der Stegen SJC, Eyquem J, Hamieh M, Piersigilli A, Sadelain M., CAR T cell-induced cytokine release syndrome is mediated by macrophages and abated by IL-1 blockade. Nature medicine 24, 731-738 (2018).
11. R. M. Sterner, Sakemura R, Cox MJ, Yang N, Khadka RH, Forsman CL, Hansen MJ, Jin F, Ayasoufi K, Hefazi M, Schick KJ, Walters DK, Ahmed O, Chappell D, Sahmoud T, Durrant C, Nevala WK, Patnaik MM, Pease LR, Hedin KE, Kay NE, Johnson AJ, Kenderian SS., GM-CSF inhibition reduces cytokine release syndrome and neuroinflammation but enhances CAR-T cell function in xenografts. Blood 133, 697-709 (2019).
12. D. W. Lee, Santomasso BD, Locke FL, Ghobadi A, Turtle CJ, Brudno JN, Maus MV, Park JH, Mead E, Pavletic S, Go WY, Eldjerou L, Gardner RA, Frey N, Curran KJ, Peggs K, Pasquini M, DiPersio JF, van den Brink MRM, Komanduri KV, Grupp SA, Neelapu SS., ASTCT Consensus Grading for Cytokine Release Syndrome and Neurologic Toxicity Associated with Immune Effector Cells. Biol Blood Marrow Transplant. 4, 625-638 (2019).
13. P. Strati, Ahmed S, Furqan F, Fayad LE, Lee HJ, lyer SP, Nair R, Nastoupil LJ, Parmar S, Rodriguez MA, Samaniego F, Steiner RE, Wang M, Pinnix CC, Horowitz SB, Feng L, Sun R, Claussen CM, Hawkins MC, Johnson NA, Singh P, Mistry H, Johncy S, Adkins S, Kebriaei P, Shpall EJ, Green MR, Flowers CR, Westin J, Neelapu SS., Prognostic impact of corticosteroids on efficacy of chimeric antigen receptor T-cell therapy in large B-cell lymphoma. Blood 137, 3272-3276 (2021).
14. R. Zeiser, Lee, S.J., Three US Food and Drug Administration-approved therapies for chronic GVHD. Blood 139, 1642-1645 (2022).
15. R. Zeiser, Polverelli, N., Ram R, Hashmi SK, Chakraverty R, Middeke JM, Musso M, Giebel S, Uzay A, Langmuir P, Hollaender N, Gowda M, Stefanelli T, Lee SJ, Teshima T, Locatelli F, for the REACH3 Investigators., Ruxolitinib for Glucocorticoid-Refractory Chronic Graft-versus-Host Disease. N Eng J Med 385, 228-238 (2021).
16. R. Zeiser, von Bubnoff, N, Butler J, Mohty M, Niederwieser D, Or R, SzerJ, Wagner EM, Zuckerman T, Mahuzier B, Xu J, Wilke C, Gandhi KK, Socié G, for the REACH2 Trial Group., Ruxolitinib for Glucocorticoid-Refractory Acute Graft-versus-Host Disease. N Eng J Med 382, 1800-1810 (2020).
17. M. Prinz, Jung S, PrillerJ., Microglia Biology: One Century of Evolving Concepts. Cell 179, 292-311 (2019).
18. M. Prinz, Masuda T, Wheeler MA, Quintana FJ., Microglia and Central Nervous System-Associated Macrophages-From Origin to Disease Modulation. Annu Rev Immunol 39, 251-277 (2021).
19. P. d'Errico, Ziegler-Waldkirch S, Aires V, Hoffmann P, Mezö C, Erny D, Monasor LS, Liebscher S, Ravi VM, Joseph K, Schnell O, Kierdorf K, Staszewski O, Tahirovic S, Prinz M, Meyer-Luehmann M., Microglia contribute to the propagation of Aβ into unaffected brain tissue. Nat Neurosci. 25, 20-25 (2022).
20. T. Goldmann, Wieghofer, P., Jordão, M.J., Prutek, F., Hagemeyer, N., Frenzel, K., et al., Origin, fate and dynamics of macrophages at central nervous system interfaces. Nat Immunol. 17, 797-805 (2016).
21. N. R. Mathew, Vinnakota, J.M., Apostolova, P., Erny, D., Hamarsheh, S., Andrieux, G., Kim, J., Hanke, K., Goldmann, T., Chappell-Maor, L., El-Khawanky, N., Ihorst, G., Schmidt, D., Duyster, J., Finke, J., Blank, T., Boerries, M., Blazar, B.R., Jung, S., Prinz, M., Zeiser, R., Graft-versus-host disease of the CNS is mediated by TNF upregulation in microglia. The Journal of clinical investigation 130, 1315-1329 (2020).
22. Y. Hao, Hao S, Andersen-Nissen E, Mauck WM 3rd, Zheng S, Butler A, Lee MJ, Wilk AJ, Darby C, Zager M, Hoffman P, Stoeckius M, Papalexi E, Mimitou EP, Jain J, Srivastava A, Stuart T, Fleming LM, Yeung B, Rogers AJ, McElrath JM, Blish CA, Gottardo R, Smibert P, Satija R., Integrated Analysis of Multimodal Single-Cell Data. Cell 184, 3573-3587.e3529 (2021).
23. M. J. C. Jordão, Sankowski, R., Brendecke, S.M., Sagar, Locatelli G, Tai YH, Tay TL, Schramm E, Armbruster S, Hagemeyer N, Groß O, Mai D, Çiçek O, Falk T, Kerschensteiner M, Grün D, Prinz M., Single-cell profiling identifies myeloid cell subsets with distinct fates during neuroinflammation. Science 363, 6425 (2019).
24. A. Nagai, Terashima, M., Sheikh AM, Notsu Y, Shimode K, Yamaguchi S, Kobayashi S, Kim SU, Masuda J., Involvement of cystatin C in pathophysiology of CNS diseases. Front. bioscience. 13, 3470-3479 (2008).
25. D. P. Schafer, Lehrman EK, Kautzman AG, Koyama R, Mardinly AR, Yamasaki R, Ransohoff RM, Greenberg ME, Barres BA, Stevens B., Microglia sculpt postnatal neural circuits in an activity and complement-dependent manner. Neuron 74, 691-705 (2012).
26. D. T. Teachey, Lacey SF, Shaw PA, Melenhorst JJ, Maude SL, Frey N, et al., Identification of Predictive Biomarkers for Cytokine Release Syndrome after Chimeric Antigen Receptor T cell Therapy for Acute Lymphoblastic Leukemia. Cancer Discovery 6, 664-679 (2017).
27. M. H. Xiu, Man LJ, Wang D, Du X, Yin G, Zhang Y, Tan YL, Chen N, Chen S, Teixeira AL, Cassidy RM, Soares JC, Zhang XY., Tumor necrosis factor-alpha -1031T/C polymorphism is associated with cognitive deficits in chronic schizophrenia patients versus healthy controls. Am J Med Genet B Neuropsychiatr Genet. 177, 379-387 (2018).
28. R. Medeiros, Prediger RD, Passos GF, Pandolfo P, Duarte FS, Franco JL, Dafre AL, Di Giunta G, Figueiredo CP, Takahashi RN, Campos MM, Calixto JB., Connecting TNF-alpha signaling pathways to iNOS expression in a mouse model of Alzheimer's disease: relevance for the behavioral and synaptic deficits induced by amyloid beta protein. J Neurosci. 27, 5394-5404 (2007).
29. R. Maldonado-Ruiz, Trujillo-Villarreal LA, Montalvo-Martinez L, Mercado-Gómez OF, Arriaga-Avila V, Garza-Ocañas L, Ortiz-López R, Garza-Villarreal EA, Guevara-Guzmán R, Camacho-Morales A., MCP-1 Signaling Disrupts Social Behavior by Modulating Brain Volumetric Changes and Microglia Morphology. Mol Neurobiol. 59, 932-949 (2022).
30. T. K. Ulland, Song, W.M., Huang, S.C.;,Ulrich, J.D., Sergushichev, A., Beatty, W.L., TREM2 Maintains Microglial Metabolic Fit-ness in Alzheimer's Disease. Cell 170, 649-663.e613 (2017).
31. Y. Li, Long, W., Gao M, Jiao F, Chen Z, Liu M, Yu L., TREM2 Regulates High Glucose-Induced Microglial Inflammation via the NLRP3 Signaling Pathway. Brain Sci. 11, 896 (2021).
32. L. Zhong, Chen, X.F., Wang T, Wang Z, Liao C, Wang Z, Huang R, Wang D, Li X, Wu L, Jia L, Zheng H, Painter M, Atagi Y, Liu CC, Zhang YW, Fryer JD, Xu H, Bu G., Soluble TREM2 induces inflammatory responses and enhances microglial survival. J Exp Med. 214, 597-607 (2017).
33. N. Manouchehri, Hussain RZ, Cravens PD, Esaulova E, Artyomov MN, Edelson BT, Wu GF, Cross AH, Doelger R, Loof N, EagarTN, ForsthuberTG, Calvier L, Herz J, Stüve O., CD11c+CD88+CD317+ myeloid cells are critical mediators of persistent CNS autoimmunity. Proc Natl AcadSci U S A 118, e2014492118 (2021).
34. E. Haroon, Miller, A.H., Sanacora G., Inflammation, Glutamate, and Glia: A Trio of Trouble in Mood Disorders. Neuropsychopharmacology 42, 193-215 (2016).
35. L. Volk, Chiu SL, Sharma K, Huganir RL., Glutamate synapses in human cognitive disorders. Annu Rev Neurosci. 38, 127-149 (2015).
36. S. Raud, Sütt S, Luuk H, Plaas M, Innos J, Kõks S, Vasar E., Relation between increased anxiety and reduced expression of alpha1 and alpha2 subunits of GABA(A) receptors in Wfs1-deficient mice. Neurosci Lett. 460, 138-142 (2009).
37. J. P. Bohnsack, Hughes BA, O'Buckley TK, Edokpolor K, Besheer J, Morrow AL., Histone deacetylases mediate GABAA receptor expression, physiology, and behavioral maladaptations in rat models of alcohol dependence. Neuropsychopharmacology. 43, 1518-1529 (2018).
38. H. Pribiag, Stellwagen D., TNF-α downregulates inhibitory neurotransmission through protein phosphatase 1-dependent trafficking of GABA(A) receptors. J Neurosci 33, 15879-15893 (2013).
39. L. F. Chen, Lyons MR, Liu F, Green MV, Hedrick NG, Williams AB, Narayanan A, Yasuda R, West AE., The NMDA receptor subunit GluN3A regulates synaptic activity-induced and myocyte enhancer factor 2C (MEF2C)-dependent transcription. J Biol Chem. 295, 8613-8627 (2020).
40. R. Yadav, Gupta SC, Hillman BG, Bhatt JM, Stairs DJ, Dravid SM., Deletion of glutamate delta-1 receptor in mouse leads to aberrant emotional and social behaviors. PLoS One 7, e32969 (2012).
41. E. Spangenberg, Severson PL, Hohsfield LA, Crapser J, Zhang J, Burton EA, Zhang Y, Spevak W, Lin J, Phan NY, Habets G, Rymar A, Tsang G, Walters J, Nespi M, Singh P, Broome S, Ibrahim P, Zhang C, Bollag G, West BL, Green KN., Sustained microglial depletion with CSF1R inhibitor impairs parenchymal plaque development in an Alzheimer's disease model. Nat Commun. 10, 3758 (2019).
42. C. Ouyang, Nie, L., Gu M, Wu A, Han X, Wang X, Shao J, Xia Z. Transforming growth factor (TGF)-β-activated kinase 1 (TAK1) activation requires phosphorylation of serine 412 by protein kinase A catalytic subunit α (PKACα) and X-linked protein kinase (PRKX). Transforming growth factor (TGF)-β-activated kinase 1 (TAK1) activation requires phosphorylation of serine 412 by protein kinase A catalytic subunit α (PKACα) and X-linked protein kinase (PRKX). J Biol Chem. 289, 24226-24237 (2014).
43. X. Liu, Yao, M., Li N, Wang C, Zheng Y, Cao X., CaMKII promotes TLR-triggered proinflammatory cytokine and type I interferon production by directly binding and activating TAK1 and IRF3 in macrophages. Blood 112, 4961-4970 (2008).
44. M. Matsumoto-Ida, Takimoto Y, Aoyama T, Akao M, Takeda T, Kita T., Activation of TGF-beta1-TAK1-p38 MAPK pathway in spared cardiomyocytes is involved in left ventricular remodeling after myocardial infarction in rats. Am J Physiol Heart Circ Physiol. 290, H709-715 (2006).
45. J. Totzke, Gurbani D, Raphemot R, Hughes PF, Bodoor K, Carlson DA, Loiselle DR, Bera AK, Eibschutz LS, Perkins MM, Eubanks AL, Campbell PL, Fox DA, Westover KD, Haystead TAJ, Derbyshire ER., Takinib, a Selective TAK1 Inhibitor, Broadens the Therapeutic Efficacy of TNF-α Inhibition for Cancer and Autoimmune Disease. Cell Chem Biol. 24, 1029-1039.e1027 (2017).
46. P. M. Panipinto, Singh AK, Shaikh FS, Siegel RJ, Chourasia M, Ahmed S., Takinib Inhibits Inflammation in Human Rheumatoid Arthritis Synovial Fibroblasts by Targeting the Janus Kinase-Signal Transducer and Activator of Transcription 3 (JAK/STAT3) Pathway. Int J Mol Sci. 22, 12580 (2021).
47. T. Goldmann, Wieghofer, P., Müller, P.F., Wolf, Y., Varol, D., Yona, S., Brendecke, S.M., Kierdorf, K., Staszewski, O., Datta, M., Luedde, T., Heikenwalder, M., Jung, S., Prinz, M., A new type of microglia gene targeting shows TAK1 to be pivotal in CNS autoimmune inflammation. Nat Neurosci. 16, 1618-1626 (2013).
48. R. Sankowski, Böttcher C, Masuda T, Geirsdottir L, Sagar, Sindram E, Seredenina T, Muhs A, Scheiwe C, Shah MJ, Heiland DH, Schnell O, Grün D, Priller J, Prinz M., Mapping microglia states in the human brain through the integration of high-dimensional techniques. Nat Neurosci. 22, 2098-2110 (2019).
49. P. Casellas, S. Galiegue, A. S. Basile, Peripheral benzodiazepine receptors and mitochondrial function. Neurochem Int 40, 475-486 (2002).
50. A. C. Kuhlmann, T. R. Guilarte, Cellular and Subcellular Localization of Peripheral Benzodiazepine Receptors After Trimethyltin Neurotoxicity. Journal of neurochemistry 74, 1694-1704 (2002).
51. M. Ikawa et al., <sup>11</sup>C-ER176, a Radioligand for 18-kDa Translocator Protein, Has Adequate Sensitivity to Robustly Image All Three Affinity Genotypes in Human Brain. Journal of Nuclear Medicine 58, 320-325 (2017).
52. M. Unterrainer, Mahler, C., Vomacka L, Lindner S, Havla J, Brendel M, Böning G, Ertl-Wagner B, Kümpfel T, Milenkovic VM, Rupprecht R, Kerschensteiner M, Bartenstein P, Albert NL., TSPO PET with [18F]GE-180 sensitively detects focal neuroinflammation in patients with relapsing-remitting multiple sclerosis. Eur J Nucl Med Mol Imaging. 45, 1423-1431 (2018).
53. W. C. Kreisl et al., A genetic polymorphism for translocator protein 18 kDa affects both in vitro and in vivo radioligand binding in human brain to this putative biomarker of neuroinflammation. Journal of cerebral blood flow and metabolism : official journal of the International Society of Cerebral Blood Flow and Metabolism 33, 53-58 (2013).
54. V. S. Sheth, Gauthier J., Taming the beast: CRS and ICANS after CAR T-cell therapy for ALL. Bone Marrow Transplant 56, 552-566 (2021).
55. M. C. Bosman, Schepers, H., Jaques, J., et al., The TAK1-NF-κB axis as therapeutic target for AML. Blood 124, 3130-3140 (2014).
56. P. F. Caimi, Pacheco Sanchez G, Sharma A, Otegbeye F, Ahmed N, Rojas P, Patel S, Kleinsorge Block S, Schiavone J, Zamborsky K, Boughan K, Hillian A, Reese-Koc J, Maschan M, Dropulic B, Sekaly RP, de Lima M. , Prophylactic Tocilizumab Prior to Anti-CD19 CAR-T Cell Therapy for Non-Hodgkin Lymphoma. Front Immunol. 12, 745320 (2021).
57. M. Nakajima, Kawaguchi, M., Ota K, Fujita J, Matsukura S, Huang SK, Morishima Y, Ishii Y, Satoh H, Sakamoto T, Hizawa N. , L-17F induces IL-6 via TAK1-NFκB pathway in airway smooth muscle cells. Immun Inflamm Dis. 5, 124-131 (2017).
58. A. R. Klatt, Klinger G, Neumüller O, Eidenmüller B, Wagner I, Achenbach T, Aigner T, Bartnik E., TAK1 downregulation reduces IL-1beta induced expression of MMP13, MMP1 and TNF-alpha. Biomed Pharmacother. 60, 55-61 (2006).
59. Y. R. Xu, Lei CQ., TAK1-TABs Complex: A Central Signalosome in Inflammatory Responses. Front Immunol. 11, 608976. (2021).
60. N. Celarain, Sánchez-Ruiz de Gordoa J, Zelaya MV, Roldán M, Larumbe R, Pulido L, Echavarri C, Mendioroz M., TREM2 upregulation correlates with 5-hydroxymethycytosine enrichment in Alzheimer's disease hippocampus. Clin Epigenetics. 8, 37 (2016).
61. L. Best, Ghadery C, Pavese N, Tai YF, Strafella AP., New and Old TSPO PET Radioligands for Imaging Brain Microglial Activation in Neurodegenerative Disease. Curr Neurol Neurosci Rep. 19, 24 (2019).
62. C. Tronel, Largeau B, Santiago Ribeiro MJ, Guilloteau D, Dupont AC, Arlicot N., Molecular Targets for PET Imaging of Activated Microglia: The Current Situation and Future Expectations. Int J Mol Sci. 18, 802 (2017).
63. P. Karschnia, Strübing F, Teske N, Blumenberg V, Bücklein VL, Schmidt C, Schöberl F, Dimitriadis K, Forbrig R, Stemmler HJ, Tonn JC, von Bergwelt-Baildon M, Subklewe M, von Baumgarten L., Clinicopathologic Findings in Fatal Neurotoxicity After Adoptive Immunotherapy With CD19-Directed CAR T-Cells. Hemasphere 5, e533 (2021).
64. J. Gust, Finney OC, Li D, Brakke HM, Hicks RM, Futrell RB, Gamble DN, Rawlings-Rhea SD, Khalatbari HK, Ishak GE, Duncan VE, Hevner RF, Jensen MC, Park JR, Gardner RA., Glial injury in neurotoxicity after pediatric CD19-directed chimeric antigen receptor T cell therapy. Ann Neurol. 86, 42-54 (2019).
65. C. Palleis et al., In Vivo Assessment of Neuroinflammation in 4-Repeat Tauopathies. Mov Disord 36, 883-894 (2021).
66. Z. I. Kolabas et al., Multi-omics and 3D-imaging reveal bone heterogeneity and unique calvaria cells in neuroinflammation. bioRxiv, 2021.2012.2024.473988 (2021).
12a.J. N. Kochenderfer, Yu Z, Frasheri D, Restifo NP, Rosenberg SA., Adoptive transfer of syngeneic T cells transduced with a chimeric antigen receptor that recognizes murine CD19 can eradicate lymphoma and normal B cells. Blood 116, 3875 (2010).
13a. A. Kobayashi, Kobayashi, S., Miyai, K., Osawa, Y., Horiuchi, T., Kato, S., et al., TAK1 inhibition ameliorates survival from graft-versus-host disease in an allogeneic murine marrow transplantation model. Int J Hematol. 107, 222 (2018).
14a. T. Goldmann, Wieghofer, P., Jordão, M.J., Prutek, F., Hagemeyer, N., Frenzel, K., et al., Origin, fate and dynamics of macrophages at central nervous system interfaces. Nat Immunol. 17, 797 (2016).
15a. H. Qin, Ishii, K., Nguyen S, Su PP, Burk CR, Kim BH, Duncan BB, Tarun S, Shah NN, Kohler ME, Fry TJ., Murine pre-B-cell ALL induces T-cell dysfunction not fully reversed by introduction of a chimeric antigen receptor. Blood 132, 1899 (2018).
16a. R. Zeiser, Nguyen, V.H., Beilhack, A., Buess, M., Schulz, S., Baker, J., Contag, C.H., Negrin, R.S., Inhibition of CD4+CD25+ regulatory T cell function by calcineurin dependent interleukin-2 production. Blood 108, 390 (2006).
17a. M. Leger, Quiedeville, A., Bouet, V., Haelewyn B, Boulouard M, Schumann-Bard P, Freret T., Object recognition test in mice. Nat Protoc. 12, 2531 (2013).
18a. N. R. Mathew, Vinnakota, J.M., Apostolova, P., Erny, D., Hamarsheh, S., Andrieux, G., Kim, J., Hanke, K., Goldmann, T., Chappell-Maor, L., El-Khawanky, N., Ihorst, G., Schmidt, D., Duyster, J., Finke, J., Blank, T., Boerries, M., Blazar, B.R., Jung, S., Prinz, M., Zeiser, R., Graft-versus-host disease of the CNS is mediated by TNF upregulation in microglia. The Journal of clinical investigation 130, 1315 (2020).
19a. R. Mazziotti, Baroncelli, L., Ceglia, N., Chelini, G., Sala, G.D., Magnan, C., et al., Mir-132/212 is required for maturation of binocular matching of orientation preference and depth perception. Nat Commun. 8, 15488 (2017).
20a. G. van Loo, De Lorenzi, R., Schmidt, H., Huth, M., Mildner, A., Schmidt-Supprian, M., Lassmann, H., Prinz, M.R., Pasparakis, M., Inhibition of transcription factor NF-kappaB in the central nervous system ameliorates autoimmune encephalomyelitis in mice. Nat Immunol. 7, 954 (2006).
21a. A. F. McDavid, G; Yajima, M. (2022).
22a. H. H. Jeppson, H; Cook, D. (2021).
23a. K. R. Blighe, S; Lewis, M, EnhancedVolcano: Publication-ready volcano plots with enhanced colouring and labeling. R package version 1.10.0. https://github.com/kevinblighe/EnhancedVolcano. (2021).
24a. T. Wu et al., clusterProfiler4.0: A universal enrichment tool for interpreting omics data. Innovation (Camb) 2, 100141 (Aug 28, 2021).

### FIGURES

The invention is further described by the following figures. These are not intended to limit the scope of the invention but represent preferred embodiments of aspects of the invention provided for greater illustration of the invention described herein.

### Description of the figures:

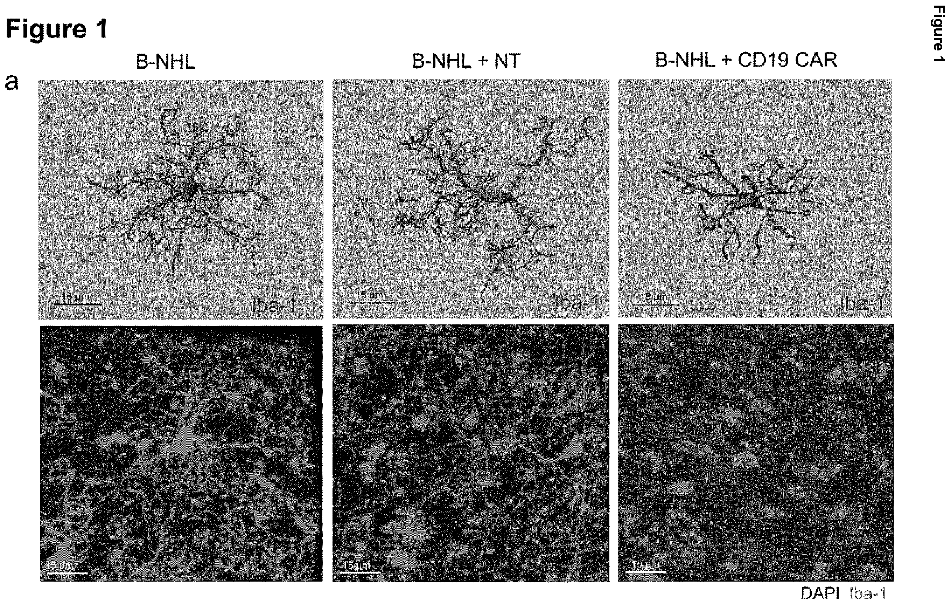

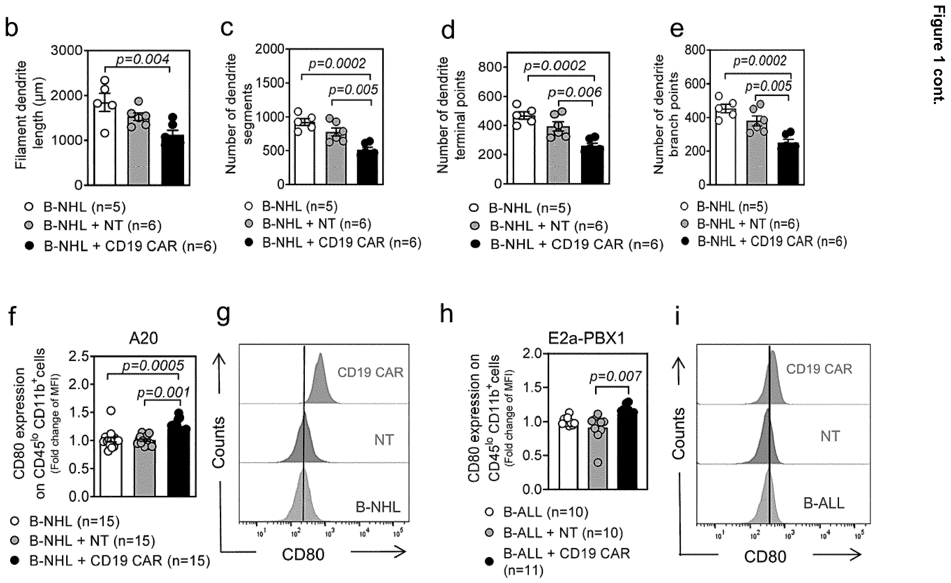

**Figure 1****: Microglia are activated upon transfer of CD19 CART cells into B-NHL bearing mice**
   (a) Representative images show Imaris (Bitplane)-based 3D-reconstruction of lba-1⁺ microglia from the cortex of B-NHL bearing mice treated with PBS (no T cells), NT T cells or CD19 CAR T cells, as indicated. Scale bar: 15µm. **(b-e)** Scatter plots depict Imaris-based automated quantification of microglial morphology including filament dendrite length, number of dendrite segments, number of dendrite branch points and number of dendrite terminal points from the microglia (in the cortex of CNS) of B-NHL (A20) bearing mice treated with either PBS (n=5), NT T cells (n=6) or CD19 CAR T cells (n=6), as indicated. The experiment was performed twice and results (mean ± s.e.m.) were pooled. *P*-values were calculated using one-way ANOVA. **(f)** The scatter plot shows the quantification (fold change of MFI) of CD80 expression on microglia (CD45^{lo} CD11b⁺) from the CNS of B-NHL (A20) bearing mice treated with either PBS (n=15), NT T cells (n=15) or CD19 CAR T cells (n=15) on day 10 after B-NHL transfer. The experiment was performed four times and results (mean ± s.e.m.) were pooled. *P*-values were calculated using one-way ANOVA. **(g)** The representative flow cytometry plot shows CD80 expression on microglia (CD45^{lo} CD11b⁺) from the CNS of B-NHL (A20) bearing mice treated with either PBS, NT T cells or CD19 CAR T cells as indicated. **(h)** The scatter plot shows the quantification (fold change of MFI) and representative flow cytometry plot shows CD80 expression on microglia (CD45^{lo} CD11b⁺) from the CNS of B-ALL (E2A-PBX1) bearing mice treated with either PBS (n=10), NT T cells (n=10) or CD19 CAR T cells (n=11). The experiment was repeated three times and results (mean ± s.e.m.) were pooled. *P*-values were calculated using one-way ANOVA. (i) The representative flow cytometry plot shows CD80 expression on microglia (CD45^{lo} CD11b⁺) from the CNS of B-ALL (E2A-PBX1) bearing mice treated with either PBS, NT T cells or CD19 CAR T cells as indicated.

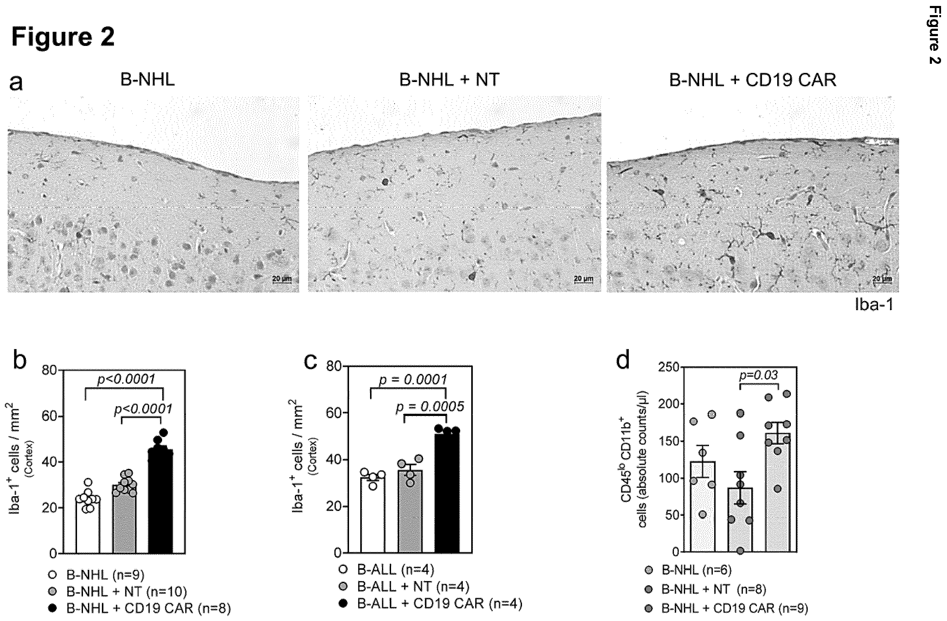

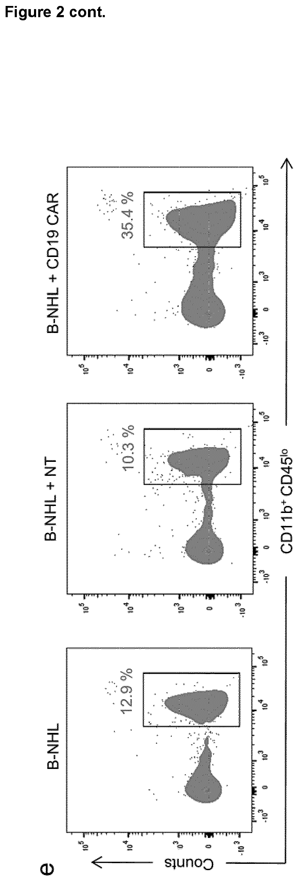

**Figure 2****: Increase of lba1+ microglia upon CD19 CAR T cells treatment**
   **(a-c)** Histology of brain samples immunostained for lba-1⁺ cells from the cortex of B-NHL or B-ALL bearing mice treated with CD19 CAR T cells or NT T cells. **(a)** Representative images from each group are shown. Scale bars, 20µm. **(b)** The scatter plots show the number of lba-1⁺ cells (per mm²) in cortex of B-NHL bearing mice treated with PBS or NT or CD19 CART cells. The experiment was performed two times. The results (mean ± s.e.m.) were pooled. The *P*-values were calculated using the unpaired student's t-test. **(c)** The scatter plots show the number of lba-1⁺ cells (per mm²) in cortex of B-ALL bearing mice treated with PBS or NT or CD19 CAR T cells. The experiment was performed once. The *P*-values were calculated using the unpaired student's t-test. **(d-e)** The scatter plot **(d)** shows the quantification (absolute counts) of microglia (CD45^{Io} CD11b⁺) isolated from the CNS of B-NHL bearing mice treated with PBS or NT or CD19 CAR T cells. **(e)** A representative flow cytometry plot from each group is shown. The experiment was performed two times. The results (mean ± s.e.m.) were pooled. The *P*-values were calculated using the student's *t*-test.

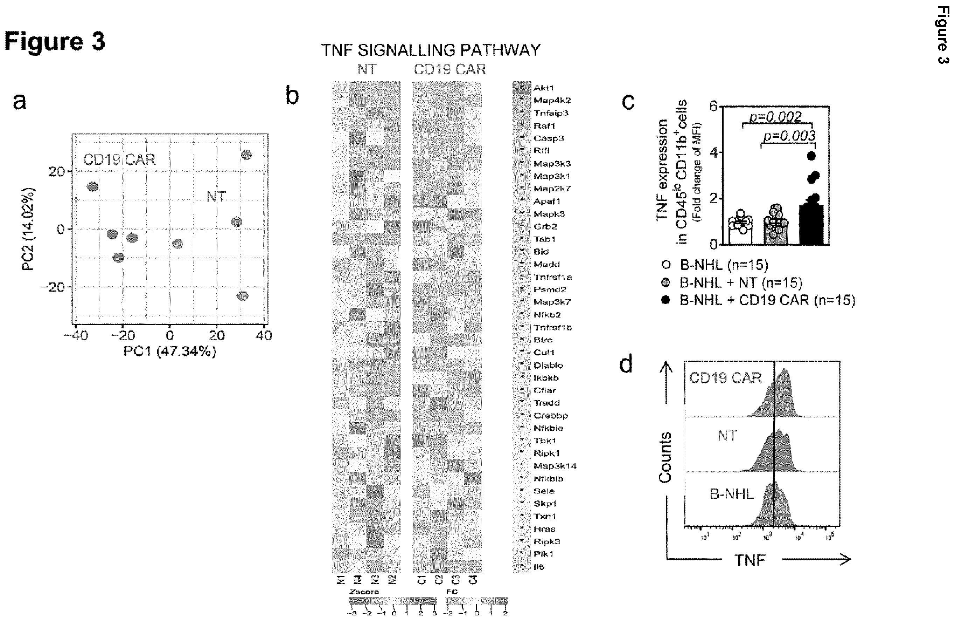

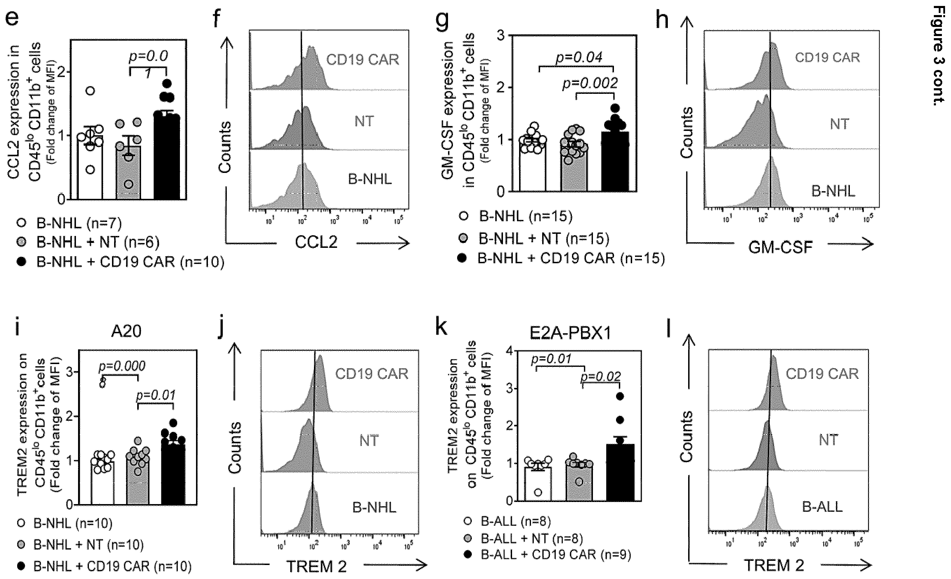

**Figure 3****: CD19 CAR T cells induce upregulation of cytokines and activation markers in microglia**
   **(a)** Principal component (PC) analysis of microarray data derived from sorted microglia isolated from the CNS of B-NHL (A20) bearing mice treated with either NT T cells (n=4) or CD19 CAR T cells (n=4). **(b)** Heat map of microarray data showing the significantly regulated genes (40) involved in the TNF pathway isolated from sorted microglia from the CNS of B-NHL (A20) bearing mice treated with either NT T cells (n=4) or CD19 CAR T cells (n=4). Color codes represent the Z-score log2 intensity (left panel) and log2 fold change (right panel). **(c, d)** The scatter plot **(c)** shows the quantification (fold change of MFI) of TNF in microglia (CD45^{lo} CD11b⁺) from the CNS of B-NHL (A20) bearing mice treated with either PBS (n=15), NT T cells (n=15) or CD19 CAR T cells (n=15). The experiment was performed four times. The results (mean ± s.e.m.) were pooled. The *P*-values were calculated using one-way ANOVA. **(d)** Representative flow cytometry plot showing TNF expression in microglia of the indicated groups. **(e, f)** The scatter plot **(e)** shows the quantification (fold change of MFI) of CCL-2 in microglia (CD45^{lo} CD11b⁺) from the CNS of B-NHL (A20) bearing mice treated with either PBS (n=7), NT T cells (n=6) or CD19 CAR T cells (n=10). The experiment was performed three times. The results (mean ± s.e.m.) were pooled.The *P*-values were calculated using one-way ANOVA. **(f)** Representative flow cytometry plot showing CCL-2 expression in microglia of the indicated groups. **(g, h)** The scatter plot **(g)** shows the quantification (fold change of MFI) of GM-CSF in microglia (CD45^{lo} CD11b⁺) from the CNS of B-NHL (A20) bearing mice treated with either PBS (n=15), NT T cells (n=15) or CD19 CAR T cells (n=15). The experiment was performed four times. The results (mean ± s.e.m.) were pooled. The *P*-values were calculated using one-way ANOVA. **(h)** Representative flow cytometry plot showing GM-CSF expression in microglia of the indicated groups. **(i, j)** The scatter plot **(i)** shows the quantification (fold change of MFI) of TREM2 on microglia (CD45^{lo} CD11b⁺) from the CNS of B-NHL (A20) bearing mice treated with either PBS (n=10), NT T cells (n=10) or CD19 CAR T cells (n=10). The experiment was performed three times. The results (mean ± s.e.m.) were pooled. *P*-values were calculated using one-way ANOVA. **(j)** Representative flow cytometry plot showing TREM2 expression in microglia of the indicated groups. **(k, I)** The scatter plot **(k)** shows the quantification (fold change of MFI) of TREM2 on microglia (CD45^{lo} CD11b⁺) from the CNS of B-ALL (E2A-PBX1) bearing mice treated with either PBS (n=8), NT T cells (n=8) or CD19 CAR T cells (n=9). The experiment was performed two times. The results (mean ± s.e.m.) were pooled. P-values were calculated using one-way ANOVA. **(I)** Representative flow cytometry plot showing TREM2 expression on microglia of the indicated groups.
**Figure 4****: Microglial depletion reduces the CD19 CAR T cell induced cognitive impairment**
   **(a)** The scatter plot shows the percentage of open arm entries made by B-ALL bearing mice treated with PBS (n=10), NT (n=11) or CD19 CAR (n=12) T cells in an elevated plus maze with respect to the total time. The experiment was performed three times. *P*-values were calculated using one-way ANOVA. **(b)** The scatter plot shows the percentage of time spent by B-ALL bearing mice treated with PBS (n=10), NT (n=11) orCD19 CAR (n=12) cells in exploring a novel object in a novel object recognition test. The experiment was performed three times. The results *P*-values were calculated using one-way ANOVA. **(c)** The scatter plot shows the grip strength normalized to body weight (N) of B-ALL bearing mice treated with either PBS (n=10), NT (n=11) or CD19 CAR (n=12) in a grip strength test. The experiment was performed three times. The *P*-values were calculated using one-way ANOVA. **(d)** Heat map of microarray data showing the top genes regulated in the *postsynaptic neurotransmitter receptor activity* isolated from sorted microglia of B-NHL (A20) bearing mice treated with either NT T cells (n=4) or CD19 CAR T cells (n=4). Color codes (shades of grey) represents the Z-score log2 intensity log2 intensity (left panel) and log2 fold change (right panel). **(e)** Scatter plot represents the quantification (fraction) of intravascular and extravascular fluorescence of vessels from the CNS of B-ALL bearing mice treated with either NT T cells (n=5) or CD19 CAR T cells (n=5). **(f)** The scatter plot shows the percentage of open arm entries made by B-ALL bearing mice treated with CD19 CAR T cells that were on a control diet (n=9) or CSF1R inhibitor diet (n=10) in an elevated plus Maze Test. The experiment was performed twice. The *P-*values were calculated using one-way ANOVA. **(g)** The scatter plot shows the percentage of time spent by B-ALL bearing mice treated with CD19 CAR T cells and maintained on either a control diet (n=9) or CSF1R inhibitor diet (n=10) in exploring a novel object in the novel object recognition test. The experiment was performed twice. *P*-values were calculated using one-way ANNOVA. **(h)** The scatter plot shows the grip strength normalized to body weight (N) of B-ALL bearing mice treated with CD19 CAR T cells and fed with either a control diet (n=9) or CSF1R inhibitor diet (n=10) in a grip strength test. The experiment was performed twice. *P*-values were calculated using one-way ANOVA.

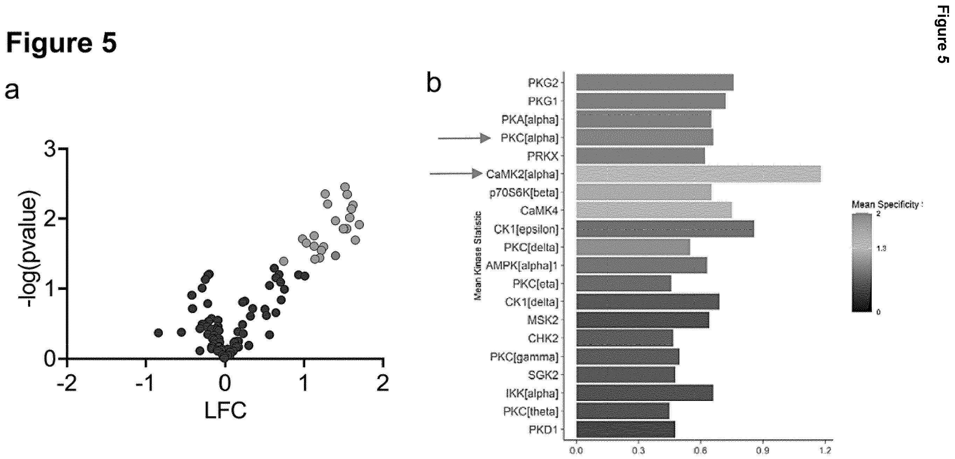

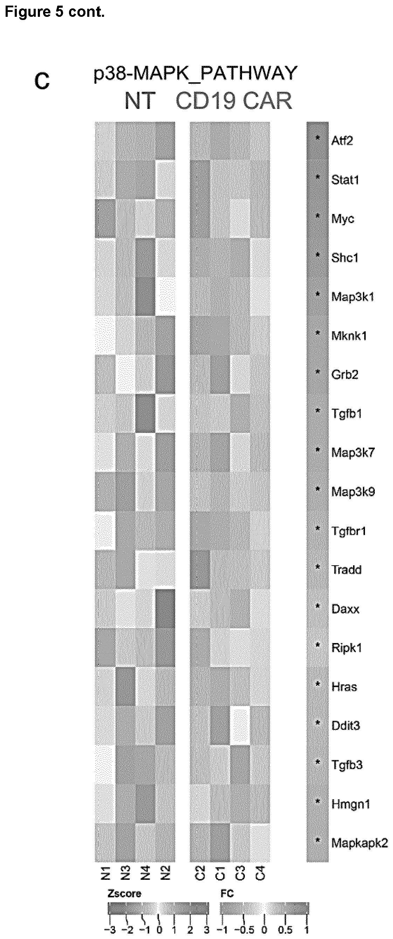

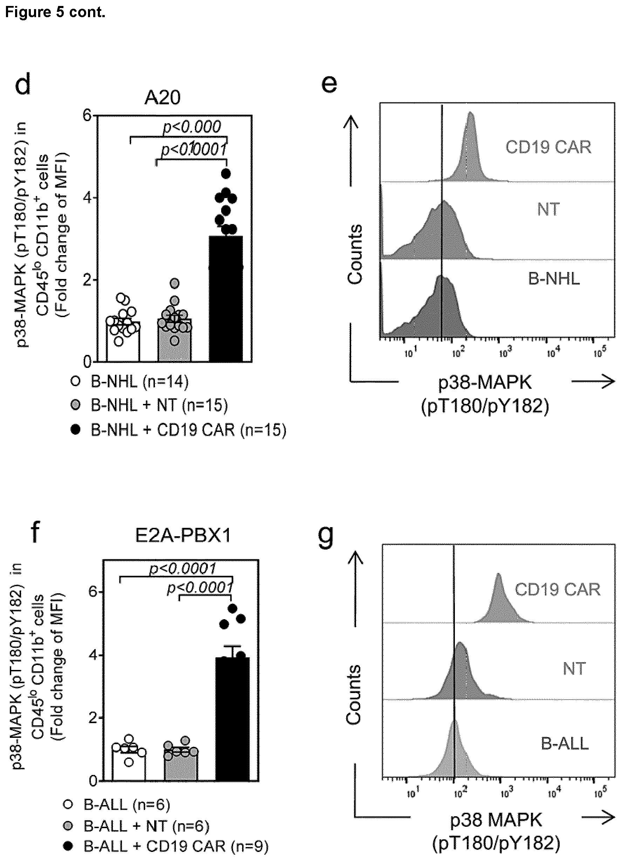

**Figure 5****: p38-MAPK activation in microglia upon CAR19 T cell transfer**
   **(a)** The volcano plot visualizes the result of the kinase activity arrays by plotting - for each test - the effect size (x-axis, LFC or delta) versus significance (y-axis, -log10 (pvalue)) of the test. Red spots are peptides that exhibit a significant difference between B-NHL (A20) bearing mice treated with CD19 CAR T cells compared to NT treated controls (p<0.05). **(b)** The bar plot shows kinases that are predicted to be more frequently phosphorylated from sorted microglia (CD45^{lo} CD11b⁺) of B-NHL (A20) bearing mice treated with CD19 CAR T cells compared to the NT T cell control mice. **(c)** Heat map based on microarray showing the significantly regulated genes involved in p38-MAPK pathway isolated from sorted microglia (CD45^{lo} CD11b⁺) from the CNS of B-NHL (A20) bearing mice treated with either NT T cells or CD19 CAR T cells. Color codes represents the Z-score log2 intensity (left panel) and log2 fold change (right panel). **(d, e)** The scatter plot **(d)** shows the quantification (fold change of MFI) of p38-MAPK expression in microglia (CD45^{lo} CD11b⁺) from the CNS of B-NHL (A20) bearing mice treated with either PBS (n=14) or NT (n=15) or CD19 CAR (n=15) T cells. The experiment was performed five times and results (mean ± s.e.m.) were pooled. *P*-values were calculated using one-way ANNOVA. **(e)** Representative flow cytometry plot of p38-MAPK (phospho) expression in microglia of the indicated groups. **(f, g)** The scatter plot **(f)** shows the quantification (fold change of MFI) of p38-MAPK (phospho) expression in microglia (CD45^{lo} CD11b⁺) from the CNS of B-ALL (E2A-PBX1) bearing mice treated with PBS (n=6), NT (n=6) or CD19 CAR T cells (n=9). The experiment was performed twice and results (mean ± s.e.m.) were pooled. *P*-values were calculated using one-way ANNOVA. **(g)** Representative flow cytometry plot of p38-MAPK expression in microglia of the indicated groups.

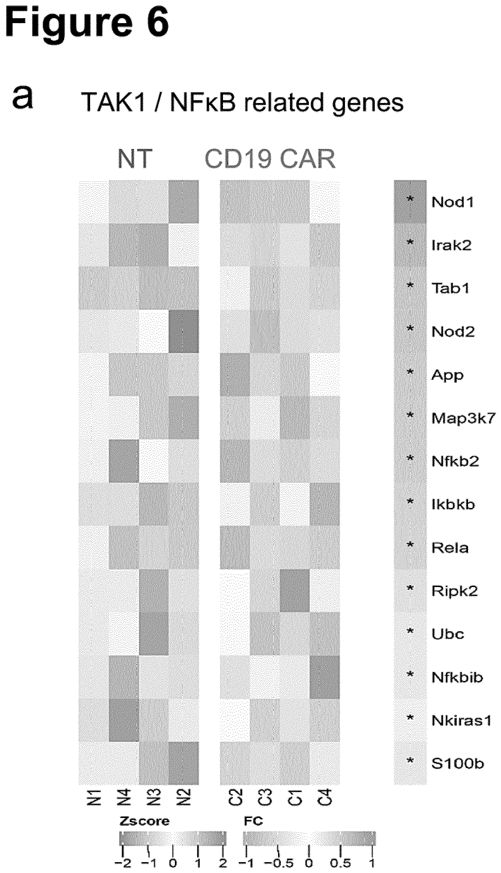

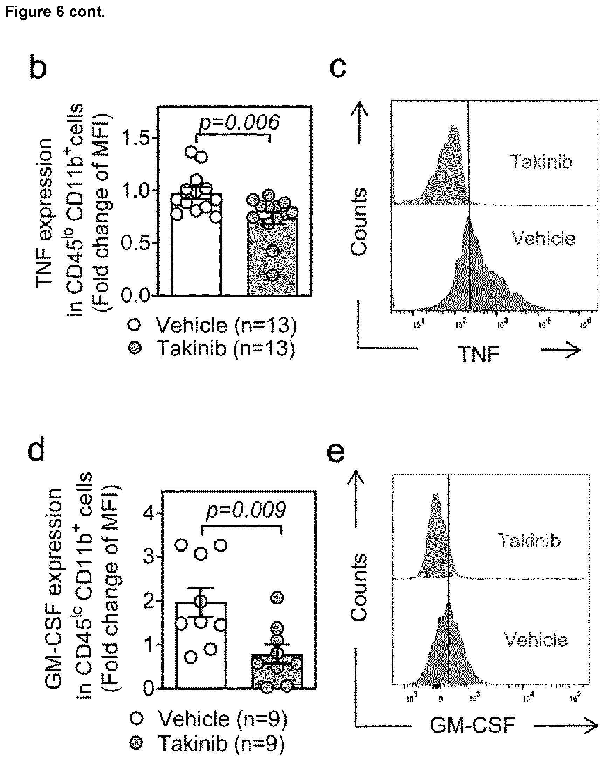

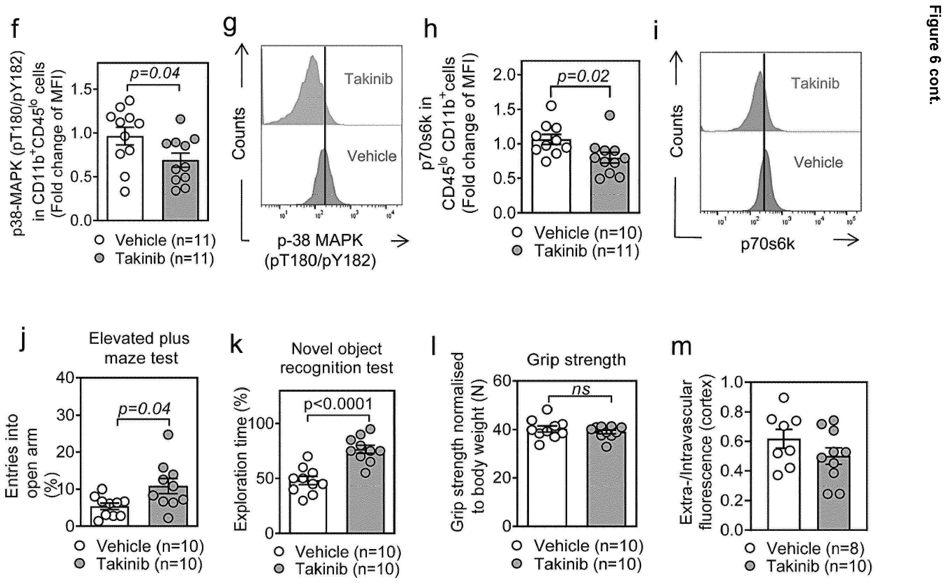

**Figure 6****: Takinib reduces microglial activation and improves cognitive function**
   **(a)** Heatmap based on microarray depicting the significantly regulated genes (*) related to the TAK1 pathway from sorted microglia of B-NHL (A20) bearing mice treated with either NT (n=4) or CD19 CAR (n=4) T cells. Color codes represents the Z-score log2 intensity. The experiment was performed once. **(b, c)** The scatter plot **(b)** shows the quantification (fold change of MFI) of TNF expression in microglia (CD45^{lo} CD11b⁺) from the CNS of B-NHL (A20) bearing mice treated with CD19 CAR T cells. As indicated, mice received intraperitoneal vehicle (n=13) or takinib (n=13) injection at a dosage of 0.15 mg/kg per mouse per day from day 3 to day 9 following B-NHL transfer. Experiments were performed three times and results (mean ± s.e.m.) were pooled. The *P*-values were calculated using an unpaired t-test. **(c)** Representative flow cytometry plot forTNF expression in microglia of the indicated groups. **(d, e)** The scatter plot **(d)** shows the quantification (fold change of MFI) of GM-CSF expression in microglia (CD45^{lo} CD11b⁺) from the CNS of B-NHL (A20) bearing mice treated with CD19 CAR T cells. As indicated, mice received intraperitoneal vehicle (n=9) or takinib (n=9) injection at a dosage of 0.15 mg/kg per mouse per day from day 3 to day 9 following B-NHL transfer. Experiments were performed three times and results (mean ± s.e.m.) were pooled. *P*-values were calculated using an unpaired t-test. **(e)** Representative flow cytometry plot for GM-CSF expression in microglia of the indicated groups. **(f, g)** The scatter plot **(f)** shows the quantification (fold change of MFI) of p38-MAPK (phospho) expression in microglia (CD45^{lo} CD11b⁺) from the CNS of B-NHL (A20) bearing mice treated with CD19 CAR T cells. As indicated, mice received intraperitoneal vehicle (n=11) or takinib (n=11) injection at a dosage of 0.15 mg/kg per mouse per day from day 3 to day 9 following B-NHL transfer. Experiments were performed three times and results (mean ± s.e.m.) were pooled. *P*-values were calculated using an unpaired t-test. **(g)** Representative flow cytometry plot for p38-MAPK expression in microglia of the indicated groups. **(h, i)** The scatter plot **(h)** shows the quantification (fold change of MFI) of p70s6k expression in microglia (CD45^{lo} CD11b⁺) from the CNS of B-NHL (A20) bearing mice treated with CD19 CAR T cells. Mice received intraperitoneal vehicle (n=11) or takinib (n=11) injection at a dosage of 0.5 mg/kg per mouse per day as indicated from day 3 to day 9 following B-NHL transfer. Experiments were performed three times and results (mean ± s.e.m.) were pooled. P-values were calculated using an unpaired t-test. **(i)** Representative flow cytometry plot for p70s6k expression in microglia of the indicated groups. **(j)** The scatter plot shows the percentage of open arm entries made by B-ALL (E2A-PBX1) bearing mice treated with either CD19 CAR T cells + vehicle (n=10) or CD19 CAR T cells + 0.5 mg/ml of takinib (n=10) with respect to the total time in an elevated plus maze test. Experiments were performed two times and results (mean ± s.e.m.) were pooled. *P-*values were calculated using an unpaired t test. **(k)** The scatter plot shows the percentage of time spent by B-ALL (E2A-PBX1) bearing mice treated with either CD19 CAR T cells + vehicle (n=10) or CD19 CAR T cells + 0.5 mg/ml of takinib (n=10) in exploring a novel object in a novel object recognition test. Experiments were performed two times and results (mean ± s.e.m.) were pooled. P-values were calculated using an unpaired t-test. **(I)** The scatter plot shows the grip strength normalized to body weight of B-ALL (E2A-PBX1) bearing mice treated with either CD19 CAR T cells + vehicle (n=10) or CD19 CAR T cells + 0.5 mg/ml of takinib (n=10) in a grip strength test. Experiments were performed two times and results (mean ± s.e.m.) were pooled. *P*-values were calculated using unpaired t-test. **(m)** Scatter plot represents the quantification (fraction) of intravascular and extravascular fluorescence from CNS of B-ALL bearing mice treated with either CD19 CAR T cells + vehicle (n=10) or CD19 CAR T cells + 0.5 mg/ml of takinib (n=10). Experiments were performed two times and results (mean ± s.e.m.) were pooled. *P*-values were calculated using unpaired t-test.

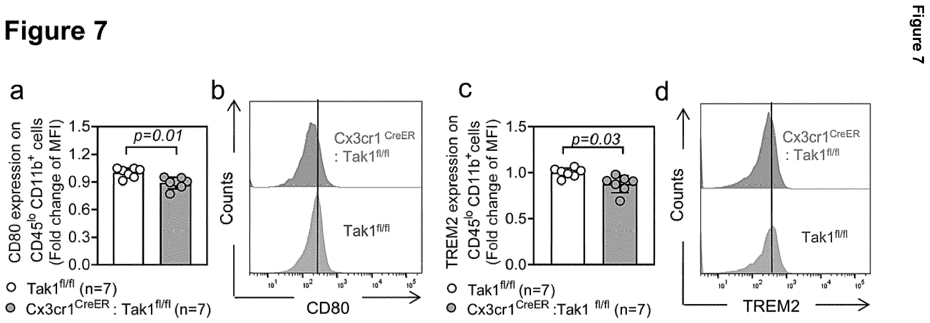

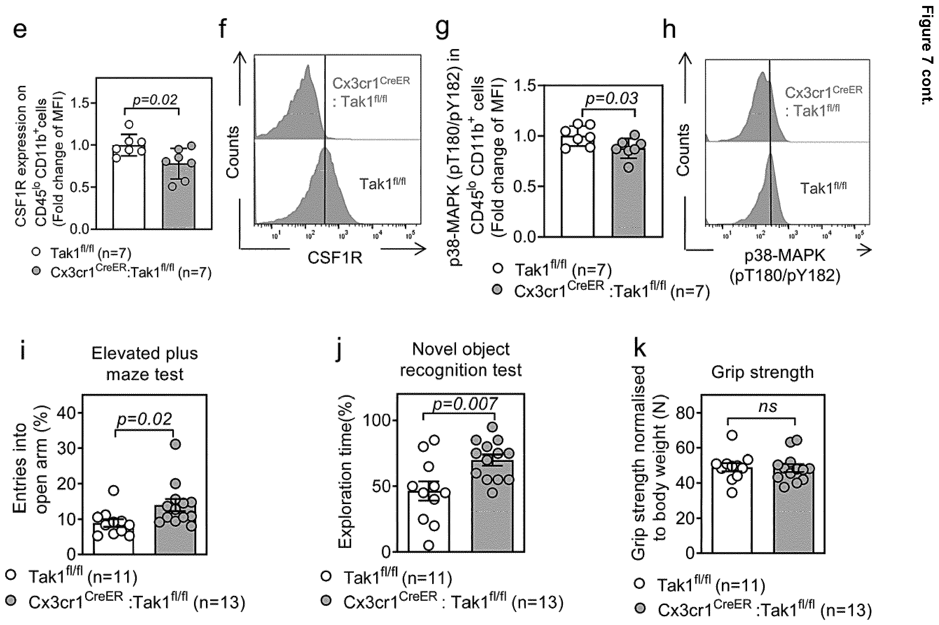

**Figure 7****: Genetic TAK1 deletion reduces microglia activation and improves cognitive function.**
   **(a, b)** The scatter plot **(a)** shows the quantification (fold change of MFI) of CD80 expression in microglia (CD45^{lo} CD11b⁺) from the CNS of B-ALL (E2A-PBX1) bearing Tak1^{fl/fl} (n=7) or Cx3cr1^{CreER}:Tak1^{fl/fl} (n=7) mice treated with CD19 CAR T cells. Experiments were performed two times and the results (mean ± s.e.m.) were pooled. The P-values were calculated using an unpaired t-test. **(b)** Representative flow cytometry plot for CD80 expression on microglia of the indicated groups. **(c, d)** The scatter plot **(c)** shows the quantification (fold change of MFI) of TREM2 expression on microglia (CD45^{lo} CD11b⁺) from the CNS of B-ALL (E2A-PBX1) bearing Tak1^{fl/fl}(n=7) or Cx3cr1^{CreER}:Tak1^{fl/fl} (n=7) mice treated with CD19 CAR T cells. Experiments were performed two times and the results (mean ± s.e.m.) were pooled. The *P*-values were calculated using an unpaired t-test. **(d)** Representative flow cytometry plot for TREM2 expression on microglia of the indicated groups. **(e, f)** The scatter plot **(e)** shows the quantification (fold change of MFI) of CSF1R expression in microglia (CD45^{lo} CD11b⁺) from the CNS of B-ALL (E2A-PBX1) bearing Tak1^{fl/fl}(n=7) or Cx3cr1^{CreER}:Tak1^{fl/fl} (n=7) mice treated with CD19 CAR T cells. Experiments were performed two times and the results (mean ± s.e.m.) were pooled. The *P*-values were calculated using an unpaired t-test. **(f)** Representative flow cytometry plot for CSF1R expression in microglia of the indicated groups. **(g, h)** The scatter plot **(g)** shows the quantification (fold change of MFI) of p38-MAPK (phospho) expression in microglia (CD45^{lo} CD11b⁺) from the CNS of B-ALL (E2A-PBX1) bearing Tak1^{fl/fl} (n=7) or Cx3cr1^{CreER}:Tak1^{fl/fl} (n=7) mice treated with CD19 CAR T cells. Experiments were performed two times and the results (mean ± s.e.m.) were pooled. *P*-values were calculated using an unpaired t-test. **(h)** Representative flow cytometry plot for p38-MAPK expression in microglia of the indicated groups. **(i)** The scatter plot shows the percentage of open arm entries made by Tak1^{fl/f} (n=11) or Cx3cr1^{CreER}:Tak1^{fl/f} (n=11) B-ALL bearing mice treated with CD19 CAR T cells in an Elevated plus Maze Test. **(j)** The scatter plot shows the percentage of time spent by Tak1^{fl/f}(n=11) or Cx3cr1^{CreER}: Tak1^{fl/fl} (n=11) B-ALL bearing mice treated with CD19 CAR T cell in exploring a novel object in a novel object recognition test. **(k)** The scatter plot shows the grip strength normalized to body weight (N) of Tak1^{fl/f}(n=11) or Cx3cr1^{CreER}: Tak1^{fl/fl}(n=11) B-ALL bearing mice treated with CD19 CAR T cell in a grip strength test. All experiments (**i-k**) were performed twice and the results (mean ± s.e.m.) were pooled. The P-values were calculated using one-way ANNOVA.

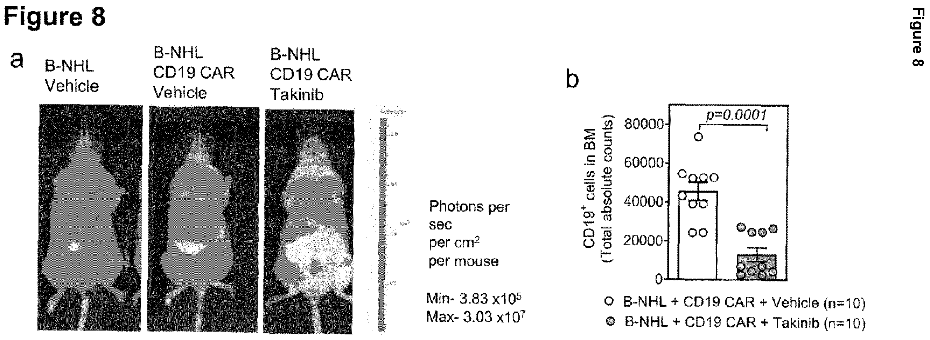

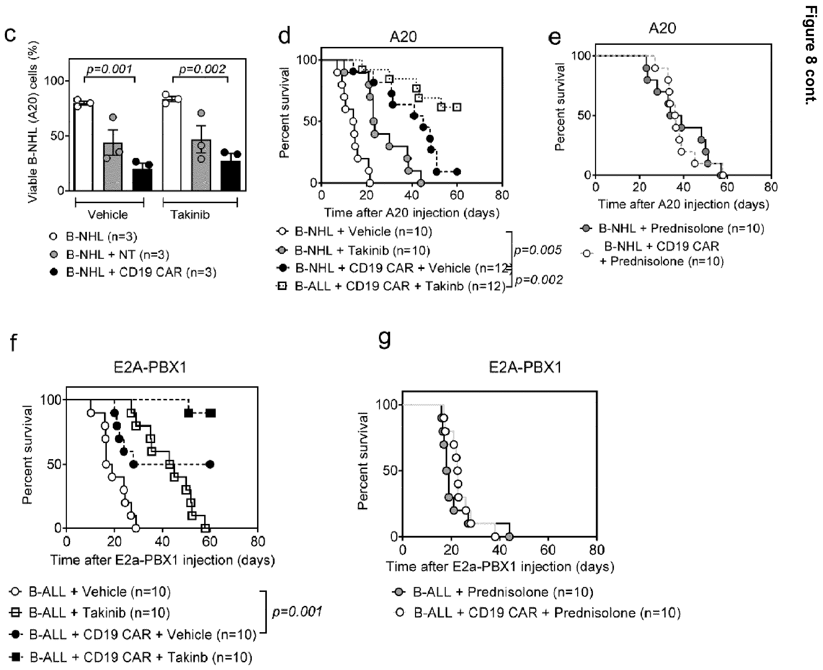

**Figure 8****: Takinib does not interfere with cytotoxicity of CAR19 T cells in vitro and in vivo**
   **(a)** Representative images showing the bioluminescence signal (dark grey clouds on top of mouse image) derived from the A20 **B-NHL** cells in mice that received **B-NHL** and vehicle, B-NHL, CD19 CAR T cells and vehicle, or B-NHL, CD19 CAR T cells and takinib at 48h post CD19 CAR T cell infusion. **(b)** The scatter plot shows the total absolute cell counts of CD19⁺ cells isolated from the bone marrow of CD19⁺ B-NHL (A20) bearing mice transplanted with CD19 CAR T cells and treated with either vehicle (n=10) or takinib (n=10). Experiments were performed twice and the results (mean ± s.e.m.) were pooled. **(c)** Scatter plot depicting percentage cell viability of B-NHL (A20) cells upon co-culture with NT cells and CD19 CAR T cells at a Effector-CD19 CAR/NT T cells and Target-A20 (E:T) ratio of 5:1 respectively upon treatment with 10µM takinib or vehicle. **(d)** Survival rates of B-NHL bearing (A20) mice that received CD19 CAR T cells and were treated with either vehicle or takinib day 3 to day 9 post B-NHL transfer as indicated. The experiments were performed three times and results were pooled. *P*-values were calculated using a two-sided Mantel-Cox test. **(e)** Survival rates of B-NHL bearing (A20) mice that received PBS (no T cells) or CD19 CAR T cells and were treated with prednisolone from day3 to day 9 post B-NHL transfer as indicated. The experiments were performed two times. *P*-values were calculated using a two-sided Mantel-Cox test. **(f)** Survival rates of B-ALL bearing (E2A-PBX1) mice that received CD19 CAR T cells and were treated with either vehicle or takinib from day 3 to day 9 post B-ALL transfer as indicated. Experiments were performed two times and the results were pooled. *P*-values were calculated using a two-sided Mantel-Cox test. **(h)** Survival rates of B-ALL bearing (E2A-PBX1) mice that received PBS (no T cells) or CD19 CAR T cells and were treated with prednisolone from day3 to day 9 post B-NHL transfer as indicated. The experiments were performed two times. *P*-values were calculated using a two-sided Mantel-Cox test.

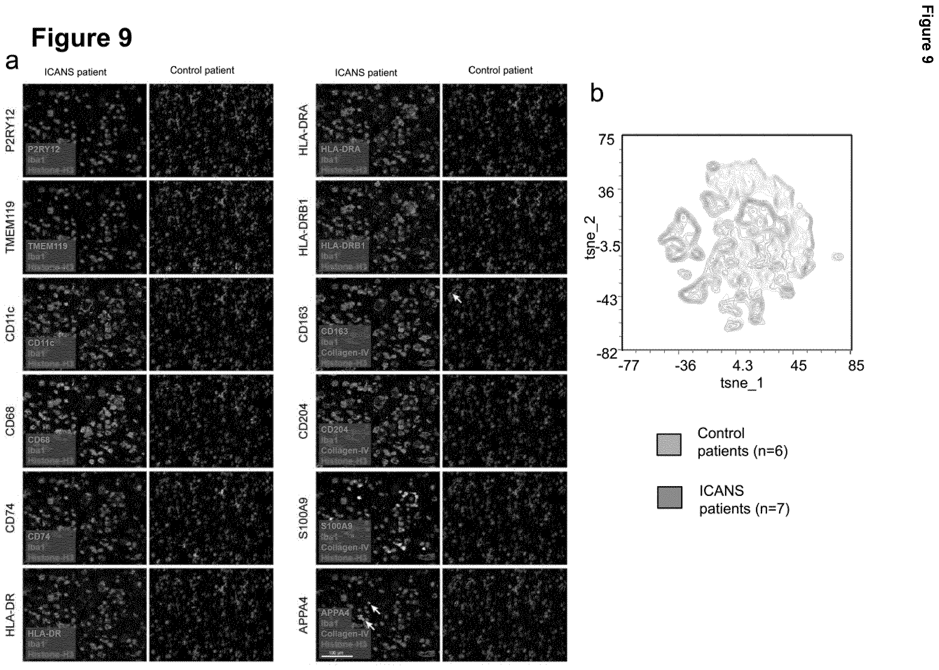

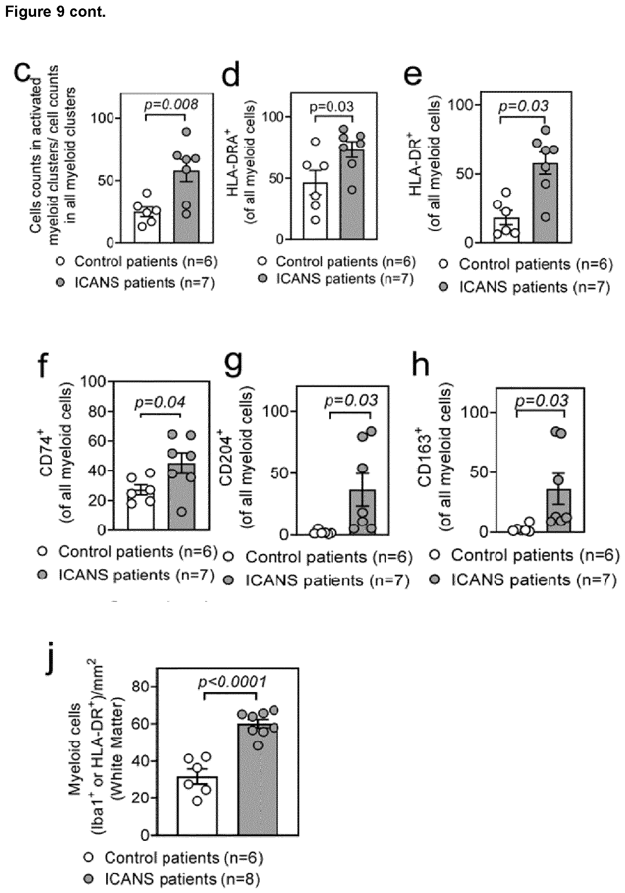

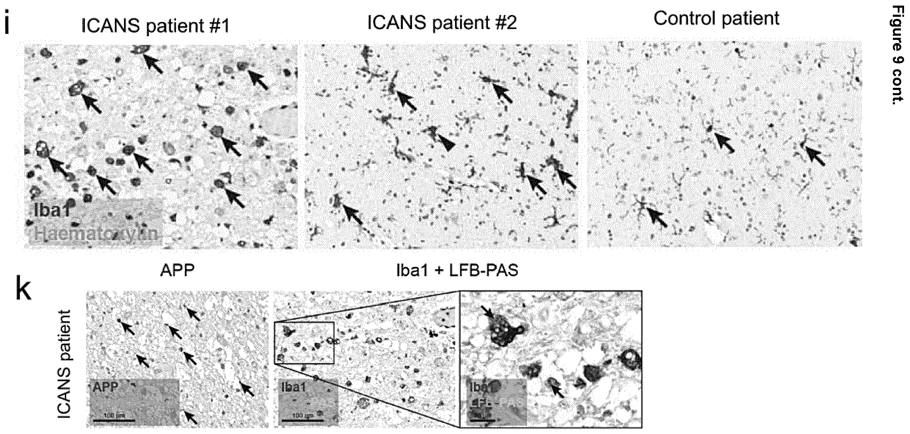

**Figure 9****: Microglia are activated upon transfer of CD19 CART cells into patients with B-NHL**
   **(a)** Representative images depicting the Imaging Mass Cytometry visualization of the white matter of the occipital lobe of CAR T cell patients and controls. Channels and colors are indicated. The scale bar depicts 100 µm. A strong microglial activation can be observed (downregulation of P2RY12, TMEM119, upregulation of CD11c, CD204, CD68 (marker for lysosomal activity)). S100A9 (MRP14) is typically expressed on round-like monocytes that are invading the CNS from the bloodstream and gradually upregulate microglial markers / complement the microglia pool / become microglia. **(b)** The tSNE plots represent the visualization of single cell myeloid marker expression after machine learning supervised segmentation of Imaging Mass Cytometry data from post-mortem FFPE CNS samples of ICANS patients with B-NHL treated with CD19 CAR T cells (red and orange color code) and aged-matched control patients without CAR T cell treatment (all other colors). **(c)** The scatter plot depicts depicts IMC based quantification of activated myeloid/microglia clusters of all clusters in post-mortem FFPE samples of occipital white matter of patients treated with CAR T cells (n=2) or age-matched controls (n=4). **(d)** The scatter plot depicts IMC based quantification of activated microglia (P2RY12^{lo} HLA-DR⁺ cells in all lba1⁺ cells) in post-mortem FFPE samples of occipital white matter of patients treated with CAR T cells (n=2) or age-matched controls (n=4). The experiment was performed once. **(e)** Histology of ICANS patients and age matched control brain samples immunostained for lba-1⁺ cells from the white matter of occipital lobe. Iba1 (upper panel, brown, exemplarily marked by arrows) and CD3 (lower panel, brown) in the white matter of the occipital lobe are depicted. Intracellular vesicles within roundish Iba1-positive cells are visible in patient #1. lba1-positive cells in patient #2 show an activated phenotype with a spiky appearance. An Iba1-positive cell with two somata (arrowhead), most likely in the process of cell division, can be identified. Counterstaining with haematoxylin. The scale bar represents 100 µm. **(f)** Scatter plot depicts the quantification of myeloid cells (either lba-1⁺ or HLA-DR⁺ cells) in white matter of post-mortem FFPE or fresh-frozen ICANS patients treated with CAR T cells (n=8) or age-matched controls (n=6). The experiment was performed once the *P*-values were calculated using the unpaired *t*-test. **(g)** The immunohistochemistry depicts characteristics of a Wallerian degeneration in post-mortem FFPE ICANS patient sample. APP deposits point towards an axonal damage. This axonal damage is followed by myelin loss and uptake of myelin by macrophages, which is accompanied by macrophage activation.

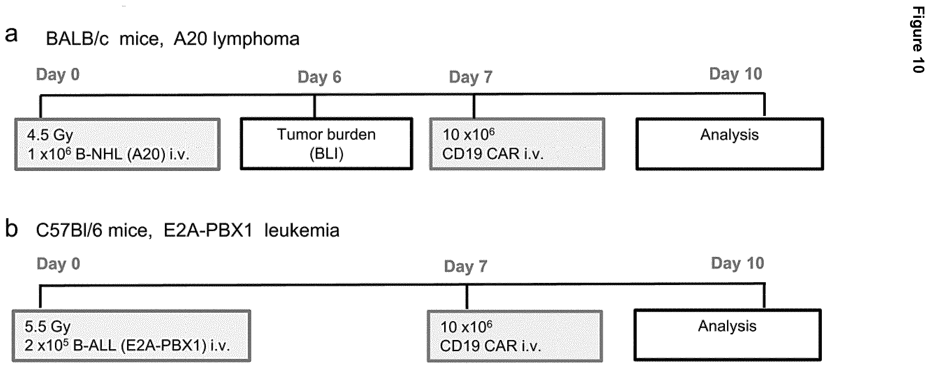

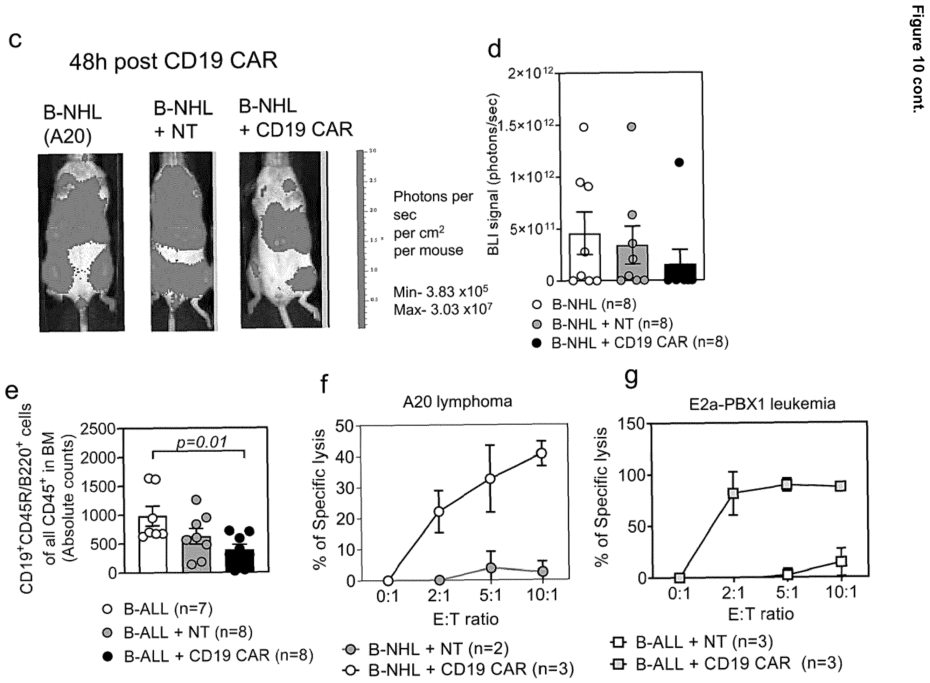

**Figure 10****:Treatment sketch, B-NHL engraftment and *in vitro* CD19 CAR T cell efficacy**
   **(a)** A schematic representation of the ICANS mouse model using A20 B cell lymphoma cells. BALB/c mice were sublethally irradiated with 4.5Gy and transplanted with 1 × 10⁶ A20 Luc⁺ GFP⁺ cells. On day 6, engraftment was quantified with bioluminescence imaging (BLI). On Day 7, 10 × 10⁶ CD19 CAR T cells were injected intravenously. Mice were sacrificed for analysis on day 10. **(b)** A schematic representation of the ICANS mouse model using B-ALL cells. C57BL/6 mice were sub-lethally irradiated with 5.5 Gy and transplanted with 2 × 10⁵ E2A-PBX1 cells. On day 7, 10 × 10⁶ CD19 CAR T cells were injected intravenously. Mice were sacrificed for analysis on day 10. **(c)** Representative images showing the lymphoma-derived bioluminescence signal of mice receiving B-NHL, B-NHL + NT T cells, or B-NHL + CD19 CAR T cells, respectively. NT T cells or CAR T cells were injected 7 days post-transplant. **(d)** Scatter plot representing the quantification of tumor burden via BLI in B-NHL, B-NHL + NT/CD19 CAR T cells treated, respectively, at 10 days post-transplant. **(e)** The scatter plot shows the quantification (absolute counts) of CD19⁺CD45R/B220⁺ cells within the CD45⁺ cell population in the bone marrow. The experiment was performed two times and the results (mean ± s.e.m) were pooled. The P-values were calculated using one-way ANOVA. **(f)** The plot represents the percentage of specific lysis of B-NHL cells upon co-culture with NT or CD19 CAR T cells at 0:1, 2:1, 5:1 and 10:1 at effector to target (E:T) ratios for 16h. **(g)** The plot represents the percentage of specific lysis of B-ALL cells upon co culture with NT or CD19 CAR T cells at 0:1, 2:1, 5:1 and 10:1 at effector to target (E:T) ratios for 16h.

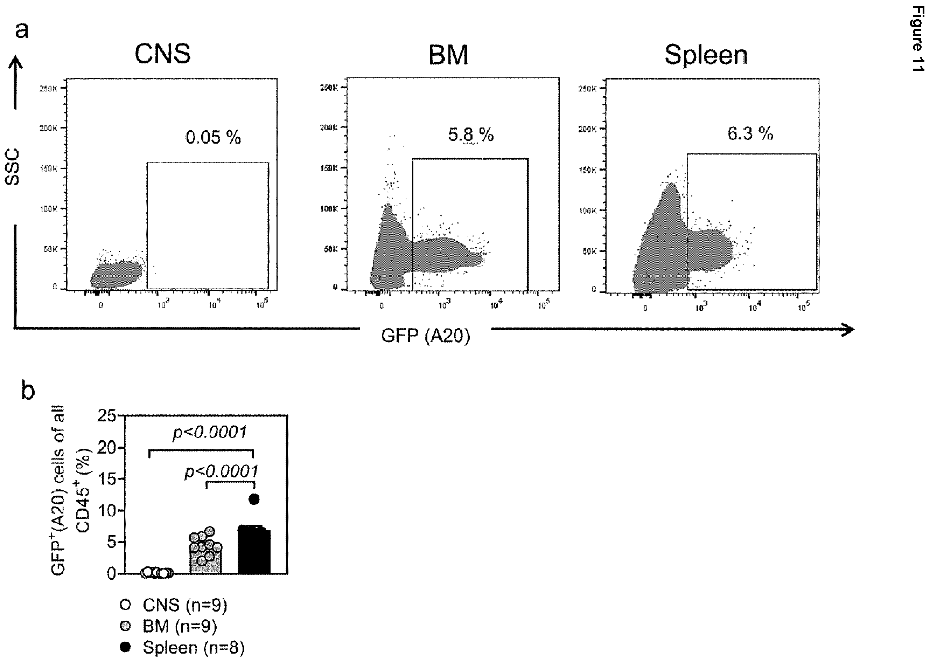

**Figure 11****: Quantification of peripheral and CNS B-NHL loads**
   **(a)** Representative flow cytometry plots indicating the proportion of GFP⁺ A20 B cell lymphoma cells in the CNS, BM, and spleen. **(b)** The scatter plot shows the quantification (percentage) of GFP⁺ A20 lymphoma cells in the total CD45⁺ population in the CNS, BM, and spleen. The experiment was performed three times and the results (mean ± s.e.m) were pooled. The *P*-values were calculated using one-way ANOVA.

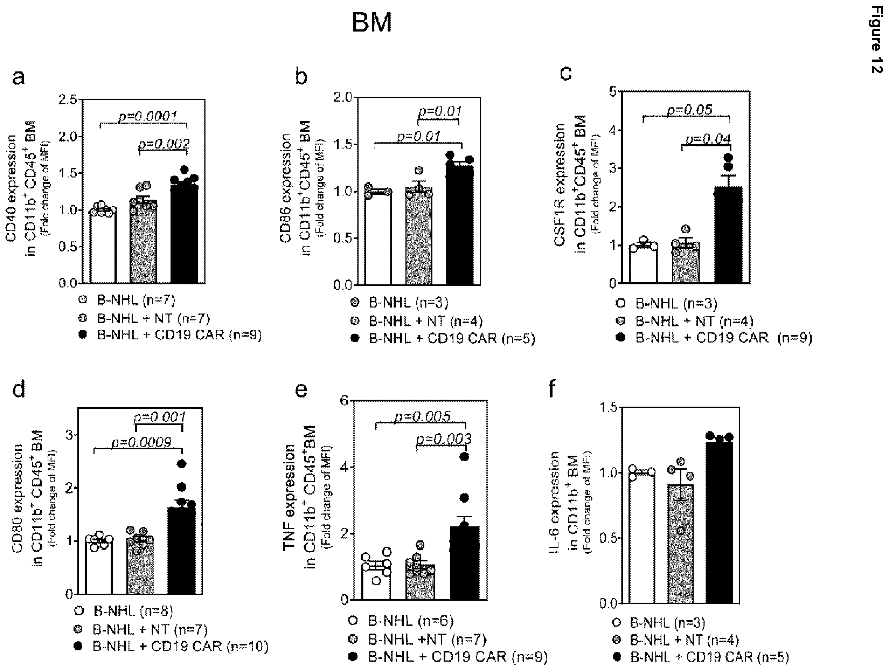

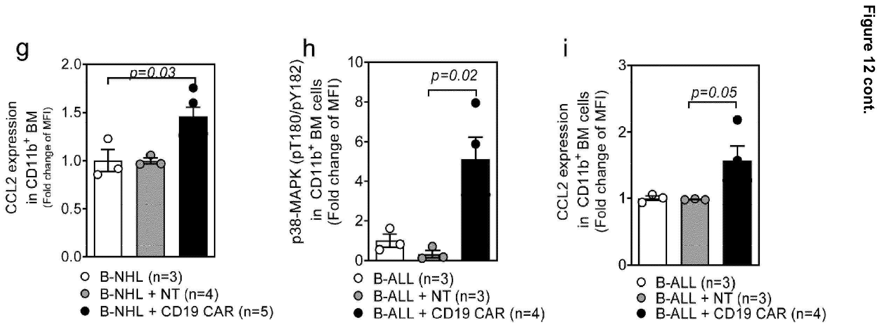

**Figure 12****: CD19 CAR T cell transfer induces myeloid cell activation in BM of B-NHL and B-ALL-bearing mice**
   **(a-c)** The scatter plow shows the quantification (fold change of MFI) of CD40 **(a),** CD86 **(b),** and CSF1R **(c)** expression in CD11b⁺ cells isolated from the BM of B-NHL (A20)-bearing mice treated with PBS, NT T cells, or CD19 CAR T cells. The experiment was performed once. *P*-values were calculated using one-way ANOVA **(b)** or Kruskal-Wallis test **(a-c). (d-e)** The scatter plot shows the quantification (fold change of MFI) of CD80 **(d)** and TNF **(e)** in CD11b⁺ cells isolated from the BM of B-NHL (A20)-bearing mice treated with PBS, NT T cells, or CD19 CAR T cells. The experiment was performed twice and the results (mean ± s.e.m) were pooled. *P*-values were calculated using one-way ANOVA **(d)** or Kruskal-Wallis test **(e). (f-g)** The scatter plot shows the quantification (fold change of MFI) of IL-6 **(f)** and CCL2 **(g)** expression in CD11b⁺ cells isolated from the BM of B-NHL (A20)-bearing mice treated with PBS, NT T cells, or CD19 CAR T cells. The experiment was performed once. *P*-values were calculated using Kuskal-Wallis test. **(h-i)** The scatter plot shows the quantification (fold change of MFI) of p38 MAPK (phospho) **(h)** and CCL2 **(i)** expression in CD11b⁺ cells isolated from the BM of B-ALL (E2a-PBX)-bearing mice treated with either PBS, NT T cells, or CD19 CAR T cells. The experiment was performed two times. *P*-values were calculated using Kuskal-Wallis test.

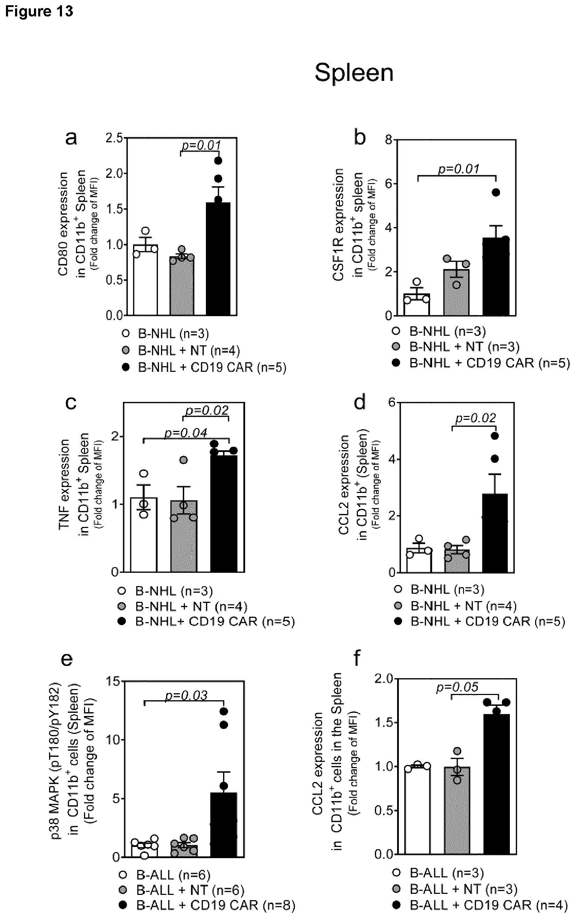

**Figure 13****: CD19 CAR T cell transfer induces myeloid cell activation in the spleen of** B-NHL **and B-ALL-bearing mice**
   **(a-b)** The scatter plot shows the quantification (fold change of MFI) of CD80 **(a)** and CSF1R **(b)** expression in CD11b⁺ cells isolated from the spleen of B-NHL (A20)-bearing mice treated with PBS, NT T cells, or CD19 CAR T cells. The experiment was performed once. *P*-values were calculated using Kuskal-Wallis test. **(c-d)** The scatter plot shows the quantification (fold change of MFI) of TNF **(c)** and CCL2 **(d)** expression in CD11b⁺ cells isolated from the spleen of B-NHL (A20)-bearing mice treated with PBS, NT T cells, or CD19 CAR T cells. The experiment was performed once. P-values were calculated using Kuskal-Wallis test. **(e-f)** The scatter plot shows the quantification (fold change of MFI) of p38 MAPK **(e)** and CCL2 **(f)** expression in CD11b⁺ cells isolated from the BM of B-ALL (E2a-PBX)-bearing mice treated with either PBS, NT T cells, or CD19 CAR T cells. The experiment was performed two times. *P*-values were calculated using Kuskal-Wallis test.

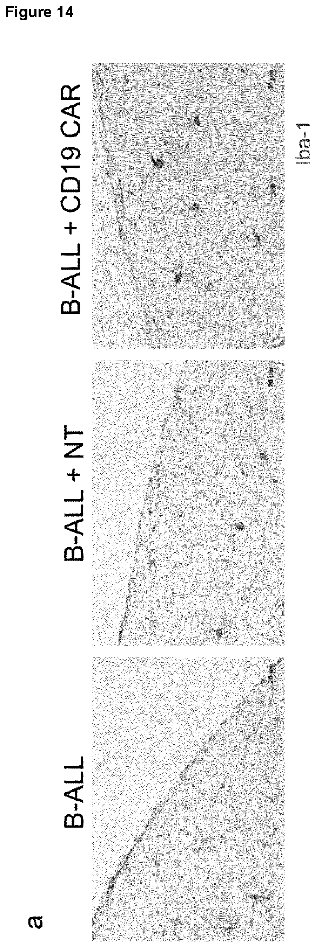

**Figure 14****: lba1⁺ cells increase in the cortex upon CAR T cell transfer**

   **(a)** Representative images of lba-1⁺ cells in the cortex of B-ALL mice treated with PBS, NT T cells, or CD19 CAR T cells. Original magnification 40x, scale bar 20µm. The experiment was performed once.
**Figure 15****: CNS immune cell infiltration upon NTICAR T cell transfer**
   **(a-c)** The scatter plots show the quantification (absolute counts) of CD11c⁺ **(a),** Ly6C⁺ **(b),** and Ly6G⁺ **(c)** cells from CD45^{hi}CD11b⁺ cells isolated from the CNS of B-NHL (A20)-bearing mice treated with PBS, NT T cells, or CD19 CAR T cells. The experiment was repeated twice and the results (mean ± s.e.m.) were pooled. *P*-values were calculated one-way ANOVA. **(d)** The scatter plot shows the quantification (absolute counts) of F4/80⁺ cells from all CD11b⁺ cells isolated from the CNS of B-NHL (A20)-bearing mice treated with PBS, NT T cells, or CD19 CAR T cells. The experiment was repeated twice and the results (mean ± s.e.m.) were pooled. *P*-values were calculated one-way ANOVA. **(e)** The scatter plot shows the quantification (absolute counts) of CD3⁺ cells from all CD45⁺ cells isolated from the CNS of B-NHL (A20)-bearing mice treated with PBS, NT T cells, or CD19 CAR T cells. The experiment was repeated twice and the results (mean ± s.e.m.) were pooled. *P*-values were calculated using one-way ANOVA. **(f)** The scatter plot shows the quantification (absolute counts) of CD3⁺ cells from all CD45⁺ cells isolated from the CNS of B-ALL (E2a-PBX1)-bearing mice treated with PBS, NT T cells, or CD19 CAR T cells. The experiment was performed twice and the results (mean ± s.e.m.) were pooled. *P*-values were calculated using one-way ANOVA.

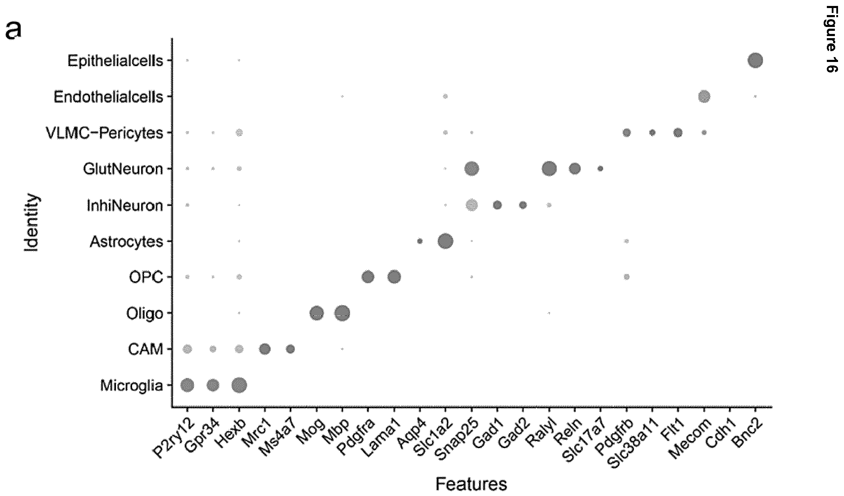

**Figure 16****: snRNA sequencing of CNS cells**

   **(a)** The plot shows gene expression of cells from the CNS of B-NHL (A20) bearing mice treated with either NT T cells compared to CD19 CAR T cells. The different cell types were determined by marker gene expression. The experiment was performed once.

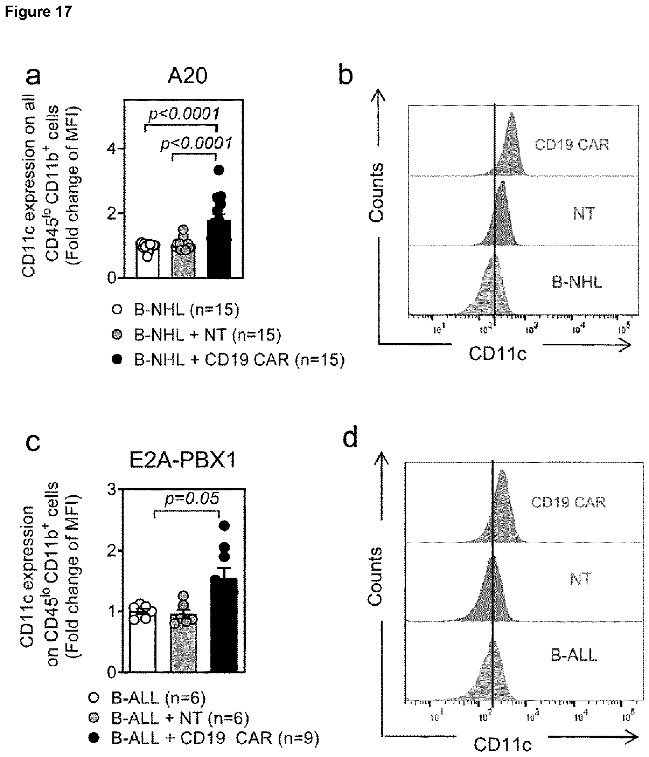

**Figure 17****: CD11c expression in microglia in mice after NTICAR T cell transfer**
   **(a, b)** The scatter plot **(a)** shows the quantification (fold change of MFI) of CD11c in microglia (CD45^{lo} CD11b⁺) from the CNS of B-NHL (A20) bearing mice, treated with either PBS (n=15), NT T cells (n=15) or CD19 CAR T cells (n=15). The experiment was repeated four times and results (mean ± s.e.m.) were pooled. P-values were calculated using one-way ANOVA. **(b)** Representative flow cytometry plots showing CD11c expression in microglia for the indicated groups. **(c, d)** The scatter plot **(c)** shows the quantification (fold change of MFI) of CD11c in microglia (CD45^{lo} CD11b⁺) from the CNS of B-ALL (E2A-PBX1) bearing mice treated with either PBS (n=6), NT T cells (n=6) or CD19 CAR T cells (n=9). The experiment was repeated three times and the results (mean ± s.e.m.) were pooled. *P*-values were calculated using one-way ANOVA. **(d)** Representative flow cytometry plots showing CD11c expression in microglia for the indicated groups.

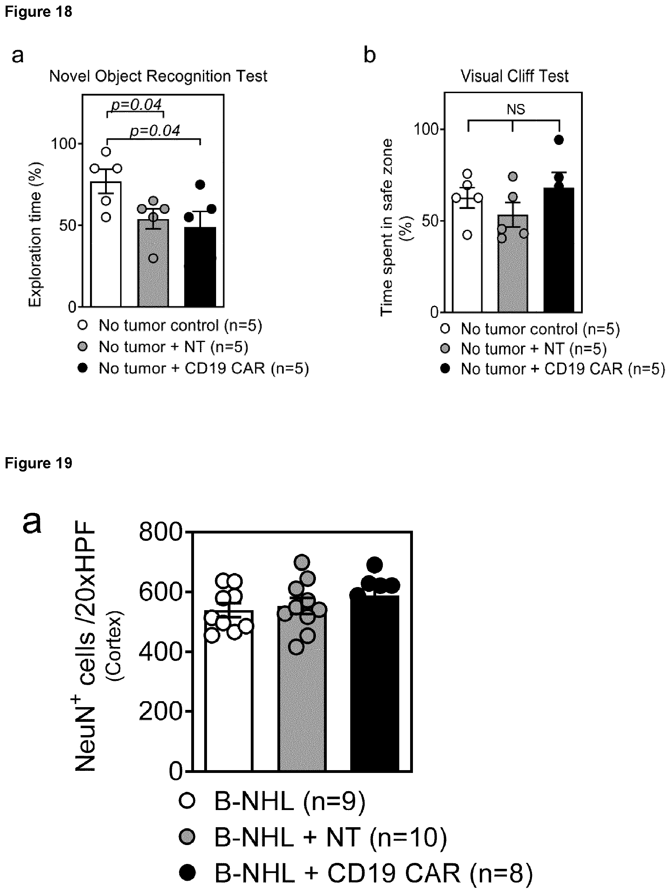

**Figure 18****: CD19 CAR T cell treatment impairs cognitive function in non-tumour-bearing mice**
   **(a)** The scatter plot shows the percentage of time spent by sub-lethally irradiated C57BI/6 (non-tumour-bearing) mice treated with PBS (n=5) , NT T cells (n=5), or CD19 CAR T cells (n=5) in exploring a novel object in the novel object recognition test. The experiment was performed once. P-values were calculated using one-way ANOVA. **(b)** The scatter plot shows the percentage of time spent by sub-lethally irradiated C57BI/6 (non-tumour-bearing) mice treated with PBS (n=5), NT T (n=5) cells, or CD19 CAR T cells (n=5) in the safe zone during the visual cliff test. The experiment was performed once. P-values were calculated using one-way ANOVA.
**Figure 19****: Neuron quantification in mice receiving NTICAR T cells**

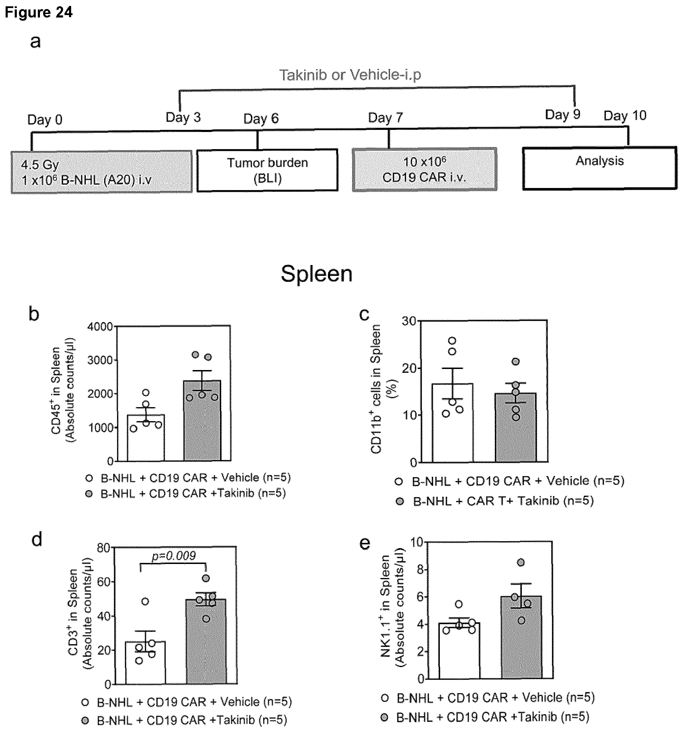

   **(a)** Quantification of immunofluorescence imaging representing the NeuN and/or DAPI positive cells in the CNS of B-NHL bearing mice treated with either NT T cells (n=5) or CD19 CAR T cells (n=5).

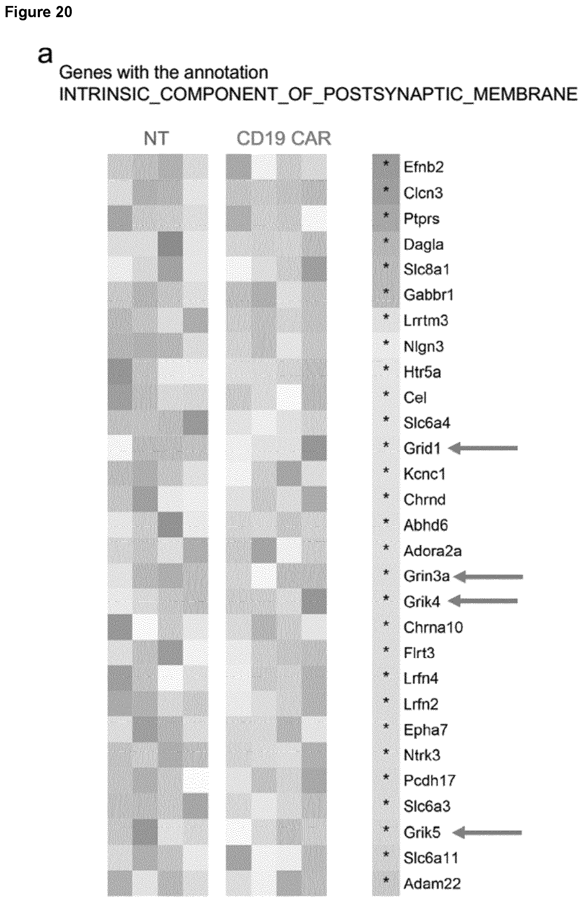

**Figure 20****: Multiple genes with the annotation *intrinsic postsynaptic membrane activity* are downregulated in B-NHL bearing mice treated with CAR T cells**
   **(a)** Heat map of microarray data showing significantly regulated genes with the annotation intrinsic post synaptic membrane activity. Cells were isolated from the CNS of B-NHL (A20) bearing mice treated with either NT T cells (n=4) or CD19 CAR T cells (n=4). Color code represents the Z-score log2 intensity. The red arrows point towards postsynaptic receptor subunits (Grin3A, Grik4, Grik5) and Grid1 encoding for glutamate D1 receptor (GluD1), which are selected because they promote the stability and activity of functional synapses.

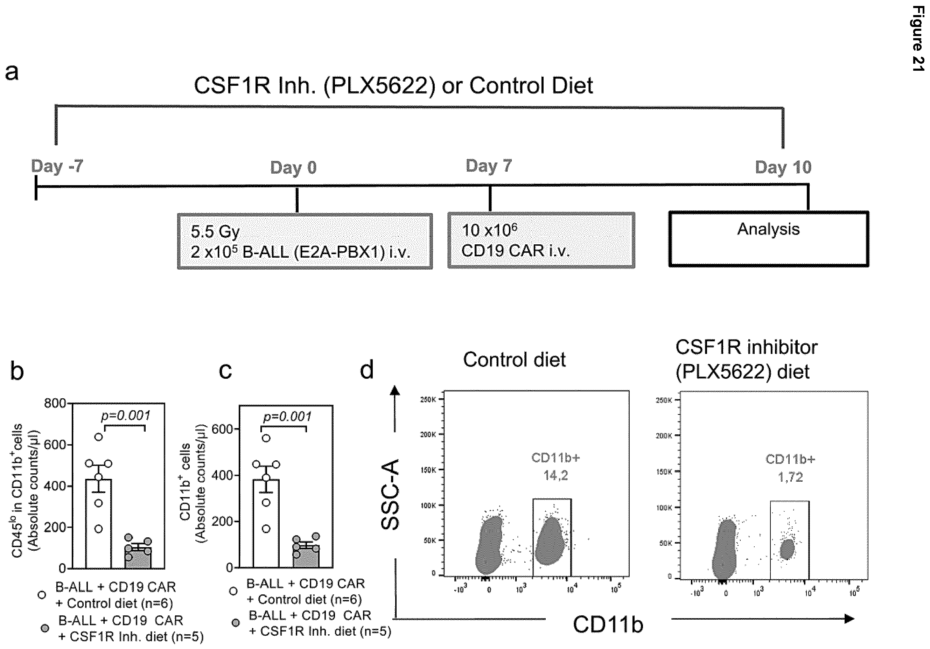

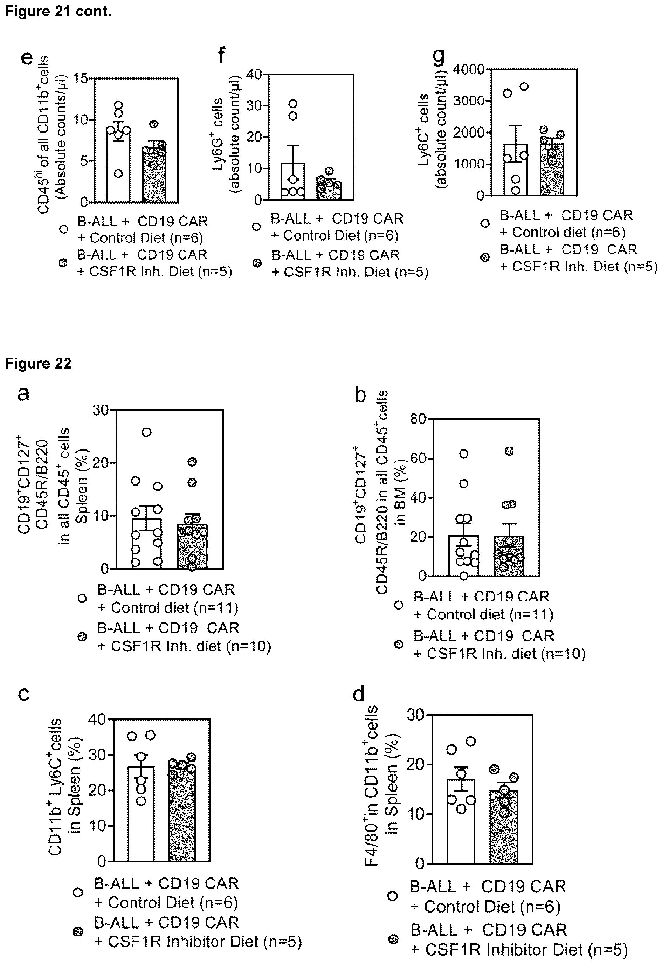

**Figure 21****: CSF1R inhibition reduces microglia cell numbers in the CNS of B-ALL bearing mice without affecting other CNS myeloid cells**
   **(a)** Schematic representation of CSF1R inhibitor treatment. Mice were maintained on CSF1R inhibitor (PLX5622) diet or control diet from day -7 until day 10. Behaviour studies were performed on day 10. **(b, c)** Flow cytometry of CD11b⁺ cells from B-ALL bearing mice maintained on either a control diet or CSF1R inhibitor (PLX5622) diet. **(b, c)** The scatter plot shows the quantification (absolute counts) of CD45^{lo} CD11b⁺ **(b)** and CD11b⁺ **(c)** cells from the CNS of B-ALL bearing mice maintained on either a control diet or CSF1R inhibitor (PLX5622) diet and transplanted with CD19 CAR T cells. The experiment was performed twice and results (mean ± s.e.m.) were pooled. *P-*values were calculated using the unpaired students t-test. **(d)** Representative flow cytometry plots showing CD11b-positive cells of the indicated groups. **(e)** The scatter plot shows the quantification (absolute count) of CD45^{hi} cells in the CD11b⁺ population isolated from the CNS of B-ALL bearing mice maintained on a control diet or CSF1R inhibitor (PLX5622) diet and transplanted with CD19 CAR T cells. The experiment was performed once. **(f, g)** The scatter plots show the quantification (absolute counts) of Ly6G⁺ **(f)** and Ly6C⁺ **(g)** cells of all CD45^{hi}CD11b⁺ cells from the CNS of B-ALL bearing mice maintained on a control diet or CSF1R inhibitor (PLX5622) diet and transplanted with CD19 CAR T cells. The experiment was performed once. *P*-values were calculated using the Mann-Whitney **(e, f)** or unpaired student's t-test **(g).**
**Figure 22****: CSF1R inhibition does not decrease B NHL/B-ALL cell counts or deplete peripheral monocytes/macrophages**
   **(a,b)** The scatter plot shows the quantification (percentage) of CD45R/B220⁺, CD19⁺ CD127⁺ cells from the spleen **(a)** and BM **(b)** of B-ALL bearing mice maintained on Control diet or CSF1R inhibitor (PLX5622) diet and transplanted with CD19 CAR T cells. The experiment was performed twice, the results (mean ± s.e.m.) were pooled. The *P*-values were calculated using the unpaired students t-test. **(c, d)** The scatter plot shows the quantification (percentage) of Ly6C⁺ **(c)** and F4/80⁺ **(d)** cells from all CD11b⁺ cells in the spleen of B-ALL-bearing mice maintained on Control diet or CSF1R inhibitor (PLX5622) diet and transplanted with CD19 CAR T cells. The experiment was performed once and the results (mean ± s.e.m.) were pooled. The *P*-values were calculated using the unpaired students t-test.

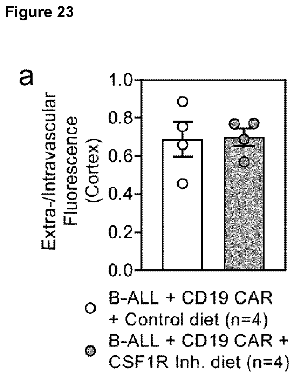

**Figure 23****: FITC-dextran as a surrogate parameter for BBB permeability in CAR T cell control/CSF1R inhibitor-treated mice**
   **(a)** Quantification of immunofluorescence imaging representing the BBB leakage via extravasation FITC-Dextran from the CNS of B-ALL bearing mice treated with CD19 CAR T cells and maintained on a control diet (n=4) or CSF1R inhibitor (PLX5622) diet (n=4
**Figure 24****: Impact of Takinib treatment on immune cells in the spleen**
   **(a)** Treatment sketch for takinib treatments. Mice were treated intra-peritoneally with takinib from day 3 until day 9. **(b-e)** The scatter plot shows the quantification (absolute counts) of NK1.1⁺ , CD45⁺, CD11b⁺ and CD3⁺ cells from the spleen of B-NHL (A20) bearing mice that received CD19 CAR T cells and treated with vehicle or Takinib The experiment was performed once, the results (mean ± s.e.m.) were pooled. The *P*-values were calculated using the unpaired students *t*-test.

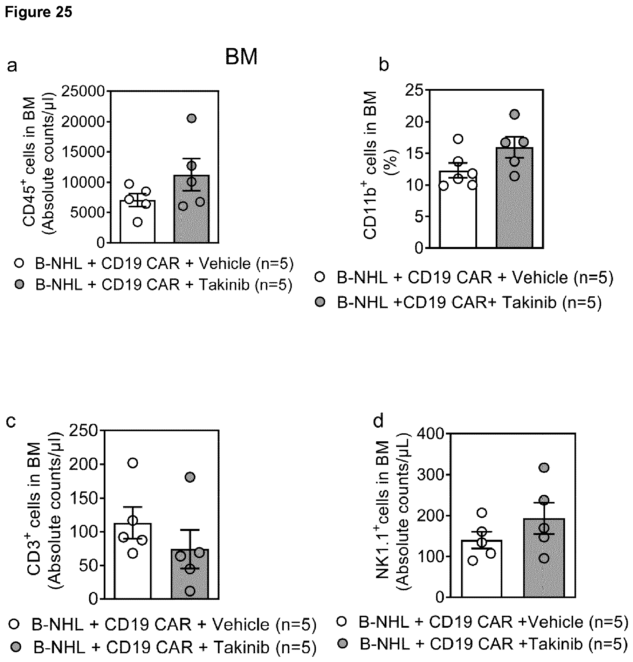

**Figure 25****: Impact of takinib treatment on immune cells in the BM**
   **(a-d)** The scatter plot shows the quantification (absolute counts) of NK1.1⁺ , CD45⁺, CD11b⁺ and CD3⁺ cells from the BM of B-NHL (A20) bearing mice that received CD19 CAR T cells and treated with vehicle or takinib The experiment was performed once, the results (mean ± s.e.m.) were pooled. The *P*-values were calculated using the unpaired students t-test.

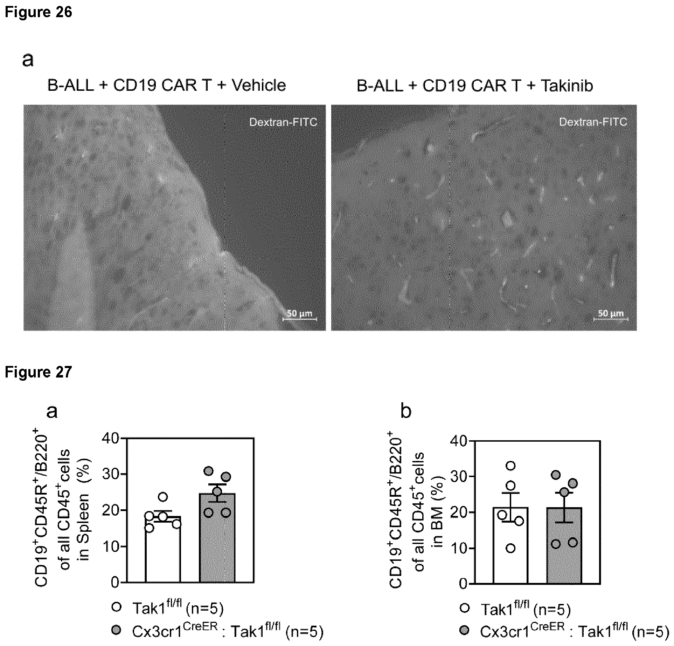

**Figure 26****: FITC-dextran as a surrogate parameter for BBB permeability in CAR T cell vehicle/takinib-treated mice**
   **(a)** Representative images representing the BBB leakage via extravasation FITC-Dextran from the CNS of B-ALL bearing mice treated with CD19 CAR T cells and vehicle or takinib as indicated.
**Figure 27****: Tumor burden in Cx3cr1Cre:ER Tak1fl/fl and Tak1fl/fl mice**
   **(a)** The scatter plot shows the quantification (percentage) of CD19⁺ CD45R/B220⁺ cells of all CD45 cells from the spleen of Tak1^{fl/fl} orCx3cr1^{CreER}: Tak1^{fl/fl} B-ALL (E2A-PBX1) bearing mice treated with CD19 CAR T cells The experiment was performed once, the results (mean ± s.e.m.) were pooled. The P-values were calculated using the unpaired students t-test. **(b)** The scatter plot shows the quantification (percentage) of CD19⁺ CD45R/B220⁺ cells of all CD45 cells from the BM of Tak1^{fl/fl} or Cx3cr1^{CreER}: Tak1^{fl/fl} B-ALL (E2A-PBX1) bearing mice treated with CD19 CAR T cells .The experiment was performed once and results (mean ± s.e.m.) were pooled. P-values were calculated using the unpaired students t-test.

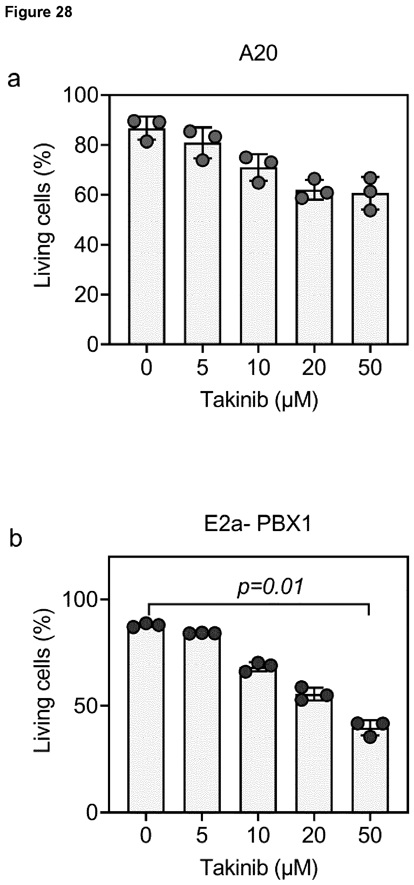

**Figure 28****: Impact of takinib on the viability of B-NHL and B-ALL cells**
   **(a)** Bar diagram representing the viability of **B-NHL** (A20) cells upon treatment with the indicated concentrations of takinib for a period of 24h. **(b)** Bar diagram representing the viability of B-ALL (E2A-PBX1) cells upon treatment with the indicated concentrations of takinib for a period of 24h.

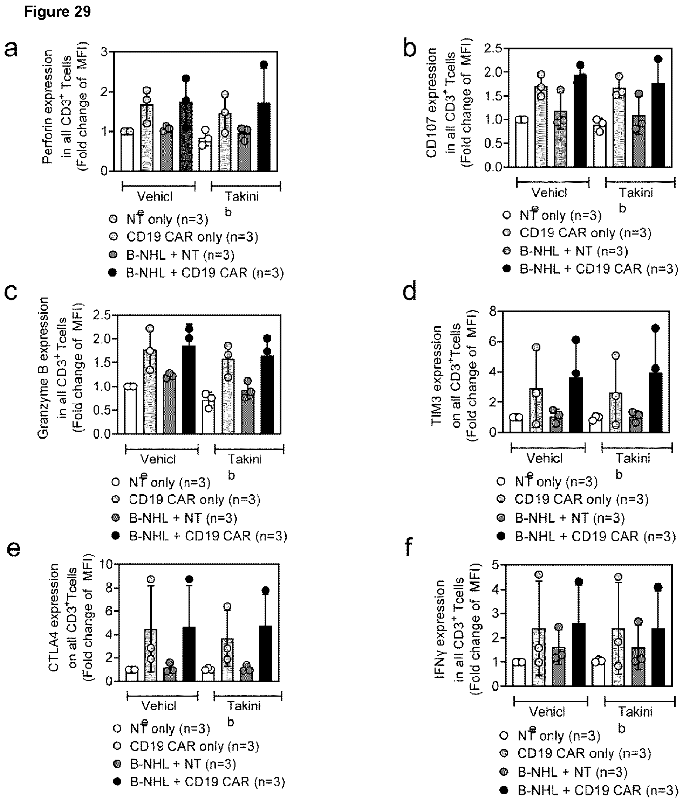

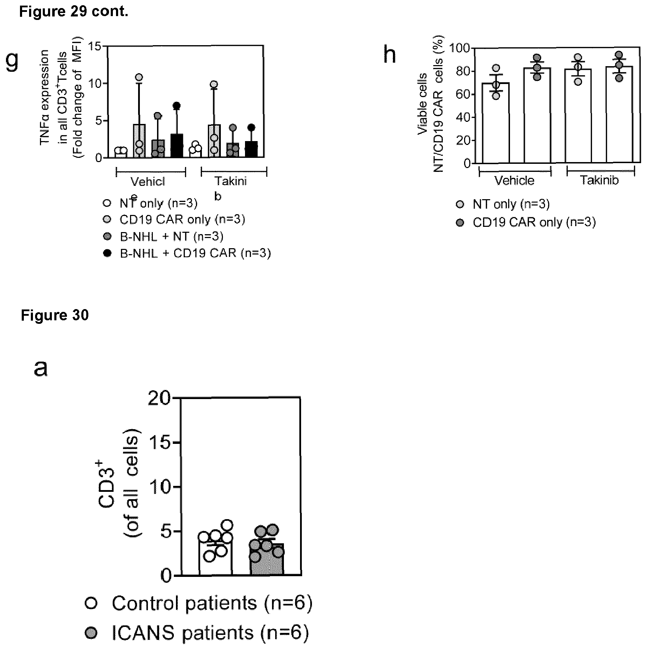

**Figure 29****: Takinib does not impair the CD19 CAR T cell viability and activation marker expression**
   **(a-c)** The scatter plots show the quantification (fold change of MFI) of **(a)** Perforin, **(b)** CD107; **(c)** Granzyme B expression in CD3⁺ T cells derived from a B-NHL and NT/CD19 CAR T cell co-culture experiment. B-NHL and NT/CD19 CAR T cells were co-cultured at a ratio of 1:5 and treated with 10µm of vehicle or takinib. The experiment was performed three times and the results (mean ± s.e.m.) were pooled. Each point represents one independent experiment. The *P*-values were calculated using one-way ANNOVA. **(d, e)** The scatter plot shows the quantification (fold change of MFI) showing **(d)** TIM-3 and **(e)** CTLA-4 expression in CD3⁺ T cells from a B-NHL and NT/CD19 CART cell co-culture experiment. B-NHL and CD19 CAR/ NTT cells were co-cultured at a ratio of 1:5 and treated with 10µM of vehicle or takinib. The experiment was repeated three times and results (mean ± s.e.m.) were pooled. Each point represents one independent experiment. The *P-*values were calculated using one-way ANNOVA. **(f, g)** The scatter plot shows the quantification (fold change of MFI) for IFN-γ **(f)** or TNF **(g)** expression in CD3⁺ T cells from a B-NHL and NT/ CD19 CAR T cell co culture experiment. B-NHL and NT/CD19 CAR T cells were co-cultured at a ratio of 1:5 and treated with either 10µm of vehicle or takinib. The experiment was performed three times and the results (mean ± s.e.m.) were pooled. Each point represents one independent experiment. The *P*-values were calculated using one-way ANNOVA. **(h)** The scatter plot shows the percent viability of NT or CD19 CAR T cells in co-culture with B-NHL (A20) cells. The cultures contained 10µM vehicle or takinib.
**Figure 30****: Imaging Mass Cytometry to detect microglia activation after CAR T cell transfer**
   **(a, b)** The histogram **(a)** and scatter plot **(b)** depict the quantification of P2RY12⁺ cells in lba1⁺ cells (absolute counts and percentage) based on imaging mass cytometry data from post-mortem FFPE samples of occipital white matter of patients treated with ICANS (n=2) or age-matched controls (n=4). The experiment was performed once (mean ± s.e.m.). **(c, d)** The histogram **(c)** and scatter plot **(d)** depict the quantification of CD11c⁺ cells in lba1⁺ cells (absolute counts and percentage) based on imaging mass cytometry data from post-mortem FFPE samples of occipital white matter of patients treated with ICANS (n=2) or age-matched controls (n=4). The experiment was performed once (mean ± s.e.m.). **(e)** The heatmap shows median marker intensity in different myeloid clusters obtained with unsupervised PhenoGraph clusterization of 23 myeloid marker in single-cell imaging mass cytometry data from post-mortem FFPE samples of occipital white matter of patients with ICANS (n=2) or age-matched controls (n=4). **f)** The heatmap shows myeloid cluster representation across samples (abundances) of post-mortem FFPE samples of occipital white matter of patients with ICANS (n=2) or age-matched controls (n=4).

### EXAMPLES

The invention is further described by the following examples. These are not intended to limit the scope of the invention but represent preferred embodiments of aspects of the invention provided for greater illustration of the invention described herein.

### Methods

### Patients

Characteristics of patients studied by IMC and IHC are shown in Suppl. Table S1 and S2. The study was approved by the local ethics committee (project-number: 20-1223). Written informed consent was obtained from all participants in accordance with the Declaration of Helsinki.

### Transduction of CD19 CART cells

The anti-CD19 CAR includes a CD28 costimulatory molecule. CD19.28Z CAR stable producer cell lines (12a) were provided by Dr. Federico Simonetta (Stanford, USA). The cell lines were generated as described earlier (12a). Briefly, the stably transduced cell lines were maintained in DMEM (Gibco) media with 10% FCS (Gibco, USA) and 1% Penicillin-Streptomycin (Gibco). T cells were isolated from the spleens of BALB/c or C57BI/6 mice using Pan T cell isolation kit as per the manufacturer's instructions (Miltenyi Biotech). Dyna beads (Gibco) were added at a concentration of 20µl per million T cells. The dyna beads were washed twice with PBS and once with the complete media. The beads were then suspended in complete media and added to the flask containing T cells. Murine T cells were supplemented with 30U/ml murine IL-2 (Peprotech) and 10ng/ml IL-7 (Peprotech). T cells were cultured in t75 flasks upright for at least 24h. Non-coated sterile 6 well plates (Co-star or Nunc) were coated with 15ug/ml of Retronectin (Takara) overnight. The plates were then blocked with 2% sterile BSA in PBS for 30min at RT. Viral supernatant was spun over retronection (15µg/ml) coated plates at 3800 rpm for 2h at 32 °C. Viral supernatant was removed and T cells supplemented with 30U/ml murine IL-2 and 10ng/ml IL-7 were added to the plates and cultured for an additional 48h. The T cells were expanded further for 48h and the efficiency of transduced T cells was checked using Protein L staining. The cells were blocked with 2% BSA for 15 min and washed. Biotin tagged Protein-L (Pierce) was added and incubated at 4° C for 30 min. The cells were washed with PBS and incubated with PE tagged Streptavidin (Biolegend) for 20 min. The beads were then removed and cells were transplanted intravenously.

### Treatment with Inhibitors

Mice were treated with either takinib (Selleckchem, Germany) or vehicle after B-NHL or B-ALL transplantation. For the treatment, stocks of 3 mg/ml and 10 mg/ml of takinib were prepared in DMSO. The stocks were diluted in 0.5% carboxymethylcellulose solution (vehicle) and administered by intraperitoneal injection at a dosage of 0.15 mg/kg (takinib-FACS) and 0.5 mg/kg (survival and behaviour studies) per mouse per day from day 3 to day 9 following either B-NHL or B-ALL transplant, as described previously (*13a*). An equal volume of corresponding vehicles was administrated to the control groups daily from day 3 until day 9. For survival expriments, 0.5 mg/kg takinib or vehicle per mouse per day was administered intraperitonially from day 3 to day 11 following either B-NHL or B -ALL transplant, as described previously.

### Mice

C57BL/6 (H-2Kb, Thy-1.2) and BALB/c (H-2Kd, Thy-1.2) mice were purchased either from Janvier Labs (France) or from the local stock of the animal facility at the University of Freiburg. Tak1fl/fl and Cx3cr1creER:Tak1fl/fl (47). Mice were used between 6 and 14 weeks of age and only female or male donor/recipient pairs were used. Animal protocols (Protocol numbers: G-17/093, G-20/075, G-17/063, G-22/052, X-20/07A, X-15/10A) were approved by the Regierungspräsidium Freiburg, (regional council), Germany (Federal Ministry for Nature, Environment and Consumers Protection).

### B-NHL mouse model

A20 Luc⁺ GFP⁺ immortalized murine lymphoma cell lines were used to induce B-NHL in mice. BALB/c recipients were sublethally irradiated with 4.5 Gy and transplanted with 1×10⁶ A20 Luc⁺ GFP⁺ intravenously. 7 days later 10×10⁶ syngeneic CD19 CAR T cells generated from BALB/c mice were administered. The mice were perfused with Ice cold PBS on day 10 and CNS was analysed. For survival experiments BALB/c recipients were sublethally irradiated with 4.5 Gy and transplanted intravenously with 1×10⁵ A20 Luc⁺ GFP⁺ cells. Seven days later 5×10⁶ syngeneic CD19 CAR T cells from BALB/c mice were administered. Tumor burden was checked by bioluminescence imaging.

### B-ALL mouse model

The E2A-PBX1 cell line (*15a*) was provided by Dr. Federico Simonetta (Stanford, USA). C57BI/6 recipients were sublethally irradiated with 5.5 Gy and transplanted with 2×10⁵ E2A-PBX1 cells intravenously. Seven days later 10×10⁶ syngeneic CD19 CAR T cells from C57BI/6 mice were administered. The mice were perfused with ice cold PBS on day 10 and the CNS was analysed. For survival experiments, C57BI/6 recipients were sub-lethally irradiated with 4.5 Gy and transplanted with 5×10³ E2A-PBX1 cells intravenously. Seven days later 5×10⁶ syngeneic CD19 CAR T cells from C57BI/6 mice were administered.

### Microglial depletion

Mice received PLX5622 (CSF1R inhibitor) chow or control chow (Research Diets) for 7 days prior to the experiment to achieve microglial depletion. C57BI/6 mice were irradiated with 5.5 Gy and transplanted with 2×10⁵ E2A-PBX1 cell lines. 10×10⁶ CD19 CAR T were injected on day 7 and behaviour studies were performed on day 10. Mice were maintained on PLX5622 chow or control chow throughout the duration of the experiment. The effectiveness of microglia depletion was determined by flow cytometry.

### T cell functional assays in-vitro

A20 Luc⁺ GFP⁺ cells were stained with cell trace violet as per the manufacturer's instructions (Life Technologies). A20 Luc⁺ GFP⁺ cells and CD19 CAR T cells were incubated at ratios 1:1, 2:1, 5:1, and 10:1 for 16h at 37° C. Following incubation, cells were harvested and stained with live/dead far red fixable viability stain (Invitrogen).

### Novel object recognition test

Novel object recognition test was performed as described previously with modifications (*17a*). During the habituation phase, each mouse was allowed to explore two similar objects within a total exploration time of 20s. We commenced the testing phase six hours after the habituation phase. During the testing session, each mouse was allowed to explore a familiar object and a novel object of different shape and texture. The position of the novel object and the familiar object was randomized between each mouse. The time spent by each mouse to explore the novel object and the familiar object was noted. The experiment was stopped when the total exploration time reached 20s. The amount of time spent by the mouse exploring the novel object to the total exploration time is calculated as an index of recognition memory.

### Grip strength test

The grip strength test was performed as described previously (*18a*) to assess the forelimb strength of the mice. Each mouse was allowed to grasp on to the metal grid of the apparatus and tail was pulled backwards until they leave the grid. A total of five trials were performed with each mouse and the average of grip strength (N) normalized to the body weight of mouse was estimated.

### Elevated plus maze test

We performed the elevated plus maze test to evaluate the anxiety behaviour in mice as described previously with minor modifications (*18a*). Each mouse was kept at the junction of closed arms and open arms of the maze. An open arm entry was considered when the mouse extended its head towards the open arm or when it entered the arm. We quantified the time they spent in the open arm in minutes. This time was then put into relation to the total time frame of six minutes. The resulting value was a percentage showing entry into open arms in relation to the total time.

### Visual cliff test

The visual cliff test was employed as described previously (*19a*) with modifications to evaluate the depth perception of the mice. The test was conducted for 10 minutes per mouse. The amount of time spent by the mice in the shallow zone (safe zone) in relation to the total time was calculated.

### Isolation of cells from the murine CNS

The CNS cells were isolated as described previously (*20a*) with modifications. Briefly, the mice were anesthetized and were perfused with 1X cold PBS. The CNS was isolated, homogenized and filtered through a 70 µm nylon filter. The homogenate was suspended in 37% isotonic Percoll solution and centrifuged at 800 × g for 30 min without a break in application. The myelin layer was removed from the top of 37% Percoll and the pelleted cells were washed with 1X PBS for further analysis.

### In-vitro T cell functional Assays

B-NHL cells were labelled with cell trace violet (Invitrogen) as per the manufacturer's instructions. B-NHL cells and NT or CD19 CAR T cells were cultured with 0:1, 1:1, 1:5 and 1:10 ratios for 16-20h. Post-incubation cells were washed and stained for live dead zombie stain and specific lysis was calculated. For analyzing the expression of T cell markers, B-NHL and NT or CD19 CAR T cells were co-cultured at a ratio of 1:5 for 16-20h and treated with either 10µM of takinib or vehicle.

### Statistical analysis

For sample size in the murine survival experiments a power analysis was performed. A sample size of at least n=8 per group was determined by 80% power to reach a statistical significance of 0.05 in order to detect an effect size of at least 1.06. Differences in animal survival (Kaplan-Meier survival curves) were analyzed by Mantel Cox test. The experiments were performed in a non-blinded fashion except for behavioral experiments with the knockout mice. For statistical analysis of 2 groups an unpaired 2 tailed Student's t test was applied. All data were tested for normality applying the Kolmogorov-Smirnov test. If the data did not meet the criteria of normality, the Mann-Whitney U test was applied. If more than 2 groups were analyzed, we used the Kruskal-Wallis-Test if non-parametric testing was suggested we performed a one-way ANOVA in case of normally distributed data. Statistical analysis was performed using GraphPad Prism (GraphPad Software; San Diego, CA). Data are presented as mean and s.e.m. (error bars). Differences were considered significant when the P-value was <0.05.

### Subclustering of microglia and further analysis

Based on the cell identity and known marker genes, subclustering of microglia were performed using the same unsupervised algorithm and downstream analysis. After removing clusters which expressed genes that are expressed in non-microglial cells, final sub-clustering was done with a modification that FindNeighbors function was run with dims = 5 and FindClusters function was run with resolution 0.5. Based on the DE genes found by the FindAIIMarkers function with test.use = "MAST" (21a), heat maps was plotted for top 10 DE genes per clusters. Merimekko plot for microglia clusters was generated using ggmosaic package (22a).

To understand the overall impact of CAR T cell treatment on microglia, FindMarkers function was used to compare "NT" vs "CAR" treatment derived microglia independent of clusters. Based on the DE genes obtained from this analysis, volcano plots were created using the EnhancedVolcano package v1 (*23a*)*.*

### Gene set enrichment analysis (GSEA) and KEGG

To perform GSEA analysis of microglial clusters, DE gene list containing only upregulated genes was generated (max.cells.per.ident = 2000, random seed = 1, only.pos = T, test.use = "MAST") and top 150 genes per cluster were included in the analysis. To automatically calculate enriched functional profiles of each microglial cluster and aggregate the results, compareCluster function from R package clusterProfiler was used (*24a*). To perform analysis, fun parameter was set to enrichGO to obtain GO analysis. To remove redundant enriched terms from GO analysis of clusters, simplify function (cutoff = 0.5) was used. Visualization of outputs was generated using the Dotplot function.

### Results

### Activation of microglia upon CAR19 T cell transfer in mice

To study the impact of CAR19 T cells on microglia, we used a B-cell lymphoma (B-NHL) model (A20) and a B-ALL model (E2a-PBX1), as these are the main clinical indications for CAR19 T cell treatment. Tumor-bearing mice were treated with syngeneic mouse CAR19 T cells at a schedule and dosage that induced anti-lymphoma and anti-leukemia activity in both models **(**Figure 10 a-g). At the time of analysis, (day 10) there was no A20-GFP lymphoma cell infiltration detectable in the CNS (**Figure 11a****-b**). Morphological analysis of the microglia by IMARIS on day 10 after tumor injection revealed major changes with respect to filament length, number of dendrite segments, number of dendrite branch points and number of dendrite terminal points upon CAR19 T cell transfer when compared to the non-transduced (NT) controls (**Figure 1a****-e**). Comparable morphological changes during cellular activation in microglia were reported during CNS disorders in amyloid beta (Aβ) plaque-driven pathology in Irf8-/- mice (19), autoimmune encephalitis (20) and GVHD (21). Additionally, we observed upregulation of CD80 on microglia (CD11b+CD45lo), indicating their activation during CAR19 T cell-induced neurotoxicity in both lymphoma and leukemia models (**Figure 1f****-i**). To determine the effect CAR19 T cell therapy on peripheral immune cells, myeloid (CD45⁺CD11b⁺) cells from bone marrow (BM) and spleen of B-NHL and B-ALL bearing mice were analyzed. We observed upregulation of co-stimulatory markers and pro-inflammatory cytokines in CD11b⁺ cells in BM and spleen of B-NHL and B-ALL bearing mice that received CAR19 T cells compared to NT T cell controls (**Figure 12a****-i, 4 a-f**), which supports the concept that mice receiving CAR T cells develop myeloid cell activation and cytokine release syndrome.

### Single nucleus RNA-sequencing (snRNA-seq) reveals major transcriptional changes in microglia after CAR19 T cell treatment

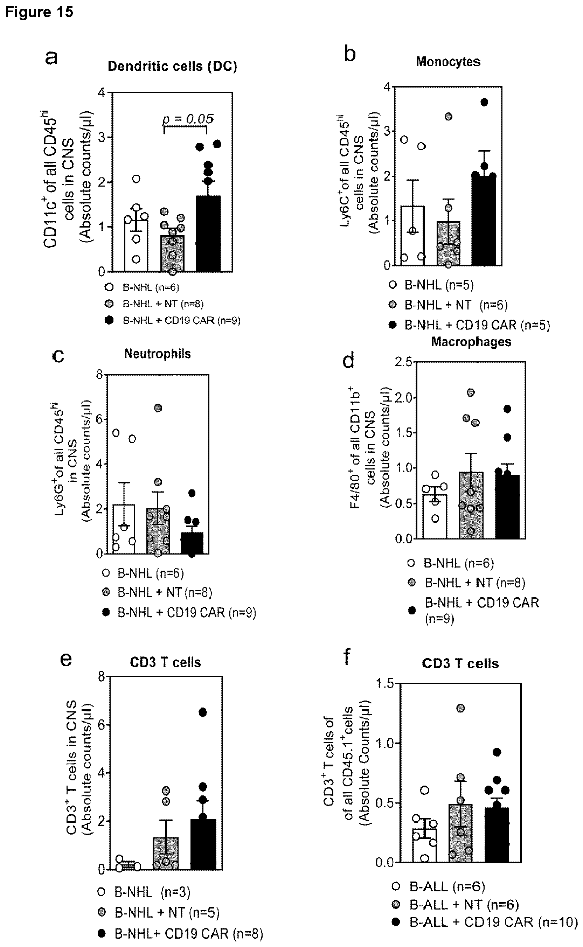

To characterize the impact on CAR19 T cell therapy on immune cell infiltration to the CNS, lba-1⁺ myeloid cells were quantified in both B-NHL and B-ALL models. CAR19 T cell treated mice exhibited increased numbers of Iba-1 cells in the cortex of B-NHL and B-ALL bearing mice when compared to the respective NT T cell controls (**Figure 2a****-c, 15a**). To understand the contribution of peripheral immune cells in mediating CAR19 T cell-induced neurotoxicity, total absolute counts of infiltrating cells were quantified. There were no notable differences in the total absolute counts of CD11c⁺, Ly6C⁺, Ly6G⁺ and F4/80⁺ cells infiltrating the CNS of B-NHL bearing mice that had received CAR19 T or NT T cells (Figure 56a-d). In addition, the total absolute counts of CD3 T cells remained unaltered in the CNS of B-NHL and B-ALL bearing mice upon CAR19 T cell treatment (**Figure 15e****-f**). Additionally, we found a significant increase in absolute numbers of CD11b⁺CD45^{lo} cells in the CNS of B-NHL bearing mice that had received CAR19 T cells **(**Figure 2d, e) indicating microglial activation. To further study the impact of CAR T cell treatment on transcriptional states of microglia in the B-NHL model, mice were treated with NT T cells or CAR19 T cells and the brains were isolated on day 10 after tumor injection (i.e. 3 days after CAR19 T cell treatment). Microglia were enriched by sorting out NeuN-Olig2 nuclei and analyzed using 10X Genomics single-nucleus 3' mRNA- sequencing technology. Following quality control, several clusters of different cell types were identified based on their marker gene expression. The resulting dataset was comprised of microglia, CNS-associated macrophages (CAMs), oligodendrocytes, astrocytes and others including vascular and leptomeningeal cells (VLMCs) (Figure 2f, Figure 16a, b). The number of genes per nucleus was similar between NT and CAR T cell groups (Figure 16c). Cell type assignment was conducted using the Azimuth algorithm based on human peripheral blood mononuclear cells with known transcriptomic and cell surface marker profiles as described before (22, 23). Gene expression in the microglia cluster was fundamentally different in the CAR T cell group compared to the NT T cell group (Figure 2g). To decipher the differential microglial gene expression in detail, sub-clustering was performed and 7 clusters were identified (Figure 2h). When assigning these microglial clusters to treatment groups, clusters 1, 3 and 4 were more frequent in the CAR19 T cell group, while clusters 2 and 5 were mainly found in the NT T cell group (Figure 2i). Differential expression (DE) analysis showed upregulation of *Cst3* (Cystatin C) in the CAR19 T cell group (Figure 2j), which was previously shown to be involved in the pathophysiology of different CNS diseases. Cystatin C microinjection into the rat hippocampus has also been shown to induce neuronal cell death (24). Gene ontology (GO) analysis based on cluster-wise DE showed that cluster 1 (CAR treatment) microglia exhibited increased expression of genes related to antigen presentation and synapse pruning including C1qa, b, c and CD68 (Figure 2k) as reported (25). Cluster 4 (CAR treatment) microglia exhibited increased expression of genes related to IL-6 production and cell migration (Figure 2k), which is interesting given the reported role of IL-6 in ICANS (9, 26). The finding of several genes expressed more abundantly in the CAR19 T cell group involved in antigen processing and presentation (Figure 2k) supports the concept of microglia activation during ICANS.

To complement the snRNA-seq analysis, that captures only a limited number of genes, we analyzed the transcriptional changes in microglia on the bulk mRNA level. FACS sorted microglia (CD11b⁺CD45^{lo}) from the CNS of A20 bearing mice treated with either NT or CAR19 T cells were submitted to mRNA microarray-based analysis. Microarray-based analyses revealed major differences in gene expression of microglia derived from mice that received NT T cells compared to CAR19 T cells, leading to differential clustering based on the gene expression signatures of the groups (**Figure 3a**). In addition, based on gene-set enrichment, we observed significantly increased expression of multiple genes related to the TNF signaling pathway in microglia derived from mice receiving CAR19 T cells compared to NT T cells (**Figure 3b**). Consistently, intracellular TNF protein increased in microglia derived from mice receiving CAR19 T cells compared to NT T cells (**Figure 3c****, d**). Pathogenic TNF production has been previously connected to cognitive deficits in chronic schizophrenia, Alzheimer's disease and GVHD (21, 27, 28). Since previous studies showed that CCL-2 can cause neurocognitive alterations (29) and that GM-CSF contributes to CRS and cognitive deficits (11) we next analyzed CCL-2 and GM-CSF expression. We observed upregulation of CCL-2 and GM-CSF in microglia derived from CAR19 T cell-treated lymphoma-bearing mice, indicating strong microglial activation during CAR19-induced neurotoxicity (**Figure 3e****-h**). Triggering receptor expressed on myeloid cells 2 (TREM2) was found to be upregulated on microglia in neurodegenerative and neuroinflammatory diseases (30). It is also known to enhance the activation of the NLRP3 inflammasome (31), and soluble TREM2 induces inflammatory responses, as well as microglia survival (32). Consistent with strong microglial activation, we observed increased TREM2 expression on microglia derived from CAR19-treated compared to NT T cell-treated mice in the B-NHL and B-ALL models (Figure 3i-l)**.** Previous work showed that activated but not naive microglia express CD11c (33). We observed that CD11c expression increased on microglia derived from CAR19 T cell-treated compared to NT T cell treated mice (**Figure 17a****-d**). These findings show that CAR19 T cell transfer into B cell malignancy bearing mice leads to morphological signs of microglial activation, transcription of pro-inflammatory cytokines and upregulation of surface markers indicative of microglial stimulation.

### Microglia depletion improves neurocognitive deficits

To analyze the functional role of microglia in CAR19-induced neurotoxicity, we performed behavior tests, including an elevated plus maze test and a novel object recognition test in mice with and without microglial depletion on day 3 after CAR T cell transfer.

We observed that B-ALL (E2A-PBX1) bearing mice treated with mouse CAR19 T cells exhibited neurocognitive deficits when compared to mice receiving B-ALL only or B-ALL plus NT T cells, when analyzed by both the elevated plus maze test and the novel object recognition test **(**Figure 4a, b). The transfer of CAR T cells alone in the absence of tumor cells induced no change in cognitive function in the novel object recognition test when compared to the NT T cell controls **(**Figure 18a, b). The neurocognitive differences seen in the leukemia-bearing mice upon CAR T cell transfer were not due to a global loss of strength, because no difference in grip strength between the respective groups was observed (**Figure 4c**). The transfer of CAR T cells or NT T cells did not alter NeuN⁺ neuronal counts in the cortex of B-NHL bearing mice (**Figure 19a****, b**). Consistent with the neurocognitive deficiency observed in mice treated with CAR19 T cells, we observed a decreased expression of multiple genes related to postsynaptic neurotransmitter receptor activity (**Figure 4d**). This includes genes encoding subunits of glutamate (*Grid1, Grik4*) and gamma-amino butyric acid (GABA) (*Gabra1*) neurotransmitter receptors. Glutamatergic signalling is required for learning and memory (34) and disruption is implicated in many cognitive and psychiatric disorders (35). Moreover, conditions in which Gabra1 expression is lost leads to increased anxiety as measured by the elevated plus maze (36, 37). Elevated levels of TNF have been shown to diminish postsynaptic GABA receptor levels (38), which may contribute in the setting of CAR19 T cell-induced neurotoxicity. Additionally, multiple genes with the annotation intrinsic component of postsynaptic membrane were downregulated in neurons from the CNS of mice treated with CAR19 T cells (**Figure 20a**). These genes included postsynaptic receptor subunits (*Grin3A)* (*39*) *and Grid1 encoding* for glutamate D1 receptor (GluD1) (40) indicating that the stability, activity, and number of functional synapses may be compromised.

In agreement with inflammation mediated by the CAR19 T cell transfer, leakage of high molecular weight protein dextran into the cortex of CNS was higher in B-ALL (E2A-PBX1) bearing mice treated with mouse CAR19 T cells compared to mice receiving B-ALL plus NT T cells, indicating increased BBB permeability (**Figure 4e****, f**). To deplete microglia, we used PLX5622, a CSF1R inhibitor (from day -7 to day 10) that has previously been described (**Figure 21a**) (41). Microglial depletion was efficient as microglia numbers declined in mice treated with the CSF1R inhibitor compared to vehicle treated mice (**Figure 21b****-d**). In addition, there were no significant changes in the CD45^{hi}, Ly6G⁺ and Ly6C⁺ myeloid cells infiltrating the CNS upon CSF1R inhibition **(****Figure 21e****-g).**

In agreement with a functional contribution of microglia, we found that depletion of microglia, starting one week prior to tumor injection until the day of analysis, improved neurocognitive deficits in the B-ALL (E2A-PBX1) bearing mice treated with mouse CAR19 T cells when analyzed using the same tests (**Figure 4g****, h**). The neurocognitive differences between the different groups were not due to a global loss of strength, because no difference in grip strength between the respective groups was observed (Figure 4i). CSF1R inhibitor treatment prior to CAR19 T cell transfer did not reduce the lymphoma burden of the mice in spleen and BM compared to vehicle-treated mice (**Figure 22a****, b**) and had no impact on Ly6C⁺ and F4/80⁺ myeloid cells in the spleen **(**Figure 22c, d). CSF1R inhibitor treatment prior to CAR19 T cell transfer did not reduce the BBB leakage of CAR19 T cell-treated mice compared to NT T cell-treated mice (**Figure 23a****, b**). Altogether, these findings show that microglia contribute to the neurocognitive defects caused by CAR19 T cells in B-ALL (E2A-PBX1) bearing mice.

### The TGF-β-activated kinase-1 (TAK1)/NF-κB p38-MAPK-pathway is activated in microglia upon CAR19 T cell transfer

To study kinase activation in microglia, we next isolated microglia from lymphoma-bearing mice that had been treated with NT T cells or CAR19 T cells and submitted them to analysis for serine-threonine kinase activity. We observed increased phosphorylation of multiple kinases at the phospho-protein level (**Figure 5a****, b**) that are down- or upstream of TAK-1, and increased transcription of genes involved in the p38-MAPK pathway (**Figure 5c**). One example is that TAK1 activation requires phosphorylation by Protein Kinase A Catalytic Subunit α (PKACα) (42), a kinase that we found to be activated in the microglia of mice that had received CAR19 T cells **(****Figure 5b****,** red arrow). A second kinase that was increased in microglia of CAR19 T cell treated mice was CaMK2α **(****Figure 5b****,** red arrow), which directly binds to and activates TAK1 in macrophages (43). In agreement with these findings, we observed increased p38-MAPK phosphorylation, a downstream target of TAK1, in microglia derived from B-NHL (A20)-bearing mice treated with mouse CAR19 T cells, compared to B-NHL bearing mice receiving NT cells or no T cells (**Figure 5d****, e**). We could confirm the increased p38-MAPK phosphorylation in microglia also in B-ALL (E2A-PBX1) bearing mice that received CAR19 T cells (**Figure 5f****, g**). These findings indicate the transfer of CAR19 T cells causes pathogenic p38-MAPK signaling in microglia. At the transcriptional level we found increased expression of genes connected to TAK1/NF-κB pathway activity (**Figure 6a**), which are also connected to the p38-MAPK pathway because TAK1 mediates p38 MAPK activation (44). To test if we could reverse the pro-inflammatory phenotype of microglia upon CAR19 transfer, we used the selective TAK1 inhibitor takinib (**Figure 24** **a**) (45) which has been shown to reduce inflammatory activation of human rheumatoid arthritis synovial fibroblasts (46). Treatment with takinib did not reduce myeloid cell, T cell or NK cell counts in the periphery (Fig. 24 b-e, 16a-d). We found that mice treated with CAR19 T cells and takinib exhibited reduced expression of TNF and GM-CSF compared to B-NHL-bearing mice receiving CAR19 T cells and vehicle (**Figure 6b****-e**). Additionally, takinib treatment reduced p38 phosphorylation and p70s6K total protein which is consistent with reduced microglial activation (**Figure 6f****-i**).

We observed that takinib treatment improved neurocognitive deficits in B-NHL (A20)-bearing mice that were treated with CAR19 T cells, compared to those receiving vehicle when analyzed by the elevated plus maze test and the novel object recognition test (**Figure 6j****, k**)**.** These differences were not due to a global loss of strength because there was no difference in the grip strength test between the groups (**Figure 6I**)**.**

We observed a trend towards a lower BBB leakage in the cortex of B-ALL (E2A-PBX1) bearing mice after CAR19 T cell transfer when treated with takinib compared to mice receiving vehicle **(****Figure 6m****,** **Figure 26 a)****.**

These findings show that TAK1/NF-κB pathway activity contributes to the neurocognitive defects caused by CAR19 T cells in lymphoma bearing mice, and that these can be antagonized by blocking TAK1 with takinib.

### Genetic TAK1 deletion in microglia improves neurocognitive deficits

To validate the pharmacological TAK1 inhibitor approach through a genetic loss-of-function approach, we next used Cx3cr1^{Cre:ER} Tak1^{fl/fl}mice, where TAK1 is deleted in microglia (47). TAK1 is initially deleted in monocytes, CAMs and microglia but while monocytes repopulate, microglia are long-lived and keep the deletion, as previously shown (47). We observed a decrease of CD80, TREM2, CSF1R and p38-MAPK activation in the microglia of B-ALL (E2A-PBX1) bearing Cx3cr1^{Cre:ER}: Tak1^{fl/fl} mice compared to Tak1^{fl/fl}control mice, with both groups being treated with CAR19 T cells **(****Figure 7a****-h**). The tumor burden was not different when comparing Cx3cr1^{Cre:ER}: Tak1^{fl/fl} mice with Tak1^{fl/fl} control mice in the spleen and BM (**Figure 27a****, b**). To understand whether there is a connection between a genetic lack of TAK1 in microglia and improved neurocognitive function of mice receiving CAR19 T cells, we next analyzed the neurocognitive activity of the mice. We observed that genetic TAK1 deletion in microglia improved neurocognitive deficits of B-ALL (E2A-PBX1) bearing Cx3cr1^{cre:ER}: Tak1^{fl/fl} mice compared to Tak1^{fl/fl} control mice, both groups being treated with CAR19 T cells, when analyzed by the elevated plus maze test and the novel object recognition test **(****Figure 7i****, j**), while the grip strength test of both groups remained unaffected **(****Figure 7k**)**.** These findings indicate that genetic TAK1 deletion in microglia reduces microglial activation, and improves the neurocognitive defects caused by CAR19 T cells in B cell malignancy bearing mice. Since TAK1 inhibition has systemic effects, the Cre/lox based genetic model that causes selective TAK1 deletion in microglia was essential to prove that TAK1 inhibition in microglia was the key intervention and not TAK1 inhibition in peripheral myeloid cells.

### TAK1 inhibition enhances CAR19 mediated anti-lymphoma activity

Since takinib treatment could be a potential therapeutic option to prevent or treat CAR T cell induced neurotoxicity, the inventors next evaluated the impact of takinib on the anti-lymphoma effect of the CAR19 T cells. The inventors observed that TAK1-inhibition enhanced the anti-lymphoma activity when combined with transferred CAR19 T cells, because the B-NHL (A20) lymphoma burden decreased in CAR19/takinib treated mice compared to CAR19/vehicle treated mice (**Figure 8a****, b**). In agreement with the intact CAR19 T cell activity *in vivo,* increased cytotoxicity against B-NHL (A20) in the presence of takinib *in vitro* was also observed **(****Figure 8c**). Takinib induced a dose-dependent cell death in the B-NHL (A20) and B-ALL (E2A-PBX1) cells (**Figure 28a****, b**). Furthermore, markers of cytotoxicity including perforin, CD107a, IFN-y, and TNF remained unaffected by exposure of CAR19 to takinib and B-NHL (A20) **(****Figure 29a****-g**). The viability of CAR19 or NT T cells also remained unaffected upon exposure of takinib and B-NHL (A20) (Figure 329h)**.** CAR19/takinib treated B-NHL bearing mice survived longer when compared to CAR19/vehicle treated mice **(****Figure 8d**). Conversely, when B-NHL (A20) bearing mice were treated with prednisolone, the standard therapy for ICANS, the protective effect of CAR19 T cells against lymphoma growth was reduced **(****Figure 8e**). Additionally, the survival of CAR19/takinib treated mice also increased compared to CAR19/vehicle treated mice in the E2a-PBX1 model **(****Figure 8f**). However, when B-ALL (E2a-PBX1) bearing mice were treated with prednisolone, the protective effect of CAR19 T cells against leukemia expansion was reduced **(****Figure 8g**). These findings indicate that takinib can be tested in patients treated with CAR19 T cells for B cell malignancies. Takinib had a clear additive anti-leukemic and anti-lymphoma effect in two models.

### Activation of microglia upon CAR19 T cell transfer in patients

To study the impact of CAR19 T cells on human microglial cells, the inventors analyzed the CNS of patients that had received CAR T cells for B-NHL, developed ICANS and underwent autopsy. Using Imaging Mass Cytometry (IMC)-based visualization of the white matter of the occipital lobe, we compared CAR T cell patients and age-/sex-matched controls (**Figure 9a**). The inventors observed strong microglial activation with a loss of the microglial homeostatic markers P2RY12 and TMEM119, along with a concomitant upregulation of CD11c, CD204, and CD68 as a marker for lysosomal activity in CAR T cell patients (ICANS patients) compared to controls (**Figure 9a**). S100A9 (MRP14) is typically expressed on monocytes that are invading the CNS from the bloodstream and gradually upregulates microglial markers to complement the microglia pool (48). The inventors observed an increase of S100A9 in the microglia of ICANS patients compared to control patients (**Figure 9a**). Single-cell data were obtained through machine learning supervised segmentation of IMC data. Single-cell marker expression in all cells and in microglia (Iba-1⁺ cells) was fundamentally different in patients with ICANS compared to controls (**Figure 9b**). Additionally, clusters of activated microglia and macrophages were predominant in patients with ICANS but not in control patients (**Figure 9c**). IMC revealed increased frequency of P2RY12^{Io} HLA-DR⁺ populations in Iba1⁺ cells in patients with ICANS compared to control patients (**Figure 9d**). There was a trend towards a reduced frequency of P2RY12⁺ cells, which are considered as resting microglia, in the CNS of ICANS patients when compared to the controls (**Figure 30a****, b**). There was a trend towards an increased frequency of CD11c⁺ cells, which are considered activated, in the CNS of ICANS patients when compared to the controls (Figure 30c, d). Clusters (C1 - C13) are defined based on the expression of marker genes (Figure 30e). The differential gene expression within the clusters are depicted in heat maps (Figure 30f).

Using conventional IHC for Iba1, HLA-DR and CD3, we confirmed an increase of myeloid cells that were either Iba-1 or HLA-DR positive in the white matter of ICANS patients when compared to controls (**Figure 9e****, f**)**.** Intracellular vesicles within roundish Iba1-positive cells were visible in patient #1. Iba1-positive cells in patient #2 exhibited an activated phenotype with a spiky appearance. To dissect the pathomechanisms causing the neurological defects in the patients after CAR Tc transfer, we analyzed the CNS using H&E staining which morphologically revealed characteristics of a Wallerian degeneration. Amyloid precursor protein (APP) deposits point towards an axonal damage. This axonal damage was potentially followed by myelin loss and uptake of myelin by macrophages, which can be accompanied by macrophage activation. In order to test this, we combined Iba1 immunohistochemistry with Luxol-Fast-Blue as a marker for demyelination and Periodic-Acid-Schiff-Stain which stains carbohydrates, indicating the blue myelin within the vesicles in Iba1-positive cells (**Figure 9g**), supporting the presence of Wallerian degeneration in a patient receiving CAR T cells.

### Discussion

ICANS is a major complication after CAR19 T cell therapy for B cell malignancies and leads to high morbidity (12, 13, 54). The role of microglia in the pathogenesis of ICANS is not well characterized. By using genetic and pharmacological approaches, the inventors herein deciphered the functional contribution of microglia to the development of ICANS in mice. The present finding that CAR19 transfer into B-NHL bearing mice induces morphological changes of microglia towards an activated phenotype is in agreement with other inflammatory conditions of the CNS, such as acute GVHD of the CNS (21) and experimental autoimmune encephalitis (EAE) (47). This increased activation was associated with a functional contribution of microglia to neurocognitive defects, as depletion of microglia reduced the severity of these defects, and we observed decreased expression of multiples genes related to postsynaptic neurotransmitter receptor activity in mice treated with CAR T cells. Reduced expression included genes encoding subunits of glutamate (Grid1, Grik4) and gamma-amino butyric acid (GABA) (Gabra1) neurotransmitter receptors. This could be responsible for CAR T cell-induced neurocognitive deficits as glutamatergic signaling is required for learning and memory (34) and disruption is implicated in many cognitive and psychiatric disorders (35). The present observation that TNF production increased in microglia derived from mice that received CAR19 T cells compared to NT T cells could also help to explain the neurocognitive defect mediated by microglia. Pathogenic TNF production has been previously reported in patients with GVHD (21), chronic schizophrenia, as well as in mice with Alzheimer's disease (27, 28). In addition, the inventors found increased CCL-2 production, which can cause neurocognitive alterations (29). Previous studies showed that GM-CSF inhibition with lenzilumab reduces CRS and neuroinflammation in xenograft models of acute leukemia (11), which is concordant with our finding that microglia produces GM-CSF in B-NHL bearing mice upon CAR T cell transfer.

The next aim was to study signaling in microglia for the identification of therapeutic targets. The inventors could identify a role for the TAK1/NF-κB pathway in microglia, which upon activation contributes to the neurocognitive defects caused by CAR19 T cells in lymphoma bearing mice. Targeting TAK1 by genetic deletion or pharmacological inhibition using the TAK1i takinib surprisingly reversed microglia activation signs and improved neurocognitive deficits as summarized in **Figure 6****,** **7****.** The TAK1-NF-κB axis was previously identified as a therapeutic target for AML (55), suggesting that targeting TAK1 for ICANS could be translated into a clinical trial without loss of anti-leukemia activity. Additionally, takinib had a clear additive anti-leukemic and anti-lymphoma effect in two models suggesting that it could be an attractive combination partner for CAR T cell therapy in clinical studies. Current prophylaxis and therapy for ICANS includes tocilizumab, a monoclonal antibody targeting interleukin-6 (56). Previous studies have shown that IL-6 production is induced via TAK1-NFκB pathway activation (57). However, in contrast to a selective blockade of the IL-6R, the use of takinib blocks TAK1, which is a central regulatory kinase in the TAK1-TAB signalosome (58, 59). TAK1 is known to mediate production of multiple pro-inflammatory cytokines involved in ICANS including TNF (58, 59). TREM2 expression was shown to be associated with the production of inflammatory cytokines in a PI3K/AKT and NF-kB-dependent manner in neuroinflammatory diseases (32, 60), which is consistent with the inventors observation that increased levels of TREM2 and TAK1 are found in activated microglia after CAR19 T cell therapy.

Novel therapies for patients receiving CAR T cells are needed as the use of corticosteroids for the treatment of ICANS has been shown to decrease progression-free survival of patients (13). Additionally, a higher cumulative dose of corticosteroids, and prolonged and early use after CAR-T cell infusion were associated with significantly shorter overall survival (13). Therefore, novel targeted strategies inhibiting selective immune activation without blocking CAR-T cell function could improve clinical outcome. In agreement with an intact anti-B-NHL and anti-B-ALL effect for CAR19 T cells, we observed that takinib did allow for cytotoxicity *in vitro* and *in vivo* against the malignant B cells. Our finding that ICANS correlates with microglia activation expands previous reports showing perivascular infiltration of CD3+/CD8+-T cells (63) and glial injury (64) in patients that died from ICANS.

In summary, the inventors surprisingly identified a novel functional role for microglia in promoting CAR19 T cell mediated neurotoxicity. Patients with ICANS exhibited microglia activation in post-mortem IMC and IHC analysis. The inventors revealed the TAKI/NF-κB p38-MAPK-axis as a pathogenic signaling pathway activated in microglia during ICANS, using both genetic and pharmacological approaches. Therefore, the inventors herein provide a TAK1-inhibitor-based (e.g., takinib-based) approach to interfere with ICANS, a major complication after CAR19 T cell-based immunotherapy that will be tested in clinical trials.

## Claims

1. A transforming growth factor-β-activated kinase-1 (TAK1) inhibitor for use in the treatment of a neuroinflammatory disorder.

2. The TAK1 inhibitor for use according to claim 1, wherein the neuroinflammatory disorder is a microglia-mediated neuroinflammation.

3. The TAK1 inhibitor for use according to any one of the preceding claims, wherein the neuroinflammatory disorder is a T-cell induced microglia-mediated neuroinflammation.

4. The TAK1 inhibitor for use according to any one of the preceding claims, wherein the neuroinflammatory disorder comprises or consist of a neuroinflammatory disorder and cytokine-release syndrome (CRS).

5. The TAK1 inhibitor for use according to any one of the preceding claims, wherein the neuroinflammatory disorder is a T-cell induced microglia-mediated neuroinflammation.

6. The TAK1 inhibitor for use according to any one of the preceding claims, wherein the TAK1 inhibitor reduces microglial activation in said subject.

7. The TAK1 inhibitor for use according to any one of the preceding claims, wherein the neuroinflammatory disorder comprises or consist of a neuroinflammatory disorder of the central nervous system (CNS).

8. The TAK1 inhibitor for use according to any one of the preceding claims, wherein said inhibitor inhibits TAK1 pharmacologically and/or genetically by inhibiting its expression.

9. The TAK1 inhibitor for use according to any one of the preceding claims, wherein the patient is suffering from one or more of the symptoms selected from cognitive deficits, neurotoxicity, seizures, pathological headaches, increased blood brain barrier (BBB) permeability, partial or complete loss of eye sight, partial or complete loss of hearing, partial or complete loss of sensation, partial or complete paralysis, muscle weakness, memory loss, numbness in one or more limbs, tremor, paresis, confusion or disorientation.

10. The TAK1 inhibitor for use according to any one of the preceding claims, wherein the neuroinflammatory disorder is associated with a condition selected from the group comprising immune effector cell-associated neurotoxicity syndrome (ICANS), multiple sclerosis (MS), amyotrophic lateral sclerosis (ALS), Alzheimer's disease (AD) or Parkinson's disease (PD).

11. The TAK1 inhibitor for use according to any one of the preceding claims wherein the neuroinflammatory disorder is an ICANS in a mammalian subject, wherein said subject is receiving a chimeric antigen receptor (CAR) T-cell based immunotherapy for the treatment of haematological (B-cell) malignancies.

12. The TAK1 inhibitor for use according claim 11, wherein the haematological (B cell) malignancies are selected from the group comprising leukemia, B-cell lymphoma, B-cell acute lymphoblastic leukaemia (B-ALL), precursor B-cell ALL, B-cell non-Hodgkin lymphoma (B-NHL), mixed lineage leukemia (MLL), large cell transformation of follicular lymphoma (trFL), Diffuse large B-cell lymphoma (DLBCL), Follicular lymphoma (FL), Chronic lymphocytic leukemia (CLL) /small lymphocytic lymphoma (SLL), mantle cell lymphoma (MCL), marginal zone lymphoma (MZL), Burkitt lymphoma (BL), lymphoplasmacytic lymphoma, hairy cell leukemia (HCL), primary central nervous system (CNS) lymphoma, primary intraocular lymphoma and monoclonal B-cell lymphocytosis (MBL).

13. The TAK1 inhibitor for use according to any one of claims 11 or 12, wherein the anti-lymphoma effects of the CAR T-cell based immunotherapy are not impaired.

14. The TAK1 inhibitor for use according to any one of the preceding claims, wherein the neuroinflammatory disorder is associated with an increased production, induction and/or activation of IL-6, p38, p70 S6K, GM-CSF, MCP-1 and/or TNF by activation of microglia, which is preferably identified by analyzing cerebrospinal fluid (CSF) of a subject.

15. The TAK1 inhibitor for use according to any one of the preceding claims, wherein administration of the TAK1 inhibitor leads to reduced microglia activation, lower production of GM-CSF, MCP-1 and TNF.

16. The TAK1 inhibitor for use according to any one of the preceding claims, wherein administration of the TAK1 inhibitor reduces neuroinflammation.

17. The TAK1 inhibitor for use according to any one of the preceding claims, wherein administration of the TAK1 inhibitor reduces symptoms selected from cognitive deficits, neurotoxicity, seizures, pathological headaches, increased blood brain barrier (BBB) permeability, partial or complete loss of eye sight, partial or complete loss of hearing, partial or complete loss of sensation, partial or complete paralysis, muscle weakness, memory loss, numbness in one or more limbs, tremor, paresis, confusion or disorientation, toxicities of cytokine release syndrome (CRS), neurotoxicity and/or neurocognitive defects in a subject.

18. The TAK1 inhibitor for use according to any one of the preceding claims, wherein the TAK1 inhibitor is selected from the group comprising Takinib (EDHS-206), HS-276, Dehydroabietic acid, RGB-286638 free base, Sarsasapogenin, 5Z-7-Oxozeaenol, NG25 and pharmaceutically acceptable salts thereof.

19. The TAK1 inhibitor for use according to any one of the preceding claims, wherein the inhibitor is administered transiently or constantly.

20. The TAK1 inhibitor for use according to any one of the preceding claims, wherein the treatment further comprises (simultaneous or sequential) administration of one or more other therapies selected from the group comprising corticosteroids, Anti-IL-6 receptor antibodies, anti-IL-1beta antibodies, anti-IL-1beta receptor antagonists (Anakinra), calcineurin inhibitors (Cyclosporine A, tacrolimus), Interferon-beta, glatiramer acetate, dimethyl fumarate, diroximel fumarate, monomethyl fumarate, teriflunomid, fingolimod, fingolimod, fingolimod, mitoxantrone, ponesimod, ozanimod fingolimod, ofatumumab, alemtuzumab, ocrelizumab, natalizumab and ublituximabxiiy.
